# EUROPÄISCHE PATENTSCHRIFT

(11) **EP 0 729 969 B1**
(45) Veröffentlichungstag und Bekanntmachung des Hinweises auf die Patenterteilung: **16.08.2000**
(21) Anmeldenummer: 96810094.1
(22) Anmeldetag: 16.02.1996
(51) Int. Cl.: C07F 17/02, C07B 31/00, C01B 33/20, C08F 112/08, C08F 110/00, C08F 120/10, C08F 136/08, C08F 136/06, C08G 69/00, C08G 18/83, B01J 21/00, C07B 53/00

(54) **Silylierte Ferrocenyldiphosphine, an anorganische oder polymere organische Träger gebundene silylierte Ferrocenyldiphosphine sowie Metallkomplexe davon, ihre Herstellung und Verwendung**
Silated ferrocene-diphosphine ligands, inorganic or polymeric organic support to which these ligands are bound and metal complexes thereof, and their preparation and use
Ligandes ferrocène-disphosphine silylée, support inorganique ou à base de polymères organiques les contenant et leurs complexes métalliques et procédé de préparation et utilisation

(30) Priorität: 24.02.1995 CH 54395
(43) Veröffentlichungstag der Anmeldung: 04.09.1996
(73) Patentinhaber: Novartis AG, 4058 Basel (CH)
(72) Erfinder: Pugin, Benoît, Dr., 4142 Münchenstein (CH)

(56) Entgegenhaltungen:
- EP-A- 0 564 406
- EP-A- 0 612 758
- WO-A-96/16971
- BULLETIN OF THE KOREAN CHEMICAL SOCIETY, Bd. 13, Nr. 6, 1992, Seiten 588-592, XP000565474 KIM, T.-J. ET AL.: "FUNCTIONALIZED ORGANOMETALLIC LIGAND (1) SYNTHESIS OF SOME FERROCENE DERIVATIVES OF CYCLOHEXYL- AND CYCLOPENTADIENYL-PHOSPHINES"

## Beschreibung

Die Erfindung betrifft Ferrocenyldiphosphin-Liganden mit einer Silylengruppe, über diese Silylengruppe an anorganische oder polymere organische Träger gebundene Ferrocenyldiphosphine, deren Herstellung sowie deren Metallkomplexe mit den Übergangsmetallen Rhodium oder Iridium. Die Erfindung betrifft auch die Verwendung dieser Komplexe zur Hydrierung von organischen Doppel oder Dreifachbindungen, insbesondere olefinischen Doppelbindungen und Kohlenstoff-Heterodoppelbindungen. Die Komplexe eignen sich insbesondere für die enantioselektive Hydrierung unter Verwendung von chiralen Diphosphinen und prochiralen ungesättigten Verbindungen.

In der EP-A-0 256 982 wird die enantioselektive Hydrierung von Ketiminen zu optisch aktiven sekundären Aminen mit Hilfe von chiralen Dioxolan-Iridiumdiphosphinkomplexen als homogenen Katalysatoren beschrieben. Die teuren Katalysatoren können jedoch nicht oder nur mit aufwendigen Trennmethoden zurückgewonnen werden, was stets mit unerwünschten Verlusten verbunden ist. Darüber hinaus weisen diese Katalysatoren im Verlaufe ihrer Verwendung einen hohen Aktivitätsverlust auf, so dass die Wiederverwendung der zurückgewonnene homogenen Katalysatoren unmöglich und/oder unwirtschaftlich ist. Es besteht daher ein Bedarf an leicht abtrennbaren und wiederverwendbaren Katalysatoren, deren Aktivität und besonders deren Selektivität bei wiederholtem Gebrauch weitgehend erhalten bleiben.

In der EP-A-0-496 699 und der EP-A-0 496 700 werden Silangruppen enthaltende Dioxolan- und Pyrrolidin-Diphosphine sowie deren Rhodium-oder Iridium-Komplexe beschrieben, die an ein anorganisches Trägermaterial wie zum Beispiel Silikate fixiert werden. Man erhält auf diese Weise bei der Hydrierung eine heterogene Reaktionsmischung, aus der sich nach beendeter Reaktion der anorganisch fixierte Katalysator leicht abtrennen lässt.

Andererseits sind auch polymere Trägermaterialien bekannt geworden, bei denen sich die Diphosphinkomponente an einem copolymerisierbaren Baustein befindet, der dann zusammen mit anderen Monomeren copolymerisiert wird, so dass das Diphosphin oder sein Metallkomplex direkt in der Polymerkette gebunden sind.

K. Achiwa beschreibt in J. Chem. Japan Soc., Chemistry Letters, Seiten 905 bis 908 (1978) Polystyrolcopolymere, deren Benzolreste mit Rhodium komplexierte Pyrrolidin-di- phosphin-N-carbonylgruppen enthalten. Die Synthese der Monomeren ist schwierig und die Hydrierung von prochiralen Olefinen mit diesen heterogenen Katalysatoren ist, verglichen mit nicht polymer gebundenen Katalysatoren, mit einer Emiedrigung der Aktivität, der Produktivität und der Enantioselektivität verbunden. Die Pyrrolidin-Diphosphin Liganden sind über eine para-Amid Bindung direkt am Benzolrest des Styrols fixiert, das den einen Teil des Copolymerisats bildet, während der andere Teil des Copolymergerüsts durch Hydroxyethylmethacrylat gebildet wird. Durch diese direkte Bindung an das Polymergrundgerüst ist die Beweglichkeit des Diphosphin-Liganden stark eingeschränkt, was sich negativ auf die katalytischen Eigenschaften auswirken kann.

Ein weiterer Nachteil dieses Fixierungskonzepts besteht darin, dass das Polymer neu aufgebaut werden muss, was aufwendig ist und zu nicht exakt vorhersehbaren Eigenschaften bezüglich Löslichkeit in organischen Lösungsmitteln, Abtrennbarkeit oder Fällbarkeit nach der Hydrierreaktion führt. Ein derartiger Polymeraufbau begünstigt die teilweise Inklusion des katalytisch aktiven Teils und führt somit zu einer weiter reduzierten Aktivität und Produktivität.

W. R. Cullen et. al. beschreiben in J. of Organometallic Chemistry, 333 (1987), 269-280 Ferrocenderivate wie zum Beispiel N,N-Dimethyl-1-(2-diphenylphosphinoferrocenyl)ethylamin, das direkt an eine oxidierte Polystyrrol Gruppe gebunden ist. Bei der dort vorgeschlagenen Arbeitsweise werden maximal 20% des eingesetzten Ferrocenderivats an den polymeren Träger gebunden und der Ferrocenylligand wird unspezifisch und unselektiv teilweise über den einen oder den anderen Cyclopentadienylring am Polymer gebunden. Durch die direkte Bindung an das Polymergerüst ist die Beweglichkeit des Phosphinliganden ebenfalls eingeschränkt.

Andererseits lassen sich organische polymere Materialien mit höheren Mengen der katalytisch wirksamen Verbindungen belegen als anorganische Träger, was bedeutet, dass weniger Katalysatormasse für die Hydrierungsreaktion eingesetzt werden muss.

Im Hinblick darauf erscheint es wünschenswert, von Trägermaterialien mit bekannten Eigenschaften auszugehen und diese so mit katalytisch aktiven Verbindungen zu modifizieren, dass die Eigenschaften nur geringfügig verändert werden und keine Inklusionen oder anderweitige Veränderungen am katalytisch aktiven Teil auftreten.

Dabei können je nach Hydrierungsreaktion anorganisch oder organisch gebundene Ferrocenyldiphosphinliganden vorteihafter sein.

Die zu katalysierende Reaktion lässt sich beispielsweise im Fall polymergebundener Ferrocenyldiphosphinliganden durch die Wahl des Polymeren heterogen oder homogen führen. Das Polymer kann so gewählt und auch nachträglich gezielt so modifiziert werden, dass sich der Katalysator nach der Reaktion leicht abtrennen und wiederverwenden lässt. Die Katalysatoren können mehrmals wiedereingesetzt werden. Durch die Wahl des Polymeren lässt sich der Katalysator optimal an das Reaktionsmedium während des Hydrierungsschritts anpassen und danach vollständig abtrennen, was insbesondere für grosstechnisch durchgeführte Hydrierungen von Bedeutung ist.

In allen Fällen wird die Rückgewinnung der enthaltenen Edelmetalle erleichtert, wenn der Katalysator nach häufiger Rezyklierung gewechselt werden muss.

Es wurde nun gefunden, dass ausgehend von Ferrocenyldiphosphinen, die einen organischen Rest über eine Silylengruppe an einem Cyclopentadienylring gebunden enthalten, funktionalisierte Ferrocenyldiphosphin-Liganden erhalten werden, die sowohl an anorganischen wie auch an polymeren organischen Trägermaterialien immobilisiert werden können. Die immobilisierten Ferrocenyldiphosphin-Liganden bilden mit Rhodium und Iridium Komplexe, die als hochwirksame Katalysatoren in enantioselektiven Hydrierungen von Kohlenstoff-Kohlenstoff, Kohlenstoff-Stickstoff oder Kohlenstoff-Sauerstoff Doppelbindungen eingesetzt werden können. Die Selektivität und die Gesamtausbeute sind für immobilisierte Systeme überraschend hoch. Die Iridium Katalysatoren sind insbesondere für die Iminhydrierung sehr gut geeignet, da sie die deutlich höchste Aktivität, Selektivität und die höchste Katalysatorproduktivität im Vergleich zu anderen immobilisierten Systemen aufweisen. Die Katalysatoren können leicht aus der Reaktionslösung abgetrennt und wieder eingesetzt werden. Es treten nahezu keine Metallverluste auf. Daher lassen sich mit diesen immobilisierten Katalysatoren insbesondere grosstechnische Hydrierungen wirtschaftlich durchführen.

Die Herstellung dieser immobilisierten Ferrocenyldiphosphine ist erst durch die Bereitstellung von entsprechend funktionalisierten Ferrocenyldiphosphinen möglich geworden. Diese Verbindungen und ihre Herstellung sind ebenfalls neu.

Ein Gegenstand der Erfindung sind daher Verbindungen der Formel I 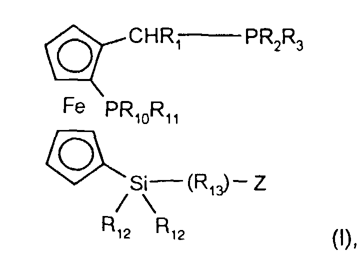 worin
R₁ C₁-C₈-Alkyl, Phenyl oder mit 1 bis 3 C₁-C₄-Alkyl oder C₁-C₄-Alkoxy substituiertes Phenyl bedeutet;
R₂, R₃, R₁₀ und R₁₁ unabhängig voneinander C₁-C₁₂-Alkyl, C₅-C₁₂-Cycloalkyl, Phenyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ ,-[⁺NR₇R₈R₉]X⁻ oder C₁-C₅-Fluoralkyl ein oder mehrfach substituiertes Phenyl darstellen; oder die Gruppen -PR₂R₃ und -PR₁₀R₁₁ unabhängig voneinander einen Rest der Formel II, lla, llb oder llc 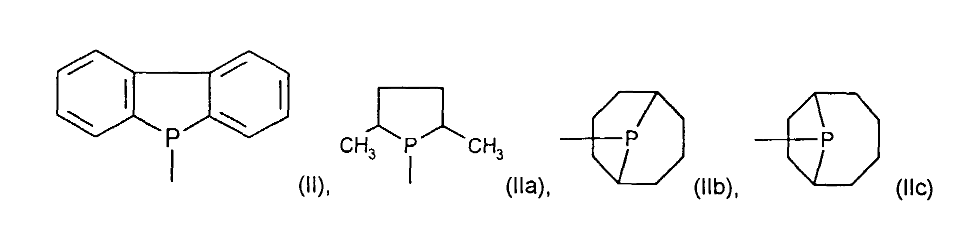
darstellen, und
R₄, R₅ und R₆ unabhängig voneinander für C₁-C₁₂-Alkyl oder Phenyl stehen;
R₇ und R₈ H, C₁-C₁₂-Alkyl, Phenyl oder
R₇ und R₈ zusammen Tetramethylen, Pentamethylen oder 3-Oxa-1,5-Pentylen darstellen,
R₉ H oder C₁-C₄-Alkyl bedeutet;
die R₁₂ gleiche oder verschiedene Reste darstellen und unabhängig voneinander C₁-C₁₂-Alkyl, C₃-C₇-Cycloalkyl, Benzyl oder Phenyl bedeuten oder zusammen C₅-C₁₂-Alkylen bedeuten und
R₁₃ C₁-C₁₂-Alkylen oder Phenylen ist,
M für H oder ein Alkalimetall steht,
X⁻ das Anion einer einbasischen Säure ist,
Z für Cl, NH₂, NH-C₁-C₁₂-Alkyl, oder für eine Gruppe -A-CO-NH-R₁₄-Si(Rₐ)ₙ (OR₁₅)₃₋ₙ steht, wobei
A NH oder N(C₁-C₁₂ -Alkyl) ist,
R₁₄ C₁-C₁₂-Alkylen bedeutet,
R₁₅ für C₁-C₁₂ Alkyl steht,
Rₐ C₁-C₄-Alkyl oder OR₁₅ ist,
n 0, 1 oder 2 ist;
und die Verbindungen der Formel I in Form ihrer Racemate und Diastereomeren oder Gemischen von Diastereomeren vorliegen.

R₁ in der Bedeutung von Alkyl ist vorzugsweise linear. Es enthält bevorzugt 1 bis 4 C-Atome. Beispiele für dieses Alkyl sind Methyl, Ethyl, n- und i-Propyl, n-, i- und t-Butyl, Pentyl, Hexyl, Heptyl und Octyl. Bevorzugt sind Methyl und Ethyl und besonders bevorzugt ist Methyl.

R₁ enthält als substituiertes Phenyl bevorzugt 1 oder 2 Substituenten. Alkylsubstituenten können zum Beispiel Methyl, Ethyl, n- und i-Propyl, n-, i- und t-Butyl sein; bevorzugt sind Methyl und Ethyl. Alkoxysubstituenten können zum Beispiel Methoxy, Ethoxy, n- und i-Propoxy, n-, i- und t-Butoxy sein; bevorzugt sind Methoxy und Ethoxy. In einer Gruppe von Verbindungen der Formel I steht R₁ vorzugsweise für Phenyl oder mit 1 oder 2 C₁-C₄-Alkyl oder C₁-C₄-Alkoxy substituiertes Phenyl.

R₂, R₃, R₁₀ und R₁₁ in der Bedeutung von Alkyl können linear oder verzweigt sein und sie enthalten bevorzugt 1 bis 8 und besonders bevorzugt 1 bis 4 C-Atome. Beispiele für dieses Alkyl sind Methyl, Ethyl, n- und i-Propyl, n-, i- und t-Butyl, Pentyl, Hexyl, Heptyl, Octyl, Nonyl, Decyl, Undecyl und Dodecyl. Bevorzugt sind Methyl, Ethyl, n- und i-Propyl, n-, i- und t-Butyl. Wenn R₂, R₃ und/oder R₁₀ und R₁₁ gleich sind, bedeuten sie als Alkyl besonders bevorzugt i-Propyl oder t-Butyl.

R₂, R₃, R₁₀ und R₁₁ in der Bedeutung von Cycloalkyl enthalten bevorzugt 5 bis 8, besonders bevorzugt 5 oder 6 Ring-C-Atome. Beispiele für Cycloalkyl sind Cyclopentyl, Cyclohexyl, Cycloheptyl, Cyclooctyl, Cyclodecyl und Cyclododecyl. Bevorzugt sind Cyclopentyl und Cyclohexyl und besonders bevorzugt ist Cyclohexyl.

Das Cycloalkyl kann substituiert sein, zum Beispiel mit 1 bis 3 Alkyl- oder Alkoxy-Substituenten, Beispiele für solche Substituenten sind zuvor angegeben worden. Bevorzugt sind Methyl und Ethyl sowie Methoxy und Ethoxy. Beispiele für substituiertes Cycloalkyl sind Methyl- und Methoxycyclopentyl und -cyclohexyl.

R₂, R₃, R₁₀ und R₁₁ können unabhängig voneinander unsubstituiertes oder substituiertes Phenyl bedeuten. In der Bedeutung von substituiertem Phenyl enthalten sie bevorzugt 1 2, oder 3 Substituenten. Sofern das Phenyl 2 oder 3 Substituenten enthält, können diese gleich oder verschieden sein.

Beispiele für die Substituenten Alkyl und Alkoxy sind zuvor angegeben worden; bevorzugte Alkyl- und Alkoxysubstituenten für Phenyl sind Methyl, Ethyl sowie Methoxy und Ethoxy.

Wenn der Phenyl-Substituent Halogen bedeutet, so handelt es sich bevorzugt um -F, -Cl und -Br.

Wenn der Phenylsubstituent C₁-C₅ -Fluoralkyl bedeutet, handelt es sich um ganz oder teilweise fluoriertes C₁-C₅-Alkyl. Beispiele hierfür sind die Stellungsisomeren von Mono- bis Decafluorpentyl, Mono- bis Octafluorbutyl, Mono- bis Hexafluorpropyl, Mono- bis Tetrafluorethyl sowie Mono- und Difluormethyl. Unter den teilweise fluoriererten Alkylresten sind solche der Formeln -CF₂H und -CF₂(C₁-C₄-Alkyl) besonders bevorzugt. Besonders bevorzugt ist ein perfluoriertes Alkyl. Beispiele hierfür sind Perfluorpentyl, Perfluorbutyl, Perfluorpropyl, Perfluorethyl und insbesondere Trifluormethyl. Die mit Fluor substituierten Alkylgruppen sind bevorzugt in den 3-, 4- und 5- Stellungen gebunden.

R₄, R₅ und R₆ können lineares oder verzweigtes Alkyl sein, das bevorzugt 1 bis 8 und besonders bevorzugt 1 bis 4 C-Atome enthält. Beispiele für Alkyl sind zuvor angegeben worden. Bevorzugtes Alkyl ist Methyl, Ethyl, n-Propyl, n-Butyl und t-Butyl. Besonders bevorzugt steht der Substituent -SiR₄R₅R₆ für Trimethylsilyl.

Unter den sauren Phenyl-Substituenten -SO₃M, -CO₂M und -PO₃M ist die Gruppe -SO₃M bevorzugt. M steht bevorzugt für H, Li, Na und K.

R₇ und R₈ enthalten als Alkyl bevorzugt 1 bis 6, besonders bevorzugt 1 bis 4 C-Atome. Das Alkyl ist bevorzugt linear. Bevorzugte Beispiele sind Methyl, Ethyl, n-Propyl und n-Butyl. R₉ stellt als Alkyl bevorzugt Methyl dar.
X⁻ stellt als Anion einer einbasischen Säure bevorzugt Cl⁻, Br⁻ oder das Anion einer Carbonsäure dar, zum Beispiel Formiat, Acetat, Trichloracetat oder Trifluoracetat dar.

Bevorzugte Beispiele für R₂, R₃, R₁₀ und R₁₁ als substituiertes Phenyl sind 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2-oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2-oder 4-(Dimethylamino)-, 2- oder 4-SO₃H-, 2- oder 4-SO₃Na-, 2- oder 4-[⁺NH₃Cl]-, 3,4,5-Trimethylphen-1-yl, 2,4,6-Trimethylphen-1-yl, 4-Trifluomethyl-phenyl oder 3,5-Di-(trifluormethyl)-phenyl.

Wenn R₂ und R₃ gleich sind, bedeuten R₂ und R₃ bevorzugt Phenyl, Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl.

Wenn R₂ und R₃ verschieden sind, bedeuten R₂ bevorzugt Phenyl und R₃ bevorzugt Cyclohexyl, 2- oder 4-Methylphen-1-yl, 2- oder 4-Methoxyphen-1-yl, 4-(Dimethylamino) phen-1-yl, 3,5-Dimethyl-4-(dimethylamino)phen-1-yl, 3,5-Dimethyl-4-methoxyphen-1-yl oder 4-t-Butylphen-1-yl.

In einer bevorzugten Ausführungsform bedeuten R₂ und R₃ gleiche Reste und bedeuten Cyclohexyl oder Phenyl.

In einer besonders bevorzugten Ausführungsform bedeuten in Formel I R₁ Methyl und R₂ und R₃ je Cyclohexyl oder Phenyl.

Wenn R₁₀ und R₁₁ gleich sind, bedeuten R₁₀ und R₁₁ bevorzugt Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl, besonders bevorzugt aber Cyclohexyl, 4-Methylphen-1-yl und t-Butyl.

Wenn R₁₀ und R₁₁ verschieden sind, bedeuten R₁₀ bevorzugt Phenyl und R₁₁ bevorzugt Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl.

Bei einer besonders bevorzugten Gruppe von Verbindungen der Formel I bedeutet R₁ Methyl und R₂ und R₃ je Cyclohexyl oder Phenyl und R₁₀ und R₁₁ stehen für Phenyl, Cyclohexyl oder t-Butyl.

Bevorzugt bedeutet R₁₂ C₁-C₄-Alkyl, besonders bevorzugt Methyl. R₁₃ steht bevorzugt für C₃-C₆ Alkylen, besonders bevorzugt für Propylen.

Bevorzugt bedeutet R₁₄ C₁-C₆-Alkylen.

Bevorzugt ist R₁₅ C₁-C₄-Alkyl, besonders bevorzugt Methyl.

Bevorzugt bedeutet Rₐ OR₁₅ .

Die Erfindung betrifft auch ein Verfahren zur Herstellung von Verbindungen der Formel I, das dadurch gekennzeichnet ist, dass man in einem ersten Schritt 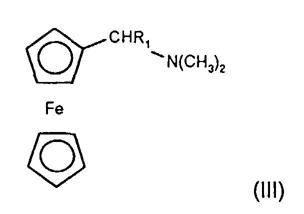
a) Verbindungen der Formel III in einem inerten organischen Lösungsmittel in Gegenwart eines Amin-Komplexbildners für Lithium in bekannter Weise mit Butyl-Lithium lithiiert, anschliessend das Reaktionsprodukt mit einem Gemisch der Verbindungen der Formel IV CIPR₁₀ R₁₁ (IV) und V CISi(R₁₂)₂-(R₁₃)-CI (V) zu Verbindungen der Formel VI 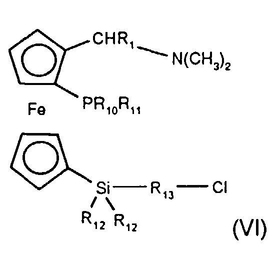 umsetzt; in einem zweiten Schritt
b) Verbindungen der Formel VI in einem organischen Lösungsmittel mit Verbindungen der Formel VII HPR₂ R₃ (VII) zu Verbindungen der Formel la 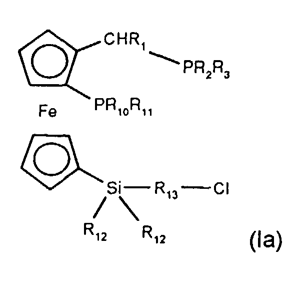 umsetzt;
c) Verbindungen der Formel la mit Verbindungen der Formel VIII NH₂(C₁-C₁₂-Alkyl) (VIII) zu Verbindungen der Formel Ib 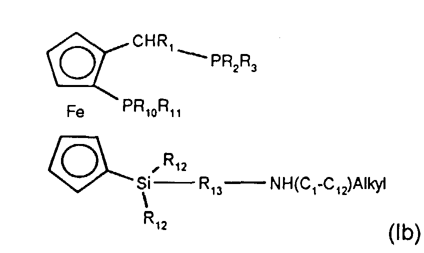 oder Verbindungen der Formel la zunächst mit K-Phthalimid und anschliessend mit Hydrazin zu Verbindungen der Formel Ic 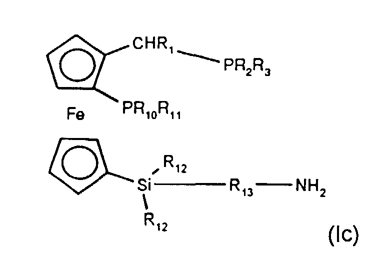 umsetzt und gegebenenfalls in einem weiteren Schritt
d) Verbindungen der Formel Ib oder Ic mit
   einer Verbindung der Formel IX (Rₐ)ₙ(R₁₅O)₃₋ₙSi-R₁₄-NCO (IX) zu Verbindungen der Formel Id 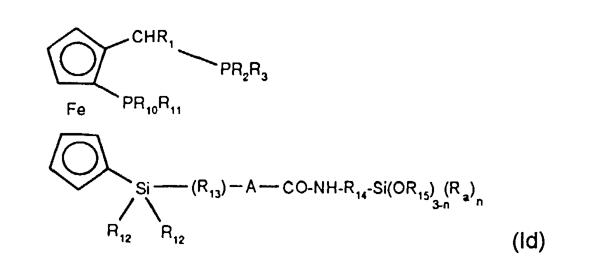 umsetzt, wobei die Reste Rₐ, R₁, R₂, R₃, R₁₀, R₁₁, R₁₂, R₁₃, R₁₄, R₁₅, A und n die vorstehend angegebenen Bedeutungen haben, einschliesslich der bevorzugten Ausführungsformen.

Ein Beispiel für einen Amin Komplexbildner für Li ist N,N,N,N-Tetramethylethylendiamin

Die Verbindungen der Formel III, IV, V, VII, VIII und IX sind bekannt und teilweise im Handel erhältlich. Sie können ansonsten nach den in der Literatur beschriebenen Verfahren hergestellt werden.

Die Verbindungen der Formeln VI, Ia, Ib, Ic und Id sind neu, Gegenstand der Erfindung und werden von der Formel I umfasst. Sie stellen wichtige Zwischenverbindungen für an anorganischen oder organischen polymeren Trägermaterialien immobilisierbare Ferrocenyldiphosphine sowie deren Rhodium und Iridium Komplexe dar.

Das Verfahren zu ihrer Herstellung ist insbesondere im Reaktionsschritt a) ebenfalls neu und Gegenstand der Erfindung. Die Reaktionsschritte b), c) und d) stellen Analogieverfahren dar, die beispielsweise für b) in der EP-A-612 758, für d) in der EP-A-496 699 beschrieben sind. Der Schritt c) ist dem Fachmann aus gängigen Lehrbüchem der organischen Chemie bekannt.

Bevorzugt liegt das Gemisch von Verbindungen der Formel IV und V im Reaktionsschritt a) in einem Molverhältnis von 1:10 bis 10:1 vor, besonders bevorzugt von 1:1 bis 10:1.

Bevorzugt wird der Reaktionsschritt a) bei einer Temperatur von -40°C bis +70°C durchgeführt , besonders bevorzugt wird das Gemisch der Verbindungen der Formel IV und V bei einer Temperatur von 0° C bis -40° C zugegeben, ganz besonders bevorzugt bei einer Temperatur von 0° C bis -15° C.

Besonders bevorzugt ist die Verbindung der Formel V im Reaktionsschritt a) 1-(Dimethylchlorsilyl)-3-Chlorpropan.

Der Reaktionsschritt b) ist beispielsweise in der EP-A-612 758 beschrieben. Die Reaktionstemperatur im Schritt b) kann zum Beispiel 20 bis 150 °C, bevorzugt 40 bis 100 °C betragen. Als Lösungsmittel eignen sich polare protische und aprotische Lösungsmittel, die alleine oder in Mischung von zwei oder mehreren Lösungsmitteln verwendet werden können. Einige Beispiele für Lösungsmittel sind Alkanole wie zum Beispiel Methanol und Ethanol, und Carbonsäuren wie zum Beispiel Ameisensäure und Essigsäure.

Die Verbindungen der Formel la bis Id werden als Racemate, reine Enantiomere oder Gemische von Enantiomeren erhalten. Racemate und Gemische von Enantiomeren können mittels bekannter Methoden in die Stereoisomeren getrennt werden, wobei im allgemeinen chromatographische Methoden bevorzugt werden.

Die Isolierung und Reinigung der Verbindungen der Formel I erfolgt nach an sich bekannten Methoden, zum Beispiel Destillation, Extraktion, Kristallisation und/oder chromatographischen Methoden.

In einer bevorzugten Verfahrensausführung wird im Reaktionsschritt c) Hydrazin als Hydrazinhydrat eingesetzt.

Im weiteren sind die Verfahrensbedingungen in den Beispielen näher ausgeführt.

Ein weiterer Gegenstand der Erfindung sind Metallkomplexe der Formel Xa und Xb von Rhodium oder Iridium mit den Verbindungen der Formel I 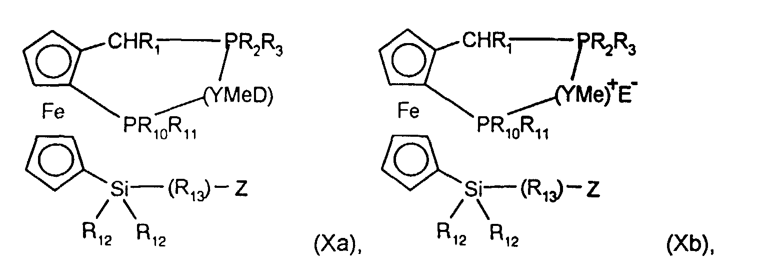
wobei R₁, R₂, R₃, R₁₀, R₁₁, R₁₂ R₁₃ und Z die vorstehend angegebenen Bedeutungen und Bevorzugungen haben;
Y für zwei Monoolefinliganden oder einen Dienliganden steht;
Me Ir oder Rh bedeutet;
D -CI, -Br, -I darstellt;
E⁻ das Anion einer Sauerstoffsäure oder Komplexsäure ist.

Bevorzugt sind Metallkomplexe, bei denen Y für 1,5-Hexadien, 1,5-Cyclooctadien oder Norbomadien steht.

Bevorzugt steht in den erfindungsgemässen Metallkomplexen D für -Cl oder -Br.

In den bevorzugten Metallkomplexen steht E⁻ für ClO₄⁻, CF₃SO₃⁻, CH₃SO₃⁻, HSO₄⁻, BF₄⁻, B(Phenyl)₄⁻, PF₆⁻, SbCl₆⁻, AsF₆⁻ oder SbF₆⁻.

Ein weiterer Gegenstand der Erfindung ist ein Verfahren zur Herstellung von Metallkomplexen, das dadurch gekennzeichnet ist, dass man Verbindungen der Formel I mit einer Metallverbindung der Formel [Me(Y)D]₂ oder Me(Y)₂⁺ E⁻ umsetzt, worin Me Rhodium oder Iridium bedeutet und Y, D und E⁻ die vorstehend angegebenen Bedeutungen und Bevorzugungen haben.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Tetrachlorkohlenstoff, Chlorbenzol), Alkanole (Methanol, Ethanol, Ethylenglykolmonomethylether), und Ether (Diethylether, Dibutylether, Ethylenglykoldimethylether) oder Gemische davon.

Die Verbindungen der Formel Xa und Xb stellen bereits homogene Katalysatoren dar, die für Hydrierungen von ungesättigten organischen Verbindungen eingesetzt werden können.

Ein weiterer Gegenstand der Erfindung sind anorganische oder organische polymere Trägermaterialien, an die Ferrocenyldiphosphine gebunden sind, die dadurch gekennzeichnet sind, dass in den 1, 2 - Stellungen des einen Cyclopentadienylrings tertiäre Phosphingruppen gebunden sind, von denen eine direkt und die andere über eine Gruppe CHR₁ am Cyclopentadienylring gebunden ist und am anderen Cyclopentadienylrest eine Silylengruppe -Si(R₁₂)₂-R₁₃-A- über das Si-Atom gebunden ist, die das eine Ende einer organischen Brückengruppe bildet und an deren anderem Ende der anorganische oder polymere organische Träger über die Gruppe A direkt oder über eine zusätzliche weitere Gruppe gebunden ist, wobei die Reste A, R₁, R₁₂ und R₁₃ die vorstehend angegebenen Bedeutungen und Bevorzugungen haben.

Die Erfindung betrifft auch ein Verfahren zur Herstellung der erfindungsgemässen Trägermaterialien, das dadurch gekennzeichnet ist, dass man ein Ferrocenyldiphosphin, das in den 1, 2 - Stellungen des einen Cyclopentadienylrings tertiäre Phosphingruppen gebunden enthält, von denen eine direkt und die andere über eine Gruppe CHR₁ am Cyclopentadienylring gebunden ist und am anderen Cyclopentadienylrest eine Silylengruppe -Si(R₁₂)₂-R₁₃-A- über das Si-Atom gebunden enthält, mit der -Si(R₁₂)₂-R₁₃-A-Gruppe
a) direkt mit den eine kovalente Bindung bildenden Gruppen eines anorganischen oder polymeren organischen Trägermaterials umsetzt; oder
b) die Gruppe -Si(R₁₂)₂-R₁₃-A- zunächst mit einer difunktionellen Brückengruppe umsetzt und diese dann mit den eine kovalente Bindung bildenden Gruppen eines anorganischen oder polymeren organischen Trägermaterials umsetzt.

Der Begriff tertiäre Phosphingruppe bezeichnet ein mit 3 Kohlenstoffatomen verbundenes Phosphoratom wie es zum Beispiel in H. Beyer Lehrbuch der organischen Chemie, S. Hirzel Verlag Leipzig, Ausgabe 1968 auf Seite 138 definiert ist.

Eine bevorzugte Gruppe wird durch ein polymeres organisches Material mit wiederkehrenden Strukturelementen der Formel Xl gebildet 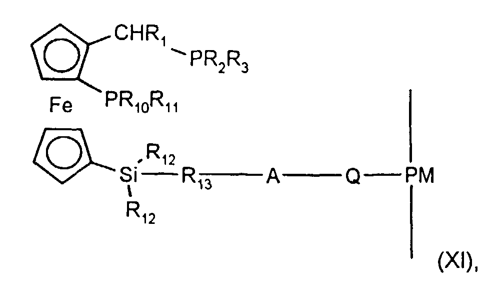 worin
A, R₁ , R₂ , R₃, R₁₀, R₁₁ , R₁₂ und R₁₃ die vorstehend angegebene Bedeutung haben; Q eine von einem Diisocyanat gebildete Brückengruppe bedeutet;
PM der Rest eines polymerbildenden Monomeren ist, welches direkt oder in einer Seitenkette eine Hydroxylgruppe oder eine Primär- oder Sekundäramingruppe als funktionelle Gruppe gebunden enthält, die über eine von einem Diisocyanat gebildete Brückengruppe Q an das Diphosphin gebunden ist.

Die Diphosphinreste der Formel I können als Enantiomerengemische vorliegen, bevorzugt sind Polymere die Reste der Formeln in Form der optisch aktiven R,R-, S,S-, R,S- oder S,R-lsomeren, bezogen auf die Stellung der Phosphin-Gruppen enthalten.

Die Wahl des Diisocyanats zur Bildung der Brückengruppe Q ist an sich unkritisch. Insbesondere kann die Brückengruppe Q durch wenigstens 2 C-Atome gebildet werden. Geeignete und grosstechnisch verfügbare Diisocyanate sind beispielsweise in Houben Weyl, Makromolekulare Stoffe, Band E 20, Seiten 1587 bis 1583 Ausgabe 1987 beschrieben.

Bevorzugt sind Diisocyanate, deren Brückengruppe Q durch ein lineares oder verzweigtes unsubstituiertes oder ein oder mehrfach mit C₁-C₆ -Alkyl, C₁-C₆ -Alkoxy substituiertes aliphatisches C₂-C₂₀ -Alkyl, unsubstituiertes oder mit C₁-C₆ -Alkyl, C₁-C₆ -Alkoxy ein oder mehrfach substituiertes C₃-C₈-Cycloalkyl oder Heterocycloalkyl, mit unsubstituiertem oder mit C₁-C₆ -Alkyl, C₁-C₆ -Alkoxy substituiertem C₃-C₈ Cycloalkyl oder Heterocycloalkyl unterbrochenes lineares oder verzweigtes unsubstituiertes oder mit C₁-C₆ -Alkyl, C₁-C₆-Alkoxy substituiertes aliphatisches C₂-C₂₀ -Alkyl, unsubstituiertes oder mit C₁-C₆ -Alkyl, C₁-C₆ -Alkoxy ein oder mehrfach substituiertes Phenyl, Naphthyl, Biphenyl oder C₃-C₁₀ Heteroaryl, durch Phenyl, Naphthyl oder C₃-C₁₀ Heteroaryl unterbrochenes lineares oder verzweigtes unsubstituiertes oder mit C₁-C₆ -Alkyl, C₁-C₆ -Alkoxy substituiertes aliphatisches C₂-C₂₀-Alkyl gebildet wird.

Heterocycloalkyl bedeutet z. B. Pyrrolidin, Piperidin, Morpholin, Oxazolidin, Dioxolan, oder eine Isocyanursäuretriestergruppe.

Heteroaryl bedeutet beispielsweise Pyridin, Pyrimidin, Pyrrol, Furan, Imidazol, Pyrazol oder Triazin.

Besonders bevorzugte Diisocyanate sind 1,6-Bis-[isocyanat]-hexan, 5-lsocyanat-3-(isocyanatmethyl)-1,1,3-trimethylcyclohexan, 1,3-Bis-[5-isocyanat-1,3,3-trimethyl-phenyl]-2,4-dioxo-1,3-diazetidin, 3,6-Bis-[9-isocyanat-nonyl]-4,5-di-(1-heptenyl)-cydohexen, Bis-[4-isocyanat-cyclohexyl]-methan, trans-1,4-Bis-[isocyanat]-cyclohexan, 1,3-Bis-[isocyanatmethyl]-benzol, 1,3-Bis-[1-isocyanat-1-methyl-ethyl]-benzol, 1,4-Bis-[2-isocyanatethyl]-cyclohexan, 1,3-Bis-[isocyanatmethyl]-cyclohexan, 1,4-Bis-[1-isocyanat-1-methylethyl]-benzol, Bis-[isocyanat]-isododecylbenzol,1,4-Bis-[isocyanat]-benzol, 2,4-Bis[isocyanat]-toluol, 2,6-Bis-[isocyanat]-toluol, 2,4-/2,6-Bis-[isocyanat]-toluol, 2-Ethyl-1,2,3-tris-[3-isocyanat-4-methyl-anilinocarbonyloxy]-propan, N,N'-Bis-[3-isocyanat-4-methylphenyl]-harnstoff, 1,4-Bis-[3-isocyanat-4-methylphenyl]-2,4-dioxo-1,3-diazetidin, 1,3,5-Tris-[3-isocyanat-4-methylphenyl]-2,4,6-trioxohexahydro-1,3,5-tdazin, 1,3-Bis-[3-isocyanat-4-methylphenyl]-2,4,5-trioxoimidazolidin, Bis-[2-isocyanatphenyl]-methan, (2-lsocyanat-phenyl)-(4-isocyanat-phenyl)-methan, Bis-[4-isocyanat-phenyl]-methan, 2,4-Bis-[4-isocyanatbenzyl]-1-isocyanatbenzol, [4-lsocyanat-3-(4-isocyanat-benzyl)-phenyl]-[2-isocyanat-5-(4-isocyanat-benzyl)-phenyl] methan, Tris-[4-isocyanat-phenyl]-methan, 1,5-Bis[isocyanat]-naphthalin, oder 4,4'-Bis[isocyanat]-3,3'-dimethyl-biphenyl.

Ganz besonders bevorzugte Diisocyanate sind 1,6-Bis-[isocyanat]-hexan, 5-lsocyanat-3-(isocyanatmethyl)-1,1,3-trimethylcyclohexan, 2,4-Bis-[isocyanat]-toluol, 2,6-Bis-[isocyanat]toluol, 2,4-/2,6-Bis-[isocyanat]-toluol oder Bis-[4-isocyanat-phenyl]-methan.

Bei den erfindungsgemässen Polymeren kann es sich um unvernetzte thermoplastische, vernetzte oder strukturvernetzte Polymere handeln.

Die Polymeren können entweder Polymerisate von olefinisch ungesättigten Monomeren sein, wie zum Beispiel Polyolefine, Polyacrylate, Polyisoprene, Polybutadien, Polystyrol, Polyphenylen, Polyvinychlorid, Polyvinylidenchlorid oder Polyallylverbindungen. Es kann sich um Polyadditionsverbindungen handeln wie zum Beispiel Polyurethane oder Polyether. Als polykondensierte Produkte sind Polyester oder Polyamide zu nennen.

Wenn die Polymeren im wesentlichen unvernetzt sind ( Thermoplaste ), kann es sich um in organischen Lösungsmitteln lösliche Polymere handeln. Teilvernetzte Polymere sind in organischen Lösungsmitteln meistens nur quellbar und bei hochvernetzten Polymeren kann es sich um unlösliche und vorteilhaft poröse Materialien handeln.

Vernetzte Polymere ( Duroplaste ) können Phenol-Aldehydharze zum Beispiel in Form von käuflichen Bakeliten®, Harnstoff- oder Melamin- Formaldehydharze, vernetzte Polyurethane oder vernetzte Epoxidharze sein.
Als Vernetzungskomponente für Epoxidharze eigenen sich insbesondere Di- oder Triamine. Möglich sind auch vernetzte Polymere auf Basis Triglycidylisocyanurat.

Es kommen auch zum Beispiel ungesättigte Polyesterharze, die sich von Copolyestern gesättigter und ungesättigter Dicarbonsäuren mit mehrwertigen Alkoholen ableiten, sowie Vinylverbindungen als Vernetzungsmittel in Frage.

Ebenso möglich sind vernetzbare Acrylharze, die sich von substituierten Acrylsäureestern ableiten, wie z.B. von Epoxyacrylaten, Urethan-acrylaten oder Polyester-acrylaten.

Eine weitere Gruppe bilden Alkydharze, Polyesterharze und Acrylatharze, die mit Melaminharzen, Harnstoffharzen, Polyisocyanaten oder Epoxidharzen vernetzt sind.

Bei den vernetzten Systemen sind solche aus olefinische ungesättigten Monomeren bevorzugt, Beispiele sind Polyacrylate, Polyolefine oder Polystyrol. Die Vernetzungskomponente ist ebenfalls olefinisch ungesättigt. Ein Beispiel ist mit Divinylbenzol vernetztes Polystyrol.

Beispiele für lineare, in organischen Lösungsmitteln lösliche Polymere sind nachfolgend angegeben.

Die erfindungsgemäss zu verwendenden Polymeren sind an sich bekannt, teilweise käuflich oder sie können mit bekannten Polymerisationsverfahren oder durch nachträgliche Modifikation von Polymeren hergestellt werden.

Bevorzugt sind am Polymeraufbau die hydroxyl-, primär- oder sekundäraminfunktionellen Monomeren von 1 bis 100 Molprozent, besonders bevorzugt von 5 bis 100 Molprozent und ganz besonders bevorzugt von 10 bis 100 Molprozent beteiligt, wenn es sich um lösliche oder quellbare Polymere handelt, bei denen die funktionelle Gruppe bereits vorhanden ist.

Handelt es sich um vernetzte Polymere, die nachträglich funktionalisiert werden so sind bevorzugt 1 bis 50 Molprozent, besonders bevorzugt 1-20 Molprozent hydroxyl-, primär- oder sekundäraminfunktionelle Gruppen vorhanden, wobei sich die Molprozentangaben auf das mehrheitlich das Polymere bildende Monomer bezieht.

Die erfindungsgemässe Beladung des Polymeren mit Ferrocenyldiphosphinen liegt bevorzugt zwischen 1 und 100 Mol-%, besonderes bevorzugt zwischen 5 und 50 Mol-%, bezogen auf die verfügbaren Hydroxyl-, Primär- oder Sekundäramingruppe des Polymeren.

Bevorzugt werden die das Polymer bildenden Monomeren ausgewählt aus der Gruppe bestehend aus Styrol, p-Methylstyrol oder α-Methylstyrol, von denen mindestens eines eine Hydroxylgruppe oder eine- Primär- oder Sekundär- Amingruppe als funktionelle Gruppe gebunden enthält.

Es können weitere Comonomere vorhanden sein, die Copolymere mit Styrolderivaten bilden, wie zum Beispiel Styrol, p-Methylstyrol oder α-Methylstyrol, Butadien, Maleinsäureanhydrid, Acrylate oder Methacrylate sowie Ethylen, Propylen oder Butylen. Es liegen dann Copolymere von z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien- Styrol, Styrol-Isopren-Styrol, Styrol-Ethylen/Butylen-Styrol oder Styrol-Ethylen/Propylen-Styrol.

Ebenfalls in Frage kommen Pfropfcopolymere von Styrol oder α-Methylstyrol, wie z.B. Styrol auf Polybutadien, Styrol auf Polybutadien-Styrol- oder Polybutadien-Acrylnitril-Copolymeren, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acryl nitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren.

Bevorzugt sind die Comonomeren von Dienen oder Acrylderivaten, wie z.B. Butadien, Acrylnitril, Alkylmethacrylat, Butadien-Alkylacrylat und -Methacrylat, Maleinsäureanhydrid, Acrylnitril-Methylacrylat, die statistische oder Block-Copolymere bilden.

Eine andere bevorzugte Gruppe von Polymeren wird von Monomeren gebildet, die sich von α,β-ungesättigten Säuren, deren Estern oder Amiden, von denen Strukturelemente eine Hydroxylgruppe oder eine Primär- oder Sekundär- Amingruppe als funktionelle Gruppe gebunden enthalten, ableiten.

Besonders bevorzugt sind die Monomeren aus der Gruppe der Acrylate und deren C₁-C₄-Alkylestern, Methacrylate und deren C₁-C₄-Alkylestern, Acrylamid und Acrylnitril, von denen Strukturelemente eine Hydroxylgruppe oder eine Primär- oder Sekundär- Amingruppe als funktionelle Gruppe in der Ester- oder Amidgruppe gebunden enthalten.

Es können auch weitere Copolymere bildende Comonomere vorhanden sein, die sich von olefinisch ungesättigten Monomeren ableiten, und die statistische Polymere oder Block-Copolymere bilden. Geeignete Comonomere sind Acrylate und deren C₁-C₄-Alkylestern, Methacrylate und deren C₁-C₄-Alkylestern, Acrylamid und Acrylnitril sowie Butadien, Vinylchlorid oder Vinylfluorid.
Eine weitere Gruppe bevorzugter Polymerer werden von Monomeren gebildet, die Vinylalkohol als Homopolymer oder Vinylalkohol als Copolymer mit Vinylacetat, -stearat,-benzoat, -maleat, Vinylbutyral, Allylphthalat, Allylmelamin enthalten.

Ebenfalls bevorzugte Polymere werden gebildet aus Phenol und einem C₁-C₄-Aldehyd, besonders bevorzugt aus Phenol und Formaldehyd. Die Polymeren sind als Phenol-Formaldehydharze, insbesondere als Novolake bekannt und im Handel erhältlich.

Eine andere bevorzugte Gruppe von Polymeren leitet sich von Bisglycidylethern und Diolen ab. Es handelt sich dabei um hydroxylfunktionelle Polyether, die zum Beispiel aus Bisgylcidylethern und Bisphenol A hergestellt werden.
Die Polyepoxide können aus Diepoxid Comonomeren mit bevorzugt 6 bis 40 und besonders bevorzugt 8 bis 30 C-Atomen, und Diolen als Comonomeren mit bevorzugt 2 bis 200 und besonders bevorzugt 2 bis 50 C-Atomen aufgebaut sein.Eine bevorzugte, davon abgleitete Gruppe wird aus Monomeren gebildet, die ein Polymeres aus cyclischen C₃-C₆-Ethern oder C₂-C₆-Alkylenglykolen mit Bisglycidylethern aufbauen. Die Bislycidylether können aromatisch, aliphatisch oder cycloaliphatisch sein.

Weitere bevorzugte Polymere mit Hydroxylgruppen als funktionellen Gruppen sind Polysaccharide.

Besonders bevorzugt sind partielle Celluloseacetate, -propionate oder -butyrate, partielle Celluloseether, Stärke, Chitin, und Chitosan.

Weitere Polymere leiten sich ab von Polymeren mit reduzierbaren Gruppen wie zum Beispiel Nitril-Gruppen, Keton-Gruppen, Carbonsäureester und Carbonsäureamide.

Es können auch im Reaktionsmedium unlösliche Polymere verwendet werden, die durch ein chemisches oder physikalisches Verfahren an der Oberfläche mit Hydroxyl oder Amingruppen funktionalisiert werden. Beispielsweise können teilweise ungesättigte Polymere durch Oxidation, z. B. mit Wasserstoffperoxid, oberflächlich mit Hydroxylgruppen versehen werden. Eine andere Möglichkeit ist die Plasmabehandlung in zum Beispiel einer Sauerstoffatmosphäre, Stickstoff- oder Ammoniakatmosphäre. Bevorzugt liegen die Polymeren als Pulver vor. Insbesondere ist unter diesen Trägermaterialien Polystyrol bevorzugt, das nachträglich nach bekannten Methoden mit Hydroxyl-, Amino- oder Hydroxymethyl-Gruppen funktionalisiert ist.

Besonders bevorzugt sind Polymere mit wiederkehrenden Strukturelementen mindestens eines Monomeren einer Verbindung der Formel Xla, Xlb Xlc oder Xld 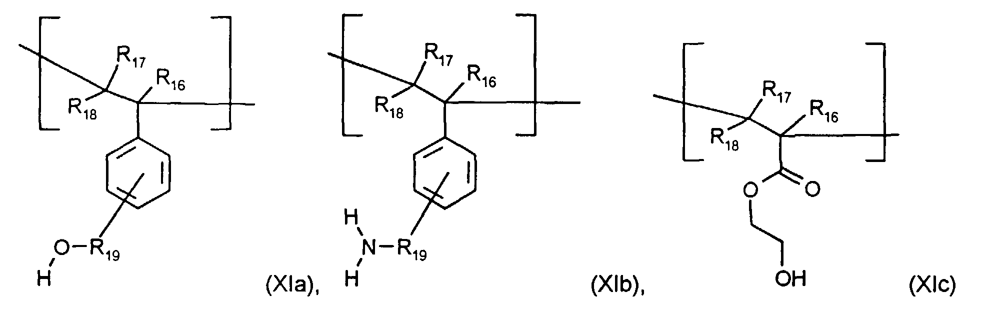 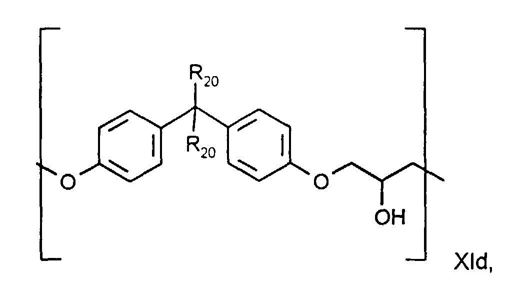 worin R₁₉ C₁-C₄-Alkylen und
R₁₆, R₁₇, R₁₈ und R₂₀ unabhängig voneinander Wasserstoff oder C₁-C₄-Alkyl sind.

Bevorzugt weisen die polymeren organische Materialien ein Molekulargewicht von 5000 bis 5 000 000 Dalton auf, besonders bevorzugt von 10 000 bis 1 000 000 oder 50 000 bis 1 000 000 Dalton.

Eine bevorzugte Untergruppe von polymeren organischen Materialien sind hochvernetztes makroporöses Polystyrol oder Polyacrylat.

Die Partikelgrösse der polymeren organischen Materialien beträgt bevorzugt 10 µm bis 2000 µm.

Bevorzugt weisen die hochvernetzten polymeren organischen Materialien eine spezifische Oberfläche 20m²/g bis 1000 m² /g auf, besonders bevorzugt 50 m² / g bis 500 m² / g bestimmt nach der BET-Methode.

Ein weiterer Gegenstand der Erfindung ist ein Verfahren zur Herstellung des erfindungsgemässen polymeren Trägermaterials, das dadurch gekennzeichnet ist, dass man Polymere mit wiederkehrenden Strukturelementen mindestens eines Monomeren, welches direkt im Polymerrückgrat oder in einer Seitenkette eine Hydroxylgruppe oder eine Primär- oder Sekundär- Amingruppe als funktionelle Gruppe gebunden enthält,
A) in einem ersten Schritt mit einem eine Brückengruppe Q bildenden Diisocyanat in einem inerten organischen Lösungsmittel ganz oder teilweise umsetzt und das Produkt in einem zweiten Schritt mit einem Diphosphin, das in den 1, 2 - Stellungen des einen Cyclopentadienylrings tertiäre Phosphingruppen gebunden enthält, von denen eine direkt und die andere über eine Gruppe CHR₁ am Cyclopentadienylring gebunden ist und am anderen Cyclopentadienylrest eine Silylengruppe -Si(R₁₂)₂-R₁₃-A- gebunden enthält, umsetzt; oder
B) in einem ersten Schritt ein Diphosphin, das in den 1, 2 - Stellungen des einen Cyclopentadienylrings tertiäre Phosphingruppen gebunden enthält, von denen eine direkt und die andere über eine Gruppe CHR₁ am Cyclopentadienylring gebunden ist und am anderen Cyclopentadienylring eine Silylengruppe -Si(R₁₂)₂-R₁₃-A- gebunden enthält ganz oder teilweise mit einem eine Brückengruppe Q bildenden Diisocyanat in einem inerten organischen Lösungsmittel umsetzt und das Produkt in einem zweiten Schritt mit einem Polymeren mit wiederkehrenden Strukturelementen mindestens eines Monomeren, welches eine Hydroxylgruppe oder eine Primär- oder Sekundär- Amingruppe als funktionelle Gruppe gebunden enthält, ganz oder teilweise umsetzt, wobei die Reste A, R₁₂ und R₁₃ die vorstehend angegebenen Bedeutungen haben und
C) gegebenenfalls noch freie Isocyanatgruppen mit einem C₂-C₂₄-Diol oder C₂-C₂₄-Diamin vernetzt oder mit einem C₂-C₁₂-Alkohol oder C₂-C₁₂-Amin abreagiert.

Auf diese Weise lässt sich das Polymer nachträglich noch in seinen Eigenschaften gezielt verändern.

Eine weitere Verfahrensvariante zur Herstellung des erfindungsgemässen polymeren Trägermaterials ist, dadurch gekennzeichnet, dass man Polymere mit wiederkehrenden Strukturelementen mindestens eines Monomeren, welches eine Hydroxylgruppe oder eine Primär- oder Sekundär- Amingruppe als funktionelle Gruppe gebunden enthält
A) in einem ersten Schritt mit einem eine Brückengruppe Q bildenden Diisocyanat in einem inerten organischen Lösungsmittel ganz oder teilweise umsetzt und das Produkt in einem zweiten Schritt mit einem Diphosphin, das in den 1, 2 - Stellungen des einen Cyclopentadienylrings tertiäre Phosphingruppen gebunden enthält, von denen eine direkt und die andere über eine Gruppe CHR₁ am Cyclopentadienylring gebunden ist und am anderen Cyclopentadienylring eine Silylengruppe -Si(R₁₂)₂-R₁₃-A- gebunden enthält ganz oder teilweise umsetzt und die noch freien Isocynatgruppen mit einem aliphatischen C₂-C₁₂-Alkohol oder C₂-C₁₂-Amin reagieren lässt.

Eine ebenfalls geeignete Verfahrensführung zur Herstellung des erfindungsgemässen polymeren Trägermaterials ist, dadurch gekennzeichnet, dass man die Polymeren mit wiederkehrenden Strukturelementen mindestens eines Monomeren, welches eine Hydroxylgruppe oder eine Primär- oder Sekundär- Amingruppe als funktionelle Gruppe gebunden enthält
A) in einem ersten Schritt mit einem eine Brückengruppe Q bildenden Diisocyanat in einem inerten organischen Lösungsmittel ganz oder teilweise umsetzt und das Produkt in einem zweiten Schritt mit einem Diphosphin, das in den 1, 2 - Stellungen des einen Cyclopentadienylrings tertiäre Phosphingruppen gebunden enthält, von denen eine direkt und die andere über eine Gruppe CHR₁ am Cyclopentadienylring gebunden ist und am anderen Cyclopentadienylring eine Silylengruppe -Si(R₁₂)₂-R₁₃-A- gebunden enthält ganz oder teilweise umsetzt und die noch freien Isocyanatgruppen mit einem aliphatischen C₂-C₂₄-Diol oder C₂-C₂₄-Diamin vernetzt.

Werden derart vernetzte Polymere hergestellt, so werden bevorzugt 0,01 bis 10 Molprozent der insgesamt vorhandenen Isocyanatgruppen vernetzt.

Die Verfahren werden bevorzugt in einem polaren oder unpolaren aprotischen Lösungsmittel durchgeführt, besonders bevorzugt ist als Lösungsmittel ein halogenierter Kohlenwasserstoff, ein Ester, ein Keton, ein Säureamid , ein Ether, Dimethylsulfoxid oder unsubstituierte oder substituierte Kohlenwasserstoffe wie zum Beispiel Xylol, Toluol, Benzol, Chlor-Benzol.

Die Umsetzung der eine Brückengruppe Q bildenden Diisocyanate mit den Amin oder Hydroxylgruppen des Polymers und des Diphosphins kann bei Raumtemperatur oder erhöhter Temperatur, zum Beispiel 30° bis 100° C nach in der Literatur bekannten Methoden erfolgen.
Die nachträgliche Einführung beispiesweise einer Hydroxy-Gruppe in hochvernetztes Polystyrol kann nach bekannten Verfahren durchgeführt werden. Es wird zunächst wie in J. Mol. Catal. 51 (1989), 13-27 beschrieben chlormethyliert und anschliessend nach der von J. M. Frechet et al. in Polymer, 20 (1979) 675-680 angegebenen Methode verseift.

Die nachträgliche Modifizierung kann auch in Masse durchgeführt werden, beispielsweise mit Plasmaverfahren. Auch chemische Verfahren in Lösung oder in Emulsion sind möglich.

Unlösliche Polymere werden zuvor nach bekannten Verfahren gemahlen und auf die gewünschte Komgrösse eingestellt.

Ein weiterer Gegenstand der Erfindung ist ein festes anorganisches Material, das dadurch gekennzeichnet ist, dass es an der Oberfläche gebundene Ferrocenyldiphosphinliganden der Formel XIII aufweist 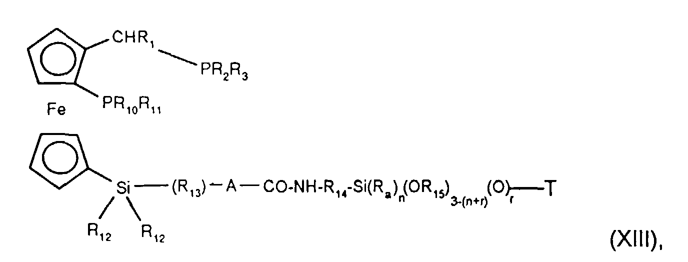
worin Rₐ, R₁, R₂, R₃, R₁₀, R₁₁, R₁₂, R₁₃, R₁₄ , R₁₅ , A und n die vorstehend angegebenen Bedeutungen haben und T ein festes anorganisches Trägermaterial darstellt; wobei
wenn n 0 ist, r für 1, 2 oder 3 steht,
wenn n 1 ist, r für 1 oder 2 steht, und
wenn n 2 ist, r für 1 steht.

Bei dem festen Trägermaterial T kann es sich um Silikate und Halbmetall- oder Metalloxide sowie um Gläser handeln, die bevorzugt als Pulver mit mittleren Teilchendurchmessem von 10 nm bis 2000 µm, bevorzugt 10 nm bis 1000 µm und insbesondere bevorzugt 10 nm bis 500 µm vorliegen. Es kann sich sowohl um kompakte als auch poröse Teilchen handeln. Poröse Teilchen weisen bevorzugt hohe innere Oberflächen auf, zum Beispiel 1 bis 1200 m², bevorzugt 30 bis 600 m². Beispiele für Oxide und Silikate sind SiO₂, TiO₂, ZrO₂, MgO, NiO, WO₃, Al₂O₃, La₂O₃, Silikagele, Tone und Zeolithe. Bevorzugte Trägermaterialien sind Silicagele, Aluminiumoxid, Titanoxid oder Glas und deren Gemische. Ein Beispiel für Gläser als Trägermaterial ist "Controlled Pore Glass", das käuflich ist.

Die Materialien der Formel XIII können durch analoge Reaktionsführung wie in der EP-A-0 496 699 beschrieben, hergestellt werden, indem man Verbindungen der Formel Id 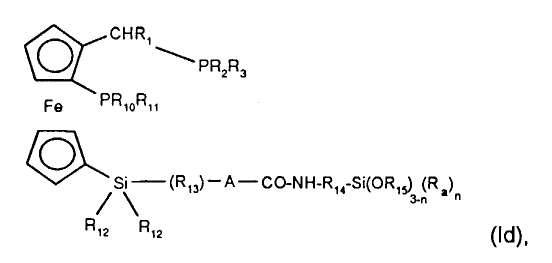 worin Rₐ, R₁, R₂, R₃, R₁₀, R₁₁, R₁₂, R₁₃, R₁₄, R₁₅, A und n die vorstehend angegebenen Bedeutungen haben, in einem inerten organischen Lösungsmittel mit einem festen anorganischen Trägermaterial T reagieren lässt, wobei vorteilhaft unter Inertgas, zum Beispiel Argon und bei einer Temperatur von 40 bis 180 °C gearbeitet wird. Zweckmässig wird das feste Material in einem Reaktionsgefäss vorgelegt, eine Lösung der Verbindung der Formel Id zugegeben und die Mischung bei erhöhter Temperatur, zum Beispiel 50 bis 110 °C gerührt. Geeignete Lösungsmittel sind zuvor erwähnt worden, besonders bevorzugte Lösungsmittel sind Toluol und Xylol. Zur Isolierung kann entweder abdekantiert, abzentrifugiert oder filtriert werden. Der Rückstand kann durch Waschen mit einem Alkanol gereinigt und dann im Hochvakuum getrocknet werden.

Ein weiterer Gegenstand der Erfindung sind Rhodium oder Iridium Metallkomplexe von anorganischen oder organischen polymeren Trägermaterialien, an die Ferrocenyldiphosphine gebunden sind, die dadurch gekennzeichnet sind, dass in den 1, 2 - Stellungen des einen Cyclopentadienylrings tertiäre Phosphingruppen gebunden sind, von denen eine direkt und die andere über eine Gruppe CHR₁ am Cyclopentadienylring gebunden ist und am anderen Cyclopentadienylrest eine Silylengruppe -Si(R₁₂)₂-R₁₃-A- über das Si-Atom gebunden ist, die das eine Ende einer organischen Brückengruppe bildet und an deren anderem Ende der anorganische oder polymere organische Träger über die Gruppe A direkt oder über eine zusätzliche weitere Gruppe gebunden ist, wobei die Reste A, R₁, R₁₂ und R₁₃ die vorstehend angegebenen Bedeutungen haben.

Bevorzugt sind Metallkomplexe von Rhodium oder Iridium mit dem polymeren organischen Material der Formel Xlla oder Xllb, 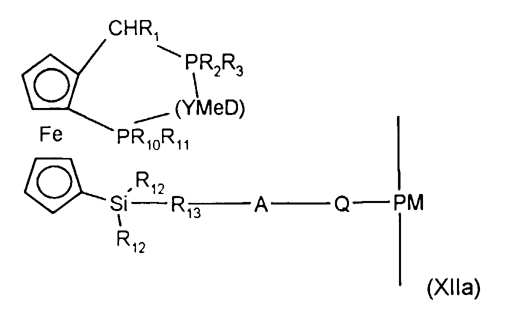 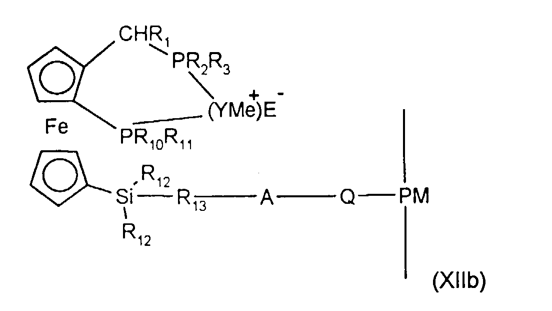 worin R₁, R₂, R₃, R₁₀, R₁₁, R₁₂, R₁₃ Q, PM, Y, Me, D und E⁻ die vorstehend angegebenen Bedeutungen und Bevorzugungen haben.

Ein weiterer Gegenstand der Erfindung ist ein Verfahren zur Herstellung von Metallkomplexen mit dem polymeren Trägermaterial, das dadurch gekennzeichnet ist, dass man Verbindungen der Formel Xl mit einer Metallverbindung der Formel [M(Y)D]₂ oder M(Y)₂⁺ E⁻ umsetzt, worin M Rhodium oder Iridium bedeutet und Y, D und E⁻ die vorstehend angegebene Bedeutung haben.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen, wenn es sich um lösliche polymer gebundene Diphosphine handelt. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Tetrachlorkohlenstoff, Chlorbenzol), Alkanole (Methanol, Ethanol, Ethylenglykolmonomethylether), und Ether (Diethylether, Dibutylether, Ethylenglykoldimethylether) oder Gemische davon.

Eine vorberechnete Menge dieser Katalysator-Lösung kann direkt für eine Hydrierreaktion verwendet werden. Durch Eindampfen des Lösungsmittels oder durch Zugabe eines Lösungsmittels, in welchem das Polymere unlöslich ist, kann der polymergebundene Katalysator auch in fester Form isoliert werden.

Es ist auch möglich den Katalysator direkt in der Hydrierlösung in situ herzustellen. Im Fall unlöslicher, teilweise oder hochvernetzter polymer gebundener Diphosphine löst man zunächst die Metallverbindungen der Formel [Me(Y)Z]₂ oder Me(Y)₂⁺ A⁻ in einem Lösungsmittel und gibt diese Lösung zu dem gelösten oder aufgeschlämten Material. Hierbei können die zuvor beschriebenen Reaktionsbedingungen angewendet werden. Das erfindungsgemässe Polymer kann entweder direkt verwendet werden oder durch Filtration isoliert und durch Waschen mit den zuvor erwähnten Lösungsmitteln gereinigt und im Vakuum getrocknet werden.

Ein anderer bevorzugter Gegenstand der Erfindung betrifft Rhodium-oder Iridiumkomplexe der Formel Xllla und Xlllb des festen anorganischen Materials der Formel XIII 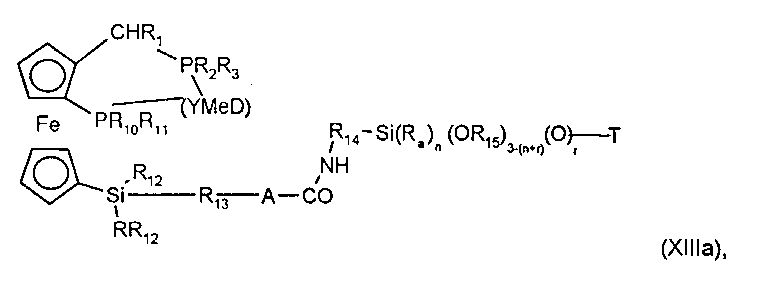 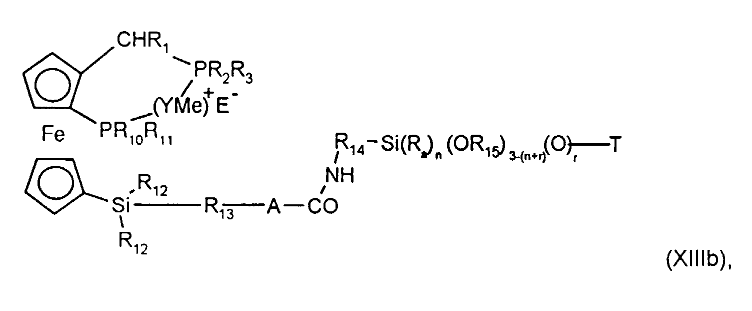 worin A, Rₐ, R₁, R₂, R₃, R₁₀, R₁₁, R₁₂, R₁₃, R₁₄, R₁₅, Y, Me, D, E, r, n und T die vorstehend angegebenen Bedeutungen und Bevorzugungen haben.

Es können die für die Herstellung von Metallkomplexen mit polymerem Trägermaterial beschriebenen Reaktionsbedingungen verwendet werden.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Tetrachlorkohlenstoff, Chlorbenzol), Alkanole (Methanol, Ethanol, Ethylenglykolmonomethylether), und Ether (Diethylether, Dibutylether, Ethylenglykoldimethylether) oder Gemische davon.

Die erfindungsgemässen anorganischen Metallkomplexe können auch vor einer Hydrierung in situ hergestellt und dann direkt als Hydrierkatalysatoren eingesetzt werden.

Die erfindungsgemässen organischen polymeren und anorganischen Metallkomplexe eignen sich hervorragend als Katalysatoren zur Hydrierung von organischen Dopel und Dreifachbindungen. Beispiel sind Verbindungen die die Gruppen C=C, C=N, C=O, C=C-N oder C=C-O enthalten ( siehe zum Beispiel K. E. König, The Applicability of Asymmetric Homogeneous Catalysis, in James D. Morrison (ed.), Asymmetric Synthesis, Vol. 5, Academic Press, 1985). Insbesondere sind die erfindungsgemässen organischen polymeren und anorganischen Metallkomplexe für die enantioselektive Hydrierung von Verbindungen mit prochiralen Kohlenstoff- und Kohlenstoff-/Heteroatomdoppelbindungen, geeignet. Beispiele für solche Verbindungen sind prochirale Alkene, Imine und Ketone. Die erfindungsgemässen Katalysatoren können nach der Reaktion in einfacher Weise, zum Beispiel durch Dekantieren, Zentrifugieren oder Filtration, praktisch vollständig vom Reaktionsgemisch abgetrennt werden.

Ein weiterer Gegenstande der Erfindung ist daher die Verwendung der erfindungsgemässen Metallkomplexe von Rhodium oder Iridium als heterogene oder homogene Katalysatoren zur asymmetrischen Hydrierung von prochiralen Verbindungen mit Kohlenstoff- oder Kohlenstoff-/Heteroatomdoppelbindungen.

Bevorzugt werden die Metallkomplexen zur asymmetrischen Hydrierung von prochiralen Verbindungen mit Kohlenstoff- oder Kohlenstoff-/Heteroatomdoppelbindungen verwendet, insbesondere die Ir-Komplexe zur Hydrierung unsymmetrischen Ketiminen.

Ein weiterer Gegenstand der Erfindung ist ein Verfahren zur asymmetrischen Hydrierung von Verbindungen mit Kohlenstoff- oder Kohlenstoff-Heteroatomdoppelbindungen, das dadurch gekennzeichnet ist, dass man die Verbindungen bei einer Temperatur von -20 bis 80 °C und einem Wasserstoffdruck von 10⁵ bis 2×10⁷ Pa in Gegenwart katalytischer Mengen eines oder mehrerer erfindungsgemässer Metallkomplexe der Formel Xlla, Xllb, Xllla und Xlllb umsetzt.

Die nachfolgenden Beispiele erläutern die Erfindung

Allgemeine Verfahrensmassnahmen bei den Synthesen:
Falls nicht anders erwähnt werden alle Reaktionen unter Inertgas und entgasten Lösungsmitteln durchgeführt. Für die Säulenchromatographie wird jeweils Kieselgel 60 der Fa. Merck verwendet.
In der experimentellen Beschreibung verwendete Abkürzungen:
TMEDA: N,N,N,N-Tetramethyläthylendiamin
BPPFA: N,N-Dimethyl-1-[1',2-bis(diphenylphosphin)ferrocenyl]ethylamin
DMF: N,N-Dimethylformamid
COD: 1,5-Cyclooctadien
Synthese der Zwischenprodukte

### Beispiel A1 Synthese von (R)-N,N-dimethyl-1-[1-(1''-dimethylsilyl-3''-chloropropyl)-(S)-2-diphenylphosphino-ferrocenyl]ethylamin (2):

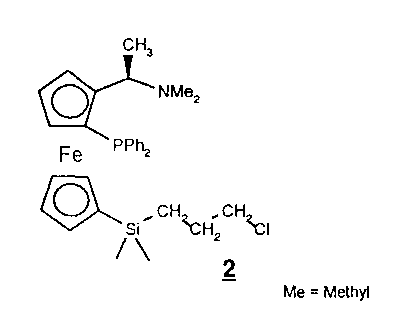

Zu einer Lösung von 10,29 g (40 mMol) (R)-N,N-Dimethyl-1-Ferrocenylethylamin in 50 ml Diethylether werden unter Rühren bei Raumtemperatur 32,5 ml einer 1,6 molaren Butyllithium - Hexan Lösung (52 mMol) zugetropft. Nach einer Stunde Rühren wird eine weitere Lösung bestehend aus 35,6 ml einer 1,6 molaren Butyllithium - Hexan Lösung (57 mMol) und 7,5 ml TMEDA (50 mMol) zugetropft und die rotbraune Reaktionslösung weitere 5 Stunden gerührt. Bei ca. -20 °C wird anschliessend eine Lösung bestehend aus einem Gemisch von 7,4 ml Chlordiphenylphosphin (40 mMol) und 22,9 ml 3-Chlorpropyl-dimethylchlorsilan (140 mMol) zugetropft. Anschliessend wird das Reaktionsgemisch über Nacht bei Raumtemperatur gerührt.
Aufarbeitung: Das Reaktionsgemisch wird bei 0 °C langsam mit 10 ml gesättigter NaHCO₃ Lösung und anschliessend 100 ml Wasser versetzt und 3 mal mit je 50 ml Essigsäureethylester ausgeschüttelt. Die organische Phase wird mit 50 ml Wasser extrahiert, mit Na₂SO₄ getrocknet und unter Vakuum [1,33-2,66 kPa (10-20 Torr)] einrotiert. Vom Rohprodukt wird dann unter Hochvakuum [1,33 Pa(ca. 0,01 Torr)] das überschüssige, hydrolisierte Chlorsilan abdestilliert (Bad-Temp. bis 70 oC). Eine grobe Säulenchromatographie (Laufmittel = Hexan / Essigsäure) liefert 15,4 g eines Gemisches der (R)-(S)-lsomeren Verbindung der Formel 2 und etwas BPPFA. Das BPPFA kann durch Auskristallisieren aus Methanol / Ethanol 1:1 entfernt werden. Es werden auf diese Weise 13,5 g mit einer Reinheit von 95% erhalten (Ausbeute 55%, rotbraunes dickflüssiges Oel). Charakterisierung:
³¹P-NMR (CDCl₃): δ -23,71 ¹H-NMR (CDCl₃): δ 0,05 (s, 3H, Si-CH₃), 0,15 (s, 3H, Si-CH₃), 0,6 (m, 2H, CH₂-Si), 1,28 (d, 3H, J 7Hz, CH-CH₃), 1,5 - 1,9 (m, 2H, CH₂-CH₂-Cl), 1,78 (s, 6H, N(CH₃)₂), 3,4 (t, 2H, J 7Hz, CH₂-Cl), 3,5 - 4,4 (m, 8H, C₅H₄FeC₅H₃CH), 7,1 - 7,7 (m, 10H, P(C₆H₅)₂).

Ausgehend von (S)-N,N-Dimethyl-1-Ferrocenylethylamin wird auf gleiche Weise die Verbindung der Formel 2 mit der (S)-(R)-Konfiguration hergestellt.

### Beispiele A2: Synthese der Verbindungen der Formel 3a, 3b, 3c:

Alle nachfolgenden Synthesen werden ausgehend von der Verbindung der Formel 2 in der (R)-(S)-Konfiguration durchgeführt und liefern die entsprechenden (R)-(S)-Liganden.

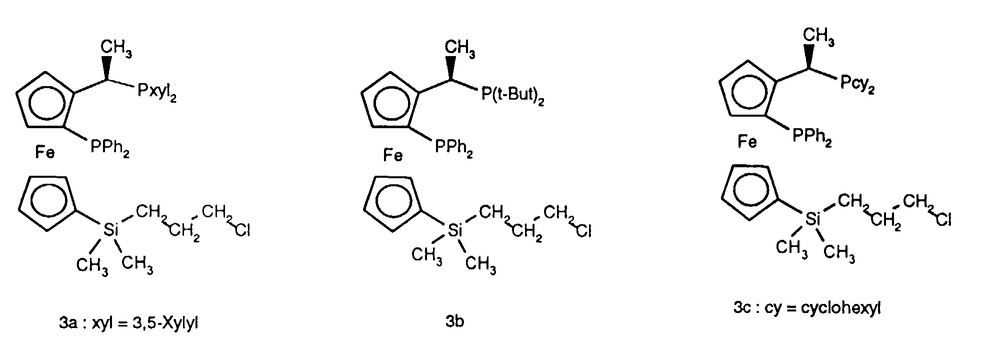

### Beispiel A2a: Synthese von (R)-1-[1'-(1''-dimethylsilyl-3''-chloropropyl)-(S)-2-diphenylphosphino-ferrocenyl]ethyldi-3,5-xyl-1-ylphosphin 3a:

Zu 2,66 g (4,6 mMol) der Beispiel A1 hergestellten Verbindung in 10 ml Essigsäure werden 1,12 g (4,6 mMol) Bis(3,5-Xylyl)phosphin in 5 ml Essigsäure zugegeben und das Gemisch 90 Minuten bei 95 °C im Oelbad gerührt. Nach Abkühlen, wird die rotbraune Lösung in 30 ml Toluol und 100 ml einer 5% wässerigen NaCl Lösung ausgeschüttelt. Die wässerige Phase wird danach mit 3 mal 15 ml Toluol ausgeschüttelt. Dann werden die organischen Phasen gesammelt, mit 50 ml Wasser gewaschen, mit Na₂SO₄ getrocknet und unter reduziertem Druck einrotiert. Das Rohprodukt wird Säulenchromatographisch gereinigt (Laufmittel: Hexan / Diethylether). Es werden 1,85 g 3a erhalten (oranges Pulver, Ausbeute 52%).
Charakterisierung:
³¹P- NMR (CDCl₃): δ - 25,46 (d, PPh₂), 6,65 (d, Pxyl₂), JPP 21Hz.

### Beispiel A2b: Synthese von (R)-1-[1'-(1''-dimethylsilyl-3''-chloropropyl)-(S)-2-diphenylphosphino-ferrocenyl]ethyldi-tert.-butylphosphin 3b.

Die Verbindung wird ausgehend von 2,18 g (3,79 mMol) 2 und 560 mg (3,8 mMol) Di-tert-butylphosphin analog Beispiel A2a hergestellt und säulenchromatographisch (Laufmittel: Hexan / Diethylether) gereinigt. Es werden 1,97 g Produkt erhalten (rotbraunes, fast festes Oel, Ausbeute 77%).
Charakterisierung:
³¹P- NMR (CDCl₃): δ - 26,6 (d, PPh₂), 50,4 (d, P(t-But)₂), JPP 54Hz.

### Beispiel A2c: Synthese von (R)-1-[1''-(1''-dimethylsilyl-3''-chloropropyl)-(S)-2-diphenylphosphino-ferrocenyl]ethyldicyclohexylphosphin 3c.

Die Verbindung wird ausgehend von 1,5 g (2,6 mMol) 2 und 0,54 ml (2,65 mMol) Dicyclohexylphosphin analog Beispiel A2a 3a hergestellt und säulenchromatographisch (Laufmittel: Hexan / Diethylether) gereinigt. Es werden 1,42 g Produkt erhalten (braunes Pulver, Ausbeute 75%).
Charakterisierung:
³¹P- NMR (CDCl₃): δ - 26,5 (d, PPh₂), 15,8 (d, Pcy₂), JPP 34Hz.

### Beispiele A3, Synthese der primären Amine der Formel 5a-c:

Die primären Amine wurden über Gabriel-Synthesen (Umsetzen des Chlorids zum Phtalimid und Freisetzen des Amins mit Hydrazinhydrat) hergestellt:

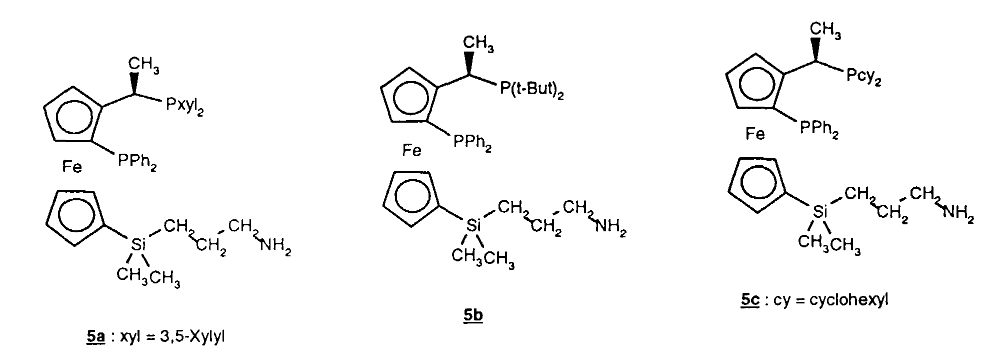

### Beispiel A3a.

Zu einer Lösung von 1,64 g (2,1 mMol) Verbindung der Formel 3a aus Beispiel A2a in 2 ml DMF werden 492 mg Kaliumphtalimid und 130 mg Hexadecyltributylphosphoniumbromid (Katalysator) gegeben und das Gemisch während 2,5 Std. bei 96 °C gerührt. Nach Abkühlen wird in Wasser / Toluol ausgeschüttelt, die organische Phase mit Natriumsulfat getrocknet und einrotiert. Nach chromatographischer Reinigung (Laufmittel: Hexan / Essigester) werden 1,8 g oranges Pulver erhalten (Ausbeute 96%).
Charakterisierung:
³¹P- NMR (CDCl₃): δ - 25,33 (d, PPh₂), 6,97 (d, Pxyl₂), JPP 22Hz. ¹H-NMR (CDCl₃): ρ charakteristische Signale 3,6 (t, 2H, J = 7, CH₂-N), 7,6 - 7,9 (m, 4H, Phtalimid).

1,8 g (2,04 mMol) des orangenen Pulvers und 0,4 ml Hydrazinhydrat in 20 ml Ethanol werden während 4 std.am Rückkfluss erhitzt. Nach Abkühlen werden 50 ml Methylenchlorid zugegeben, die Suspension filtriert und gewaschen. Die Lösung wird unter reduziertem Druck einrotiert, das Produkt nochmals mit 50 ml Methylenchlorid aufgeschlämmt und filtriert. Nach Einrotieren werden 1,5 g oranges Pulver der Verbindung der Formel 5a erhalten (Ausbeute 97%).
Charakterisierung:
³¹P- NMR (CDCl₃): δ - 25,38 (d, PPh₂), 6,6 (d, Pxyl₂), JPP 22Hz.
¹H-NMR (CDCl₃): δ charakteristische Signale 2,58 (t, 2H, J = 7, CH₂-N).

### Beispiel A3b.

Zu einer Lösung von 2 g (2,9 mMol) Verbindung der Formel 3b aus Beispiel A2b in 5 ml DMF werden 800 mg Kaliumphtalimid und 14 mg Hexadecyltributylphosphoniumbromid (Katalysator) gegeben und das Gemisch während 11 Std. bei 96 °C gerührt. Nach Abkühlen wird in Wasser / Toluol ausgeschüttelt, die organische Phase mit Natriumsulfat getrocknet und einrotiert. Nach chromatographischer Reinigung (Laufmittel: Hexan / Essigester) werden 2,15 g oranges Pulver erhalten (Ausbeute 94%).
Charakterisierung:
³¹P- NMR (CDCI₃): δ - 26,7 (d, PPh₂), 50,2 (d, P(t-But)₂), JPP 54Hz.
¹H-NMR (CDCl₃): δ charakteristische Signale3,6 (t, 2H, J = 7, CH₂-N), 7,6 - 7,9 (m, 4H, Phtalimid).

2,1 g (2,67 mMol) des orangenen Pulvers und 0,5 ml Hydrazinhydrat in 20 ml Ethanol werden während 2 std. rückflussiert. Nach Abkühlen werden 50 ml Methylenchlorid zugegeben, die Suspension filtriert und gewaschen. Die Lösung wird unter reduziertem Druck einrotiert, das Produkt mit 15 ml Diethylether aufgeschlämmt, filtriert und nochmals gewaschen. Nach Einrotieren werden 1,7 g oranges, fast festes Oel der Verbindung der Formel 5b erhalten (Ausbeute 97%).
Charakterisierung:
³¹P- NMR (CDCl₃); δ - 26,6 (d, PPh₂), 50,3 (d, P(t-But)₂), JPP 54Hz.
¹H-NMR (CDCl₃): δ charakteristische Signale2,6 (t, 2H, J = 7, CH₂-N).

### Beispiel A3c.

Zu einer Lösung von 1,4 g (1,94 mMol) Verbindung der Formel 3c aus Beispiel A2c in 3 ml DMF werden 450 mg Kaliumphtalimid und 120 mg Hexadecyltributylphosphoniumbromid (Katalysator) gegeben und das Gemisch während 1,5 Std. bei 96 °C gerührt. Nach Abkühlen wird in Wasser / Toluol ausgeschüttelt, die organische Phase mit Natriumsulfat getrocknet und einrotiert. Nach chromatographischer Reinigung (Laufmittel: Hexan / Essigester) werden 1,32 g oranges Pulver erhalten (Ausbeute 81%).
Charakterisierung:
³¹P- NMR (CDCl₃); δ - 26,5 (d, PPh₂), 15,8 (d, Pcy₂), JPP 34Hz.
¹H-NMR (CDCl₃): δ charakteristische Signale 3,58 (t, 2H, J = 7, CH₂-N), 7,6 - 7,9 (m, 4H, Phtalimid).
1,24 g (1,48 mMol) des orangenen Pulvers und 0,3 ml Hydrazinhydrat in 12 ml Ethanol werden während 2 std. am Rückfluss erhitzt. Nach Abkühlen werden 25 ml Methylenchlorid zugegeben, die Suspension filtriert und gewaschen. Die Lösung wird unter reduziertem Druck einrotiert, und das Produkt chromatographisch gereinigt (Laufmittel MeOH mit 2%Triäthylamin). Es werden 0,98 g oranges, fast festes Oel der Verbindung der Formel 5c erhalten (Ausbeute 94%).
Charakterisierung:
³¹P- NMR (CDCl₃): δ - 26,5 (d, PPh₂), 15,7 (d, Pcy₂), JPP 33Hz.
¹H-NMR (CDCl₃): δ charakteristische Signale 2,6 (t, 2H, J = 7, CH₂-N).

### Beispiel A4, Herstellung des sekundären Amins der Formel 6a:

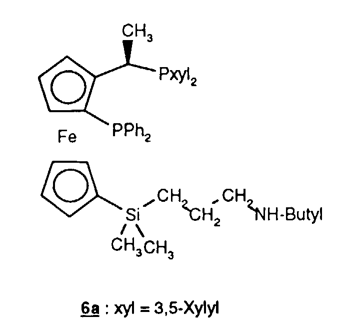

400 mg (0,518 mMol) Verbindung der Formel 3a aus Beispiel A2a werden in Gegenwart von 12 mg Tetrabutylammoniumjodid während 20 std. in 5 ml n-Butylamin am Rückfluss erhitzt. Anschliessend wird das Butylamin unter reduziertem Druck abdestilliert und das Gemisch in Wasser / Essigsäureethylester ausgeschüttelt. Die organische Phase wird mit Na2SO4 getrocknet, einrotiert und das Rohprodukt chromatographisch gereinigt (Laufmittel: Essigsäureethylester mit 2% Triäthylamin). Es werden 0,38 g oranges, zähes Oel der Verbindung der Formel 6a erhalten (Ausbeute 88%).
Charakterisierung:
³¹P- NMR (CDCl₃): δ - 25,1 (d, PPh₂), 6,8 (d, Pxyl₂), JPP 21Hz.
¹H-NMR (CDCl₃): δ charakteristische Signale 0,9 (t, 3H, J = 7, CH₂-CH₃), 2,45 - 2,6 (m, 4H, zwei CH₂-N).

### Beispiel A5, Synthese der auf anorganischen Trägern immobilisierbaren Liganden der Formel 7a-c und 8a:

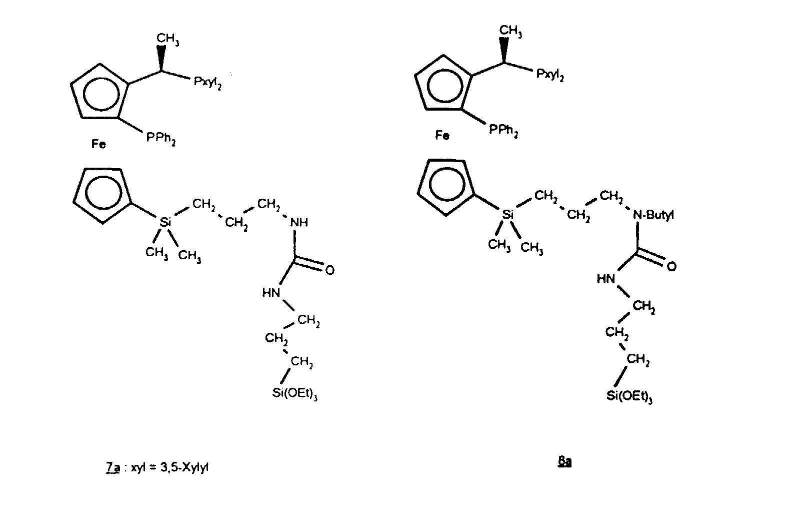

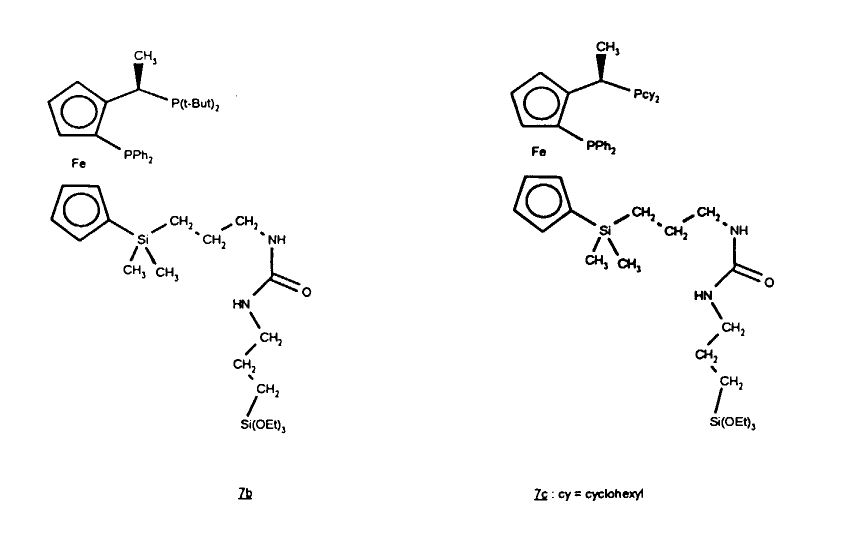

### Beispiel A5a:

Zu einer Lösung von 1,48 g (1,97 mMol) Verbindung der Formel 5a aus Beispiel A3a in 20 ml Methylenchlorid werden 0,65 ml (2,46 mMol) 1-Triethoxysilyl-3-isocyanatopropan zugetropft und das Gemisch über Nacht bei Raumtemperatur gerührt. Anschliessend wird das Lösungsmittel unter reduziertem Druck abrotiert und das Rohprodukt chromatographisch (Laufmittel: Hexan / Essigsäureethylester) gereinigt. Es werden 1,45g oranger, zäher Schaum der Verbindung der Formel 7a erhalten (Ausbeute 74%).
Charakterisierung:
³¹P- NMR (CDCl₃): δ - 25,2 (d, PPh₂), 6,7 (d, Pxyl₂), JPP 21Hz.
¹H-NMR (CDCl₃): δ 1,22 (t, J = 7, 9H, O-CH₂-CH₃), 3,81 (q, J = 7, 6H, O-CH₂).

### Beispiel A5b:

Zu einer Lösung von 602 mg (0,91 mMol) der Verbindung der Formel 5b aus Beispiel A3b in 10 ml Methylenchlorid werden 0,3 ml (1,15 mMol) 1-Triethoxysilyl-3-isocyanatopropan zugetropft und das Gemisch über Nacht bei Raumtemperatur gerührt. Anschliessend wird das Lösungsmittel unter reduziertem Druck abrotiert und das Rohprodukt chromatographisch (Laufmittel: Hexan / Essigsäureethylester) gereinigt. Es werden 600 mg oranger, zäher Schaum der Verbindung der Formel 7b erhalten (Ausbeute 72%).
Charakterisierung:
³¹P- NMR (CDCl₃): δ - 26,7 (d, PPh₂), 50,2 (d, P(t-But)₂), JPP 55Hz.
¹H-NMR (CDCl₃): δ 1,22 (t, J = 7, 9H, O-CH₂-CH₃), 3,81 (q, J = 7, 6H, O-CH₂).

### Beispiel A5c:

Zu einer Lösung von 506 mg (0,71 mMol) Verbindung der Formel 5c aus Beispiel A3c in 10 ml Methylenchlorid werden 0,24 ml (0,9 mMol) 1-Triethoxysilyl-3-isocyanatopropan zugetropft und das Gemisch über Nacht bei Raumtemperatur gerührt. Anschliessend wird das Lösungsmittel unter reduziertem Druck abrotiert und das Rohprodukt chromatographisch (Laufmittel: Essigsäureethylester) gereinigt. Es werden 530 mg oranger, zäher Schaum der Verbindung der Formel 7c erhalten (Ausbeute 72%).
Charakterisierung:
³¹P- NMR (CDCl₃): δ - 26,5 (d, PPh₂), 15,7 (d, Pcy₂), JPP 33Hz.
¹H-NMR (CDCl₃): δ 1,22 (t, J = 7, 9H, O-CH₂-CH₃), 2,95 - 3,25 (m, 4H, CH₂-NH-C(O)-NH-CH₂) 3,81 (q, J = 7, 6H, O-CH₂).

### Beispiel A5d:

Zu einer Lösung von 251 mg (0,31 mMol) Verbindung der Formel 6a aus Beispiel A4 in 4 ml Methylenchlorid werden 0,1 ml (0,37 mMol) 1-Triethoxysilyl-3-isocyanatopropan zugetropft und das Gemisch 2,5 Std. bei Raumtemperatur gerührt. Anschliessend wird das Lösungsmittel unter reduziertem Druck abrotiert und das Rohprodukt chromatographisch (Laufmittel: Hexan / Essigsäureethylester) gereinigt. Es werden 230 mg oranges, zähes Oel Verbindung der Formel 8a erhalten (Ausbeute 70%).
Charakterisierung:
³¹P- NMR (CDCl₃): δ - 25,3 (d, PPh₂), 6,7 (d, Pxyl₂), JPP 21Hz.
¹H-NMR (CDCl₃): δ charakteristische Signale 0,9 (t, 3H, J = 7, CH₂-CH₃), 1,22 (t, J = 7, 9H, O-CH₂-CH₃).

### Beispiele B, Auf Silikagel immobilisierte Liganden:

Immobilisierung: Das Trägermaterial wird jeweils vor Gebrauch bei 130 °C während 3 Std. am Hochvakuum getrocknet und anschliessend unter Argon gesetzt. Dann wird eine Lösung von immobilisierbarem Ligand aus den Beispiel A5 in Toluol zugegeben und das Gemisch während 20 Std. bei 85-90 oC gerührt. Nach Abkühlen und Absetzen lassen wird die überstehende Lösung mit einer Spritze abgezogen. Das Gemisch wird anschliessend 6 mal mit MeOH gewaschen (jeweils 7 ml pro g Täger) und schliesslich bei 40-50 °C am Hochvakuum getrocknet. Das Ergebnis ist in Tabelle 1 dargestellt.

**Tabelle 1**

| Nr. | Immobilisier barer Ligand Nr. | Menge [mg] | Träger - Typ | Menge [g] | Menge Toluol [ml] | Analytik P-Gehalt [%] | Belegung mMol Ligand pro g Träger |
|---|---|---|---|---|---|---|---|
| | | | | | | | |
| B1 | 7a | 400 | Grace 332 | 3,3 | 15 | 0,59 | 0,095 |
| B2 | 7a | 300 | Grace 332 | 6,3 | 28 | 0,25 | 0,04 |
| B3 | 7a | 120 | Grace 332 | 7,5 | 38 | 0,08 | 0,013 |
| | | | | | | | |
| B4 | 7b | 290 | Grace 332 | 3 | 13,5 | 0,62 | 0,1 |
| B5 | 7b | 181 | Grace 332 | 5 | 22,5 | 0,27 | 0,043 |
| | | | | | | | |
| B6 | 7c | 238 | Grace 332 | 2,2 | 9,8 | 0,61 | 0,098 |
| B7 | 7c | 100 | Grace 332 | 2,2 | 9,8 | 0,26 | 0,041 |
| | | | | | | | |
| B8 | 8a | 80 | Grace 332 | 2 | 9 | 0,18 | 0,028 |
| B9 | 8a | 60 | Grace 500A | 6 | 26 | 0,04 | 0,007 |

Verwendete Träger der Fa. W. R. Grace: Grace 332: spez. Oberfläche = 320 m²/g, Partikelgrösse = 35 - 70 Mikrometer. Grace 500A: Controlled Pore Glas, spez. Oberfläche = 80 m²/g, Porendurchmesser = 50 nm.

### Beispiele C. Polymer gebundene Liganden

### Beispiel C1, Immobilisierung des Liganden der Formel 5a aus Beispiel A3a auf funktionalisiertem Polystyrol .

In einem Gefäss mit Rührer, welches mit einer Fritte versehen ist, werden 930 mg am Hochvakuum bei 50 °C getrocknetes Polymer (aminomethyliertes Polystyrol, vernetzt mit 1% Divinylbenzol, Amin-Gehalt = 0,56 mMol/g, Lieferant: Novabiochem) in 35 ml Methylenchlorid gerührt bis das Trägermaterial gequollen ist. Dann werden schnell 1,2 ml (8,3 mMol) 2,4-Toluylendiisocyanat (TDI) zugegeben und das Gemisch während 1 Std. weitergerührt. Das überschüssige TDI wird anschliessend durch Abfiltrieren der Lösung und 5 mal Waschen mit 30ml Methylenchlorid entfernt. Der mit TDI umgesetzte Träger wird dann in 30 ml Methylenchlorid gerührt und es wird eine Lösung von 94 mg (0,125 mMol) der Verbindung der Formel 5a aus Beispiel A3a in 2 ml Methylenchlorid zugetropft. Das Gemisch wird über Nacht gerührt. Um die restlichen Isocyanatgruppen zu Carbamaten überzuführen, werden 10 ml Ethylalkohol mit 30 Microliter Triäthylamin als Katalysator zugegeben, und das Gemisch während 8 Std. bei 40 °C gerührt. Der gelb-orange Träger wird dann abfiltriert und 5 mal mit je 20 ml Methylenchlorid gewaschen. Schliesslich wird er am Hochvakuum getrocknet.
Analytik: P-Gehalt = 0,59%. Dies entspricht einer Belegung von 0,095 mMol Ligand pro g Träger.

### Beispiele E Herstellung von N-(2',6'-Dimethylphen-1'-yl)-N-(methoxyacetyl)-1-methoxycarbonyl-ethylamin

Einsatz der Liganden aus Beispiel B6 und B7. Hydrierung mit Rhodiumkomplexen. Allgemeine Verfahrensmassnahmen. Alle Manipulationen werden unter Inertgas durchgeführt. Die Hydrierungen werden in einem 50 ml Glaskolben mit einem Magnetrührer (1500 Upm), einem Inertgas-Anschluss und einem Gummiseptum durchgeführt. Die Reagenzien und der Wasserstoff werden mit Spritzen und Nadeln zugeführt. Die Hydrierungen der Beispiele E1 und E2 mit den Rhodiumkomplexen werden unter Wasserstoff-Normaldruck durchgeführt. Das Inertgas im Autoklaven wird vor der Hydrierung jeweils in 4 Zyklen (Vakuum, Wasserstoff-Normaldruck) durch Wasserstoff verdrängt. Die Hydrierung wird durch Einschalten des Rührers gestartet. Der Umsatz wird jeweils durch den Wasserstoffverbrauch oder mittels ¹H-NMR und die optische Ausbeute mittels HPLC (Säule: Chiracel OJ) bestimmt.

### Beispiel E1:

Zu 122 mg Ligand aus Beispiel B6 (Ligand 7c) wird eine Lösung von 4,06 mg [Rh(COD)₂]BF₄ in 3,3 ml Methanol zugegeben, und das Gemisch langsam gerührt, wobei sich die gelbe Lösung vollständig entfärbt. Dann werden 554 mg Substrat (N-(2',6'-Dimethylphen-1'-yl)-N-(methoxyacetyl)-1-methoxycarbonyl-ethenylamin) gelöst in 5 ml Methanol zugegeben, das Gemisch in einem Oelbad auf 40°C erwärmt und bei dieser Temperatur hydriert. Nach 1 Std. wird die Reaktion abgebrochen und der Wasserstoff im Hydrierkolben gegen Inertgas ausgewechselt. Man lässt den Katalysator sich setzen und zieht die überstehende Lösung mit einer Spritze ab. Der Umsatz ist vollständig und die optische Ausbeute beträgt 82,2% (R).
Wiedereinsatz: Zum abgetrennten Katalysator aus Beispiel E1 wird nochmals eine Lösung von 554 mg Substrat in 8,3 ml Methanol zugegeben, das Gemisch wieder auf 40°C erwärmt und bei dieserTemperatur hydriert. Nach 1 Std. wird die Reaktion abgebrochen und der Katalysator abfiltriert. Der Umsatz ist vollständig und die optische Ausbeute beträgt 81,7% (R). Rh-Analytik: der Rh-Gehalt in der Reaktionslösung liegt unter der Nachweisgrenze (3ppm) der benutzten Messmethode. Dies bedeutet, dass mehr als 98% des eingesetzten Katalysators zurückgewonnen wird.

### Beispiel E2:

Zu 122 mg Ligand aus Beispiel B7 wird eine Lösung von 4,06 mg [Rh(COD)₂]BF₄ in 3,3 ml Methanol zugegeben, und das Gemisch langsam gerührt, wobei sich die gelbe Lösung vollständig entfärbt. Dann werden 554 mg Substrat (N-(2',6'-Dimethylphen-1'-yl)-N-(methoxyacetyl)-1-methoxycarbonyl-ethenylamin) gelöst in 5 ml Methanol zugegeben, das Gemisch in einem Oelbad auf 40°C erwärmt und bei dieser Temperatur hydriert. Nach 1 Std. wird die Reaktion abgebrochen und der Wasserstoff im Hydrierkolben gegen Inertgas ausgewechselt. Man lässt den Katalysator sich setzen und zieht die überstehende Lösung mit einer Spritze ab. Der Umsatz ist vollständig und die optische Ausbeute beträgt 80,9% (R).
Wiedereinsatz: Zum abgetrennten Katalysator aus Beispiel E2 wird nochmals eine Lösung von 554 mg Substrat in 8,3 ml Methanol zugegeben, das Gemisch wieder auf 40°C erwärmt und bei dieserTemperatur hydriert. Nach 1 Std. wird die Reaktion abgebrochen und der Katalysator abfiltriert. Der Umsatz ist vollständig und die optische Ausbeute beträgt 80% (R). Rh-Analytik: der Rh-Gehalt in der Reaktionslösung liegt unter der Nachweisgrenze (3 ppm) der benutzten Messmethode. Dies bedeutet, dass mehr als 98% des eingesetzten Katalysators zurückgewonnen wird.

Beispiele E3 bis E5; Imin-Hydrierung mit Iridiumkomplexen.
Herstellung von N-(2'-Methyl-6'-ethyl-phen-1'yl)-N-(1-Methoxymethyl)-ethylamin

Allgemeines: Alle Manipulationen werden unter Inertgas durchgeführt. Der 50 ml Stahlautoklav ist mit einem Magnetrührer (1500 Upm) und Strömungsbrecher ausgerüstet. Das Inertgas im Autoklaven wird vor der Hydrierung jeweils in 4 Zyklen (10 bar, Normaldruck) durch Wasserstoff verdrängt. Dann wird der gewünschte Wasserstoffdruck im Autoklaven eingestellt und die Hydrierung durch Einschalten des Rührers gestartet. Der Umsatz wird jeweils durch Gaschromatographie und die optische Ausbeute mittels HPLC (Säule: Chiracel OD) bestimmt, wobei dazu eine durch Flashchromatographie (Kieselgel Merck 60, Laufmittel = Hexan / Essigsäureethylester) gereinigte Probe verwendet wird.

### Beispiel E3

Zu 260 mg (0.0118 mmol) Ligand B2 werden auf einmal eine Lösung von 3.3 mg [Ir(COD)Cl]₂ (0.0097 mMol Ir) in 2 ml THF gegeben und langsam gerührt, wobei sich die gelbe Lösung entfärbt. Dann lässt man den Katalysator absetzen, zieht mit einer Spritze das überstehende THF ab und trocknet den Katalysator am Hochvakuum. In einem zweiten Kolben werden 9.6 mg Tetrabutylammoniumjodid und schliesslich 4 g (19.5 mMol) N-(2'-Methyl-6'-ethyl-phen-1'yl)-N-(1-Methoxymethyl)-ethylimin gegeben, die Lösung unter Inertgas gesetzt und zum Katalysator gegeben. Das Reaktionsgemisch wird dann mit einer Stahlkapillare unter Inertgas-Gegenstrom in einen 50 ml Stahlautoklaven gepresst und anschliessend bei 80 bar Wasserstoffdruck bei 25 °C hydriert. Nach 2 Std. wird der Wasserstoff entspannt und der Katalysator unter Argon abfiltriert. Der Umsatz ist vollständig, die optische Ausbeute beträgt 76.8 % (S).
Wiedereinsatz:
Zum abgetrennten Katalysator werden auf einmal 4 g (19.5 mMol) N-(2'-Methyl-6'-ethylphen-1'yl)-N-(1-Methoxymethyl)-ethylimin und 9.6 mg Tetrabutylammoniumjodid gegeben. Das Reaktionsgemisch wird dann mit einer Stahlkapillare unter Inertgas-Gegenstrom in einen 50 ml Stahlautoklaven gepresst und anschliessend bei 80 bar Wasserstoffdruck bei 25 °C hydriert. Nach 2 Std. wird der Wasserstoff entspannt und der Katalysator unter Argon abfiltriert. Der Umsatz ist vollständig, die optische Ausbeute beträgt 77.1 % (S).

### Beispiel E4

Zu 130 mg (0.0059 mmol) Ligand B2 werden auf einmal eine Lösung von 1.65 mg [Ir(COD)Cl]₂ (0.0049 mMol Ir) in 2 ml THF gegeben und langsam gerührt, wobei sich die gelbe Lösung entfärbt. Dann lässt man den Katalysator absetzen, zieht mit einer Spritze das überstehende THF ab und trocknet den Katalysator am Hochvakuum. In einem zweiten Kolben werden 24 mg Tetrabutylammoniumjodid und schliesslich 10 g (19.5 mMol) N-(2'-Methyl-6'-ethyl-phen-1'yl)-N-(1-Methoxymethyl)-ethylimin gegeben, die Lösung unter Inertgas gesetzt und zum Katalysator gegeben. Das Reaktionsgemisch wird dann mit einer Stahlkapillare unter Inertgas-Gegenstrom in einen 50 ml Stahlautoklaven gepresst und anschliessend bei 80 bar Wasserstoffdruck bei 25 °C hydriert. Nach 3 Std. wird die Hydrierung abgebrochen, indem der Wasserstoff entspannt wird. Der Katalysator wird abfiltriert. Der Umsatz beträgt nach dieser Zeit 54%, die optische Ausbeute 77.9 % (S).

### Beispiel E5

60 mg (0.0059 mmol) Ligand C1 werden während 5 min. in 2 ml THF gerührt. Dann wird eine Lösung von 1.6 mg [Ir(COD)Cl]₂ (0.0049 mMol Ir) in 2 ml THF zugegeben und langsam gerührt, wobei sich die gelbe Lösung entfärbt. In einem zweiten Kolben werden 4.8 mg Tetrabutylammoniumjodid und 2 g (9.8 mMol) N-(2'-Methyl-6'-ethyl-phen-1'-yl)-N-(1-Methoxymethyl)-ethylimin gegeben, die Lösung unter Inertgas gesetzt und zum Katalysator gegeben. Das Reaktionsgemisch wird dann mit einer Stahlkapillare unter Inertgas-Gegenstrom in einen 50 ml Stahlautoklaven gepresst und anschliessend bei 80 bar Wasserstoffdruck bei 25 °C hydriert. Nach 16 Std. wird die Hydrierung abgebrochen, der Wasserstoff entspannt und der Katalysator abfiltriert. Der Umsatz beträgt nach dieser Zeit 62%, die optische Ausbeute 70.3% (S).

## Patentansprüche

1. Verbindungen der Formel I 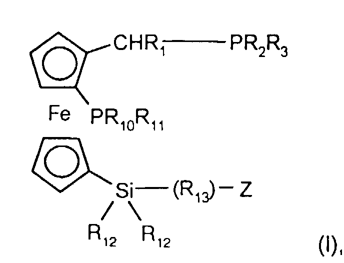 worin
R₁ C₁-C₈-Alkyl, Phenyl oder mit 1 bis 3 C₁-C₄-Alkyl oder C₁-C₄-Alkoxy substituiertes Phenyl bedeutet;
R₂, R₃, R₁₀ und R₁₁ unabhängig voneinander C₁-C₁₂-Alkyl, C₅-C₁₂-Cydoalkyl, Phenyl, mit C₁-C₄-Alkyl oder C₁-C₄-Alkoxy substituiertes C₅-C₁₂-Cydoalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ ,-[⁺NR₇R₈R₉]X⁻ oder C₁-C₅-Fluoralkyl ein oder mehrfach substituiertes Phenyl darstellen; oder die Gruppen -PR₂R₃ und -PR₁₀R₁₁ unabhängig voneinander einen Rest der Formel II, IIa, IIb oder IIc 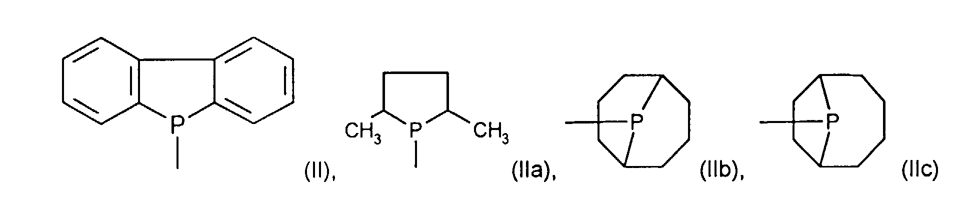 darstellen, und
R₄, R₅ und R₆ unabhängig voneinander für C₁-C₁₂-Alkyl oder Phenyl stehen;
R₇ und R₈ H, C₁-C₁₂-Alkyl, Phenyl oder
R₇ und R₈ zusammen Tetramethylen, Pentamethylen oder 3-Oxa-1,5-Pentylen darstellen,
R₉ H oder C₁-C₄-Alkyl bedeutet;
die R₁₂ gleiche oder verschiedene Reste darstellen und unabhängig voneinander C₁-C₁₂-Alkyl, C₃-C₇-Cydoalkyl, Benzyl oder Phenyl bedeuten oder zusammen C₅-C₁₂-Alkylen bedeuten und
R₁₃ C₁-C₁₂-Alkylen oder Phenylen ist,
M für H oder ein Alkalimetall steht,
X⁻ das Anion einer einbasischen Säure ist,
Z für Cl, NH₂, NH-C₁-C₁₂-Alkyl, oder für eine Gruppe -A-CO-NH-R₁₄-Si(Rₐ)ₙ (OR₁₅)₃₋ₙ steht, wobei
A NH oder N(C₁-C₁₂ -Alkyl) ist,
R₁₄ C₁-C₁₂-Alkylen bedeutet,
R₁₅ für C₁-C₁₂ Alkyl steht,
Rₐ C₁-C₄-Alkyl oder OR₁₅ ist,
n 0, 1 oder 2 ist;
und die Verbindungen der Formel I in Form ihrer Racemate und Diastereomeren oder Gemischen von Diastereomeren vorliegen.

2. Verbindungen der Formel I nach Anspruch 1, dadurch gekennzeichnet, dass R₁ als Alkyl linear ist.

3. Verbindungen der Formel I nach Anspruch 2, dadurch gekennzeichnet, dass R₁ Methyl oder Ethyl ist.

4. Verbindungen der Formel I nach Anspruch 1, dadurch gekennzeichnet, dass R₁ Phenyl oder mit 1 oder 2 C₁-C₄-Alkyl oder C₁-C₄-Alkoxy substituiertes Phenyl ist.

5. Verbindungen der Formel I nach Anspruch 1, dadurch gekennzeichnet, dass R₂, R₃, R₁₀ und R₁₁ unabhängig voneinander C₁-C₈-Alkyl darstellen.

6. Verbindungen der Formel I nach Anspruch 5, dadurch gekennzeichnet, dass R₂ und R₃ gleich sind und i-Propyl oder t-Butyl darstellen.

7. Verbindungen der Formel I nach Anspruch 1, dadurch gekennzeichnet, dass R₂, R₃, R₁₀ und R₁₁ als Cycloalkyl 5 bis 8 C-Atome enthalten.

8. Verbindungen der Formel I nach Anspruch 1, dadurch gekennzeichnet, dass R₂, R₃, R₁₀ und R₁₁ unabhängig voneinander unsubstituiertes oder mit 1, 2 oder 3 Substituenten substituiertes Phenyl bedeuten.

9. Verbindungen der Formel I nach Anspruch 8, dadurch gekennzeichnet, dass R₂ , R₃ , R₁₀ und R₁₁ als substituiertes Phenyl 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamino)-, 2-oder 4-SO₃H-, 2- oder 4-SO₃Na-, 2- oder 4-[⁺NH₃CI]-, 3,4,5-Trimethylphen-1-yl, 2,4,6-Trimethylphen-1-yl, 4-Trifluomethyl-phenyl oder 3,5-Di-(trifluormethyl)-phenyl bedeuten.

10. Verbindungen der Formel I nach Anspruch 1, dadurch gekennzeichnet, dass R₂ und R₃ gleich sind und für Phenyl, Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl stehen.

11. Verbindungen der Formel I nach Anspruch 1, dadurch gekennzeichnet, dass R₂ und R₃ gleiche Reste sind und Cyclohexyl oder Phenyl bedeuten.

12. Verbindungen der Formel I nach Anspruch 1, dadurch gekennzeichnet, dass R₁₀ und R₁₁ gleich sind und Cyclohexyl, Phenyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl bedeuten.

13. Verbindungen der Formel I nach Anspruch 1, dadurch gekennzeichnet, dass R₁ Methyl und R₂ und R₃ je Cyclohexyl oder Phenyl bedeuten und R₁₀ und R₁₁ für Phenyl, Cyclohexyl oder t-Butyl stehen.

14. Verbindungen der Formel I nach Anspruch 1, dadurch gekennzeichnet, dass R₁₂ C₁-C₄-Alkyl bedeutet.

15. Verbindungen der Formel I nach Anspruch 1, daurch gekennzeichnet, dass R₁₃ für C₃-C₆ Alkylen steht.

16. Verbindungen der Formel I nach Anspruch 1, dadurch gekennzeichnet, dass R₁₄ C₁-C₆-Alkylen ist.

17. Verbindungen der Formel I nach Anspruch 1, dadurch gekennzeichnet, dass R₁₅ C₁-C₄-Alkyl ist.

18. Verbindungen der Formel I nach Anspruch 1, dadurch gekennzeichnet, dass Rₐ OR₁₅ bedeutet.

19. Verfahren zur Herstellung von Verbindungen der Formel I nach Anspruch 1, dadurch gekennzeichnet, dass man in einem ersten Schritt 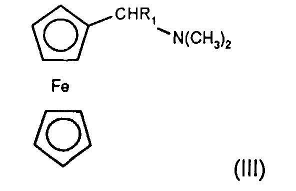
a) Verbindungen der Formel III in einem inerten organischen Lösungsmittel in Gegenwart eines Amin-Komplexbildners für Li mit Butyl-Lithium umsetzt, anschliessend das Reaktionsprodukt mit einem Gemisch der Verbindungen der Formel IV ClPR₁₀ R₁₁ (IV) und V ClSi(R₁₂)₂-(R₁₃)-Cl (V) zu Verbindungen der Formel Vl 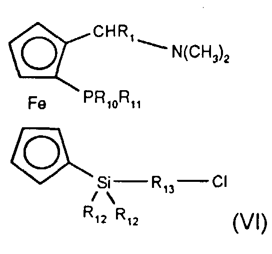 umsetzt;
in einem zweiten Schritt
b) Verbindungen der Formel VI in einem organische Lösungsmittel mit Verbindungen der Formel Vll HPR₂ R₃ (VII) zu Verbindungen der Formel la 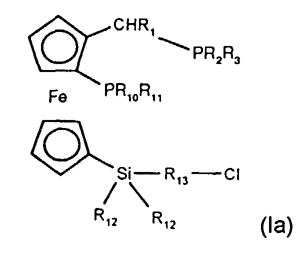 umsetzt;
c) Verbindungen der Formel la mit Verbindungen der Formel VIII NH₂(C₁-C₁₂-Alkyl) (VIII) zu Verbindungen der Formel Ib 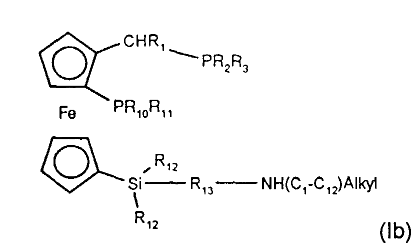 oder Verbindungen der Formel la zunächst mit K-Phthalimid und anschliessend mit Hydrazin zu Verbindungen der Formel Ic 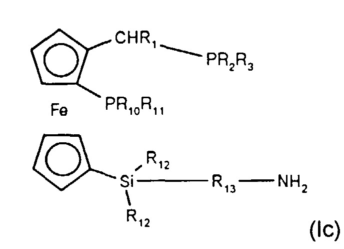 umsetzt und gegebenenfalls in einem weiteren Schritt
d) Verbindungen der Formel Ib oder Ic mit
einer Verbindung der Formel IX (Rₐ)ₙ(R₁₅O)₃₋ₙSi-R₁₄-NCO (IX) zu Verbindungen der Formel Id 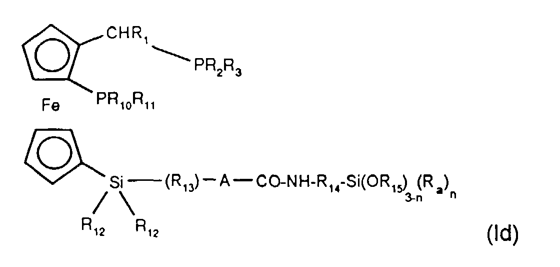 umsetzt, wobei die Reste Rₐ, R₁, R₂, R₃, R₁₀, R₁₁, R₁₂, R₁₃, R₁₄, R₁₅, A und n die in Anspruch 1 angegebenen Bedeutungen haben.

20. Verfahren nach Anspruch 19, dadurch gekennzeichnet, dass das Gemisch der Verbindungen der Formel IV CIPR₁₀ R₁₁ (IV) und V ClSi(R₁₂)₂-(R₁₃)-Cl (V) im Verfahrensschritt a) in einem Verhältnis von 1:10 bis 10:1 vorliegt.

21. Verfahren nach Anspruch 19, dadurch gekennzeichnet, dass im Verfahrensschritt a) nach der Zugabe des Gemisches der Verbindungen der Formel IV CIPR₁₀ R₁₁ (IV) und V ClSi(R₁₂)₂-(R₁₃)-Cl (V) bei einer Temperatur von 0° C bis -40° C gearbeitet wird.

22. Metallkomplexe der Formel Xa und Xb der Verbindungen der Formel I 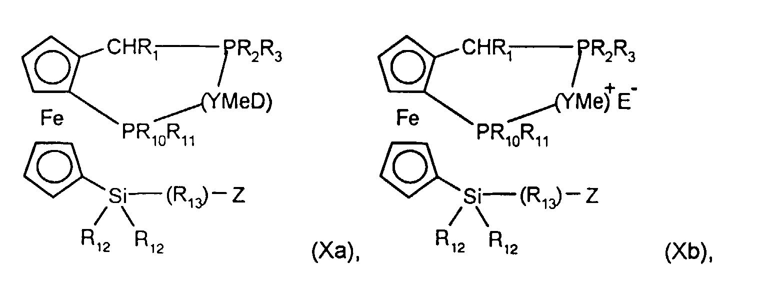
wobei R₁, R₂, R₃, R₁₀, R₁₁, R₁₂, R₁₃ und Z die in Anspruch 1 angegebenen Bedeutungen haben;
Y für zwei Monoolefinliganden oder einen Dienliganden steht;
Me Ir oder Rh bedeutet;
D -Cl, -Br, -I darstellt;
E ⁻ das Anion einer Sauerstoffsäure oder Komplexsäure ist.

23. Metallkomplexe der Formel Xa oder Xb nach Anspruch 22, dadurch gekennzeichnet, dass Y für 1,5-Hexadien, 1,5-Cyclooctadien oder Norbomadien steht.

24. Metallkomplexe der Formel Xa oder Xb nach Anspruch 22, dadurch gekennzeichnet, dass D für -Cl oder -Br steht.

25. Metallkomplexe der Formel Xa oder Xb nach Anspruch 22, dadurch gekennzeichnet, dass E⁻ für ClO₄⁻, CF₃SO₃⁻, CH₃SO₃⁻, HSO₄⁻, BF₄⁻, B(Phenyl)₄⁻, PF₆⁻, SbCl₆⁻, AsF₆⁻ oder SbF₆⁻ steht.

26. Verfahren zur Herstellung von Metallkomplexen der Formel Xa oder Xb nach Anspruch 22, dadurch gekennzeichnet, dass man Verbindungen der Formel I mit einer Metallverbindung der Formel [Me(Y)D]₂ oder Me(Y)₂⁺ E⁻ umsetzt, worin Me Rhodium oder Iridium bedeutet und Y, D und E⁻ die in Anspruch 22 angegbenen Bedeutungen haben.

27. Anorganische oder polymere organische Trägermaterialien an die Ferrocenyldiphosphine gebunden sind, dadurch gekennzeichnet, dass 2 tertiäre Phosphingruppen in den 1, 2 - Stellungen des einen Cyclopentadienylrings gebunden sind, von denen eine direkt und die andere über eine Gruppe CHR₁ am Cyclopentadienylring gebunden ist und am anderen Cyclopentadienylring eine Silylengruppe -Si(R₁₂)₂-R₁₃-A- über das Si-Atom gebunden ist, die das eine Ende einer organischen Brückengruppe bildet und an deren anderem Ende der anorganische oder polymere organische Träger über die Gruppe A direkt oder über eine weitere Brückengruppe gebunden ist, wobei die Reste A, R₁, R₁₂ und R₁₃ die in Anspruch 1 angegebene Bedeutung haben.

28. Polymeres organisches Trägermaterial nach Anspruch 27 dadurch gekennzeichnet, dass es wiederkehrende Strukturelemente der Formel XI enthält 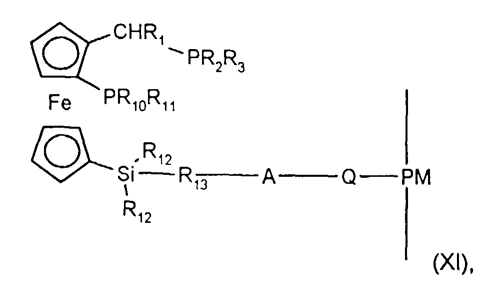 worin
A, R₁ , R₂ , R₃, R₁₀, R₁₁ , R₁₂ und R₁₃ die in Anspruch 1 angegebene Bedeutung haben; Q eine von einem Diisocyanat gebildete Brückengruppe bedeutet;
PM der Rest eines polymerbildenden Monomeren ist, welches direkt oder in einer Seitenkette eine Hydroxylgruppe oder eine Primär- oder Sekundäramingruppe als funktionelle Gruppe gebunden enthält, die über eine von einem Diisocyanat gebildete Brückengruppe Q an das Diphosphin gebunden ist.

29. Polymeres organisches Trägermaterial nach Anspruch 28, dadurch gekennzeichnet, dass die Diisocyanate für die brückenbildende Gruppe Q ausgewählt werden aus der Gruppe bestehend aus 1,6-Bis-[isocyanat]-hexan, 5-lsocyanat-3-(isocyanatmethyl)-1,1,3-trimethylcyclohexan, 1,3-Bis-[5-isocyanat-1,3,3-trimethyl-phenyl]-2,4-dioxo-1,3-diazetidin, 3,6-Bis-[9-isocyanat-nonyl]-4,5-di-(1-heptenyl)-cyclohexen, Bis-[4-isocyanat-cyclohexyl]methan, trans-1,4-Bis-[isocyanat]-cyclohexan, 1,3-Bis-[isocyanatmethyl]-benzol, 1,3-Bis-[1-isocyanat-1-methyl-ethyl]-benzol, 1,4-Bis-[2-isocyanat-ethyl]-cyclohexan, 1,3-Bis-[isocyanatmethyl]-cyclohexan, 1,4-Bis-[1-isocyanat-1-methylethyl]-benzol, Bis-[isocyanat]-isododecylbenzol,1,4-Bis-[isocyanat]-benzol, 2,4-Bis-[isocyanat]-toluol, 2,6-Bis-[isocyanat]-toluol, 2,4-/2,6-Bis-[isocyanat]-toluol, 2-Ethyl-1,2,3-tris-[3-isocyanat-4-methyl-anilinocarbonyloxy]-propan, N,N'-Bis-[3-isocyanat-4-methylphenyl]-harnstoff, 1 ,4-Bis-[3-isocyanat-4-methylphenyl]-2,4-dioxo-1,3-diazetidin, 1,3,5-Tris-[3-isocyanat-4-methylphenyl]-2,4,6-trioxohexahydro-1,3,5-triazin, 1,3-Bis-[3-isocyanat-4-methylphenyl]-2,4,5-trioxoimidazolidin, Bis-[2-isocyanatphenyl]-methan, (2-lsocyanat-phenyl)-(4-isocyanat-phenyl)-methan, Bis-[4-isocyanat-phenyl]-methan, 2,4-Bis-[4-isocyanatbenzyl]-1-isocyanatbenzol, [4-lsocyanat-3-(4-isocyanat-benzyl)-phenyl]-[2-isocyanat-5-(4-isocyanat-benzyl)-phenyl] methan, Tris-[4-isocyanat-phenyl]-methan, 1,5-Bis[isocyanat]-naphthalin, oder 4,4'-Bis[isocyanat]-3,3'-dimethyl-biphenyl.

30. Polymeres organisches Trägermaterial nach Anspruch 28, dadurch gekennzeichnet, dass die Diisocyanate für die brückenbildende Gruppe Q ausgewählt werden aus der Gruppe bestehend aus 1,6-Bis-[isocyanat]-hexan, 5-lsocyanat-3-(isocyanatmethyl)-1,1,3-trimethylcyclohexan, 2,4-Bis-[isocyanat]-toluol, 2,6-Bis-[isocyanat]-toluol, 2,4-/2,6-Bis[isocyanat]-toluol oder Bis-[4-isocyanat-phenyl]-methan.

31. Polymeres organisches Trägermaterial nach Anspruch 28, dadurch gekennzeichnet, dass es sich um unvernetzte thermoplastische, vernetzte oder strukturvernetzte Polymere handelt.

32. Polymeres organisches Trägermaterial nach Anspruch 28, dadurch gekennzeichnet, dass es sich um im wesentlichen unvernetzte in organischen Lösungsmitteln lösliche, teilvernetzte quellbare oder hochvernetzte poröse Polymere handelt.

33. Polymeres organisches Trägermaterial nach Anspruch 28, dadurch gekennzeichnet, dass das hydroxyl-, primär- oder sekundäraminfunktionelle Monomere von 1 bis 100 Molprozent am Aufbau des Polymeren beteiligt ist.

34. Polymeres organisches Trägermaterial nach Anspruch 33, dadurch gekennzeichnet, dass das hydroxyl-, primär- oder sekundäraminfunktionelle Monomere von 5 bis 100 Molprozent am Aufbau des Polymeren beteiligt ist.

35. Polymeres organisches Trägermaterial nach Anspruch 33, dadurch gekennzeichnet, dass das hydroxyl-, primär- oder sekundäraminfunktionelle Monomere von 10 bis 100 Molprozent am Aufbau des Polymeren beteiligt ist.

36. Polymeres organische Trägermaterial nach Anspruch 28, dadurch gekennzeichnet, dass 1 bis 100 Molprozent der im Polymer vorhandenen Hydroxyl-, Primär- oder Sekundäramingruppen zu Verbindungen der Formel Xl nach Anspruch 28 umgesetzt sind.

37. Polymeres organisches Trägermaterial nach Anspruch 28, dadurch gekennzeichnet, dass die das Polymer bildenden Monomeren ausgewählt werden aus der Gruppe bestehend aus Styrol, p-Methylstyrol oder α-Methylstyrol, von denen mindestens eines eine Hydroxylgruppe oder eine Primär- oder Sekundär- Amingruppe als funktionelle Gruppe gebunden enthält.

38. Polymeres organisches Trägermaterial nach Anspruch 28, dadurch gekennzeichnet, dass weitere Comonomere von Dienen oder Acrylderivaten, insbesondere Butadien, Acrylnitril, Alkylmethacrylat, Butadien-Alkylacrylat und -Methacrylat, Maleinsäureanhydrid, Acrylnitril-Methylacrylat vorhanden sind, die statistische oder Block-Copolymere bilden.

39. Polymeres organisches Trägermaterial nach Anspruch 28, dadurch gekennzeichnet, dass die das Polymer bildenden Monomeren ausgewählt werden aus der Gruppe bestehend aus α,β-ungesättigten Säuren, deren Estern oder Amiden von denen mindestens eines eine Hydroxylgruppe oder eine Primär- oder Sekundär- Amingruppe als funktionelle Gruppe gebunden enthält.

40. Polymeres organisches Trägermaterial nach Anspruch 39, dadurch gekennzeichnet, dass die das Polymer bildenden Monomeren ausgewählt werden aus der Gruppe bestehend aus Acrylaten und deren C₁-C₄-Alkylestern, Methacrylaten und deren C₁-C₄-Alkylestern, Acrylamid und Acrylnitril, von denen mindestens eines eine Hydroxylgruppe oder eine Primär- oder Sekundär- Amingruppe als funktionelle Gruppe gebunden enthält.

41. Polymeres organisches Trägermaterial nach Anspruch 39, dadurch gekennzeichnet, dass weitere olefinisch ungesättigte Monomere vorhanden sind, die statistische oder Block-Copolymere bilden.

42. Polymeres organisches Trägermaterial nach Anspruch 28, dadurch gekennzeichnet, dass das Polymer von Monomeren gebildet wird, die Vinylalkohol als Homopolymer oder Vinylalkohol als Copolymer mit Vinylacetat, -stearat, -benzoat, -maleat, Vinylbutyral, Allylphthalat, Allylmelamin enthalten.

43. Polymeres organisches Trägermaterial nach Anspruch 28, dadurch gekennzeichnet, dass die das Polymer bildenden Monomeren Phenol und ein C₁-C₄-Aldehyd sind.

44. Polymeres organisches Trägermaterial nach Anspruch 28, dadurch gekennzeichnet, dass die das Polymer bildenden Monomeren ein Polymeres aus cyclischen C₃-C₆-Ethern oder C₂-C₆-Alkylenglykolen und Bisglycidylethern bilden.

45. Polymeres organisches Trägermaterial nach Anspruch 28, dadurch gekennzeichnet, dass es Polysaccharide sind.

46. Polymeres organisches Trägermaterial nach Anspruch 45, dadurch gekennzeichnet, dass die Polysaccharide Cellulose, Celluloseacetate, -propionate oder -butyrate, Celluloseether, Stärke, Chitin, und Chitosan sind.

47. Polymeres organisches Trägermaterial nach Anspruch 28, dadurch gekennzeichnet, dass es Polymere mit wiederkehrenden Strukturelementen mindestens eines Monomeren, einer Verbindung der Formel Xla, Xlb Xlc oder Xld sind 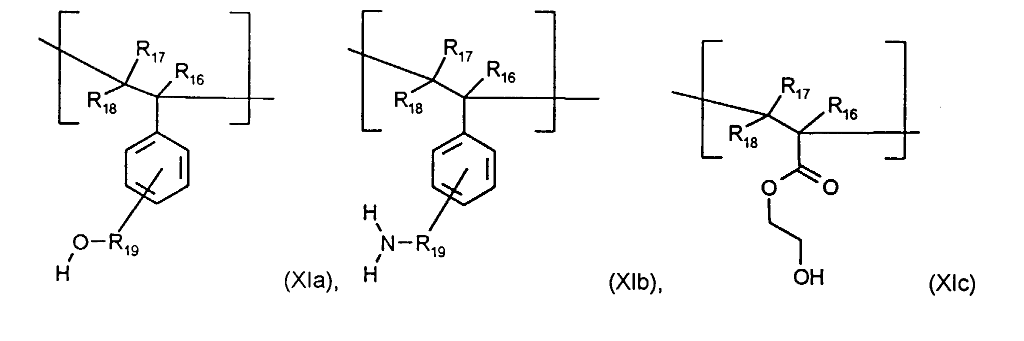 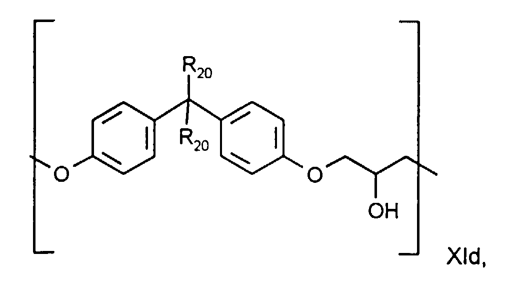 worin R₁₉ C₁-C₄-Alkylen und
R₁₆, R₁₇, R₁₈ und R₂₀ unabhängig voneinander Wasserstoff oder C₁-C₄-Alkyl sind.

48. Polymeres organisches Trägermaterial nach Anspruch 28, dadurch gekennzeichnet, dass das Molekulargewicht 5000 bis 5 000 000 Dalton beträgt.

49. Polymeres organisches Trägermaterial nach Anspruch 28 dadurch gekennzeichnet, dass das Molekulargewicht 10 000 bis 1 000 000 oder 50 000 bis 1 000 000 Dalton beträgt.

50. Polymeres organisches Trägermaterial nach Anspruch 28, dadurch gekennzeichnet, dass es sich um hochvernetztes makroporöses Polystyrol oder Polyacrylat handelt.

51. Polymeres organisches Trägermaterial nach Anspruch 50, dadurch gekennzeichnet, dass die Partikelgrösse 10 µm bis 2000 µm beträgt.

52. Polymeres organisches Trägermaterial nach Anspruch 50, dadurch gekennzeichnet, dass die spezifische Oberfläche der porösen hochvernetzten Polymeren 50 bis 500 m²/g aufweist.

53. Verfahren zur Herstellung des polymeren Trägermaterials nach Anspruch 28, dadurch gekennzeichnet, dass man die Polymeren mit wiederkehrenden Strukturelementen mindestens eines Monomeren, welches direkt im Polymerrückgrat oder in einer Seitenkette eine Hydroxylgruppe oder eine Primär- oder Sekundär- Amingruppe als funktionelle Gruppe gebunden enthält
A) in einem ersten Schritt mit einem eine Brückengruppe Q bildenden Diisocyanat in einem inerten organischen Lösungsmittel ganz oder teilweise umsetzt, und das Produkt in einem zweiten Schritt mit einem Diphosphin, das an einem Cyclopentadienylrest 2 tertiäre Phosphingruppen in den 1, 2 - Stellungen gebunden enthält, von denen eine direkt und die andere über eine Gruppe CHR₁ am Cyclopentadienylring gebunden ist, und am anderen Cyclopentadienylrest eine Silylengruppe -Si(R₁₂)₂-R₁₃-A- gebunden enthält, umsetzt; oder
B) in einem ersten Schritt ein Diphosphin, das an einem Cyclopentadienylrest 2 tertiäre Phosphingruppen in den 1, 2 - Stellungen gebunden enthält, von denen eine direkt und die andere über eine Gruppe CHR₁ am Cyclopentadienylring gebunden ist, und am anderen Cyclopentadienylrest eine Silylengruppe -Si(R₁₂)₂-R₁₃-A- gebunden enthält mit einem eine Brückengruppe Q bildenden Diisocyanat in einem inerten organischen Lösungsmittel umsetzt und das Produkt in einem zweiten Schritt mit einem Polymeren mit wiederkehrenden Strukturelementen mindestens eines Monomeren, welches eine Hydroxylgruppe oder eine Primär- oder Sekundär- Amingruppe als funktionelle Gruppe gebunden enthält, ganz oder teilweise umsetzt, wobei die Reste A, R₁, R₁₂ und R₁₃ die im Anspruch 1 angegebenen Bedeutungen haben; und
C) gegebenenfalls noch freie Isocyanatgruppen mit einem C₂-C₂₄-Diol oder C₂-C₂₄-Diamin vernetzt oder mit einem C₂-C₁₂-Alkohol oder C₂-C₁₂-Amin abreagiert.

54. Verfahren zur Herstellung des polymeren Trägermaterials nach Anspruch 28, dadurch gekennzeichnet, dass man Polymere mit wiederkehrenden Strukturelementen mindestens eines Monomeren, welches eine Hydroxylgruppe oder eine Primär- oder SekundärAmingruppe als funktionelle Gruppe gebunden enthält
A) in einem ersten Schritt mit einem eine Brückengruppe Q bildenden Diisocyanat in einem inerten organischen Lösungsmittel ganz oder teilweise umsetzt und das Produkt in einem zweiten Schritt mit einem Diphosphin, das an einem Cyclopentadienylrest 2 tertiäre Phosphingruppen in den 1, 2 - Stellungen gebunden enthält, von denen eine direkt und die andere über eine Gruppe CHR₁ am Cyclopentadienylring gebunden ist, und am anderen Cyclopentadienylrest eine Silylengruppe -Si(R₁₂)₂-R₁₃-A- gebunden enthält ganz oder teilweise umsetzt und die noch freien Isocyanatgruppen mit einem aliphatischen C₂-C₁₂-Alkohol oder C₂-C₁₂-Amin reagieren lässt, wobei die Reste A, R₁, R₁₂ und R₁₃ die im Anspruch 1 angegebenen Bedeutungen haben.

55. Verfahren zur Herstellung des polymeren Trägermaterials nach Anspruch 28, dadurch gekennzeichnet, dass man die Polymeren mit wiederkehrenden Strukturelementen mindestens eines Monomeren, welches eine Hydroxylgruppe oder eine Primär- oder Sekundär- Amingruppe als funktionelle Gruppe gebunden enthält
A) in einem ersten Schritt mit einem eine Brückengruppe Q bildenden Diisocyanat in einem inerten organischen Lösungsmittel ganz oder teilweise umsetzt und das Produkt in einem zweiten Schritt mit einem Diphosphin, das an einem Cyclopentadienylrest 2 tertiäre Phosphingruppen in den 1, 2 - Stellungen gebunden enthält, von denen eine direkt und die andere über eine Gruppe CHR₁ am Cyclopentadienylring gebunden ist, und am anderen Cyclopentadienylrest eine Silylengruppe -Si(R₁₂)₂-R₁₃-A- gebunden enthält ganz oder teilweise umsetzt und die noch freien Isocynatgruppen mit einem aliphatischen C₂-C₂₄-Diol oder C₂-C₂₄-Diamin vernetzt.

56. Verfahren zur Herstellung des polymeren Trägermaterials nach Anspruch 55 dadurch gekennzeichnet, dass 0,01 bis 10 Molprozent der insgesamt vorhandenen Isocyanatgruppen vernetzt werden.

57. Festes anorganisches Trägermaterial nach Anspruch 27, dadurch gekennzeichnet, dass es an der Oberfläche gebundene Ferrocenyldiphosphinliganden der Formel XIII aufweist 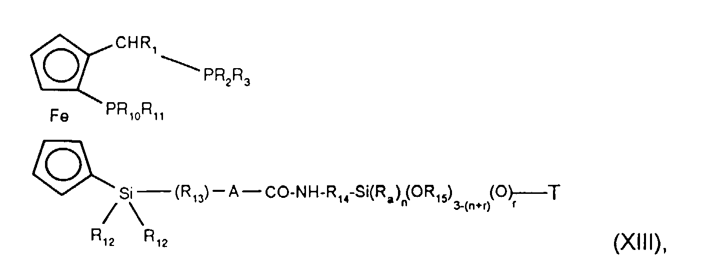
worin Rₐ, R₁, R₂, R₃, R₁₀, R₁₁, R₁₂, R₁₃, R₁₄ , R₁₅ , A und n die im Anspruch 1 angegebenen Bedeutungen haben und T ein festes anorganisches Trägermaterial darstellt; wobei
wenn n 0 ist, r für 1, 2 oder 3 steht,
wenn n 1 ist, r für 1 oder 2 steht, und
wenn n 2 ist, r für 1 steht.

58. Festes anorganisches Trägermaterial T nach Anspruch 57, dadurch gekennzeichnet, dass das anorganische Trägermaterial ausgewählt wird aus der Gruppe, bestehend aus Silikaten, Halbmetall- oder Metalloxiden und Gläsern oder Gemischen davon.

59. Verfahren zur Herstellung eines festen anorganischen Trägermaterials der Formel XIII nach Anspruch 58, dadurch gekennzeichnet, dass man Verbindungen der Formel Id 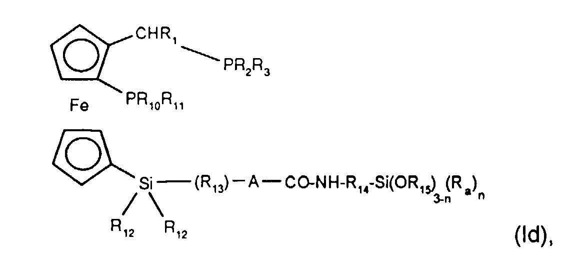 worin Rₐ, R₁, R₂, R₃, R₁₀, R₁₁, R₁₂, R₁₃, R₁₄ , R₁₅ , A und n die im Anspruch 1 angegebenen Bedeutungen haben in einem inerten organischen Lösungsmittel mit einem festen anorganischen Trägermaterial T reagieren lässt.

60. Metallkomplexe von Rhodium oder Iridium mit den anorganischen oder organischen polymeren Trägermaterialien nach Anspruch 27, an die Ferrocenyldiphosphine gebunden sind, dadurch gekennzeichnet, dass in den 1, 2 - Stellungen des einen Cyclopentadienylrings tertiäre Phosphingruppen gebunden sind, von denen eine direkt und die andere über eine Gruppe CHR₁ am Cyclopentadienylring gebunden ist und am anderen Cyclopentadienylrest eine Silylengruppe -Si(R₁₂)₂R₁₃-A- über das Si-Atom gebunden ist, die das eine Ende einer organischen Brückengruppe bildet und an deren anderem Ende der anorganische oder polymere organische Träger über die Gruppe A direkt oder über eine zusätzliche weitere Gruppe gebunden ist, wobei die Reste A, R₁, R₁₂ und R₁₃ die im Anspruch 1 angegebenen Bedeutungen haben.

61. Metallkomplexe von Rhodium oder Iridium mit dem polymeren organischen Material der Formel Xlla oder Xllb nach Anspruch 28, 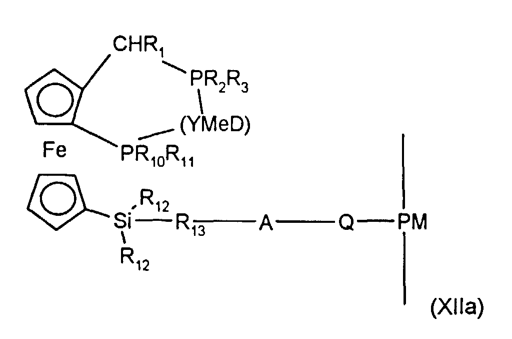 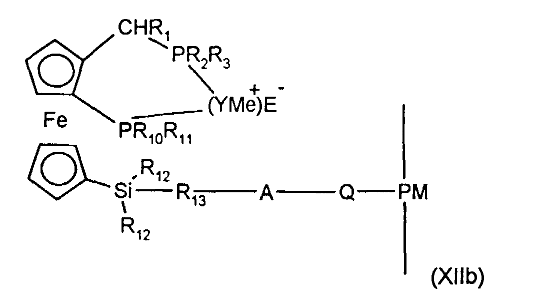 worin R₁, R₂, R₃, R₁₀, R₁₁, R₁₂, R₁₃ Q, PM, Y, Me, D und E⁻ die in Anspruch 1 und Anspruch 22 angegebenen Bedeutungen haben.

62. Verfahren zur Herstellung von Metallkomplexen nach Anspruch 61, dadurch gekennzeichnet, dass man Verbindungen der Formel Xl mit einer Metallverbindung der Formel [M(Y)D]₂ oder M(Y)₂⁺ E⁻ umsetzt, worin M Rhodium oder Iridium bedeutet und Y, D und E⁻ die in Anspruch 22 angegebenen Bedeutungen haben.

63. Rhodium-oder Iridiumkomplexe der Formel Xllla und Xlllb des festen anorganischen Trägermaterials der Formel XIII nach Anspruch 57 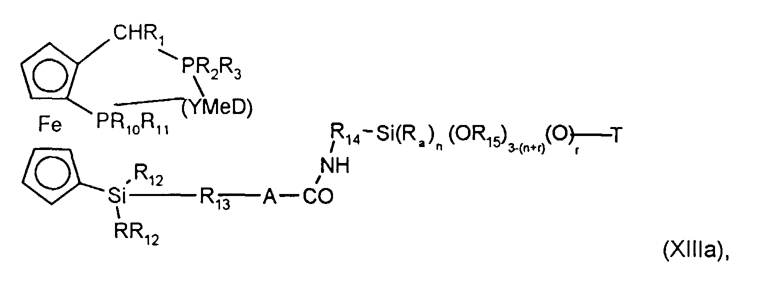 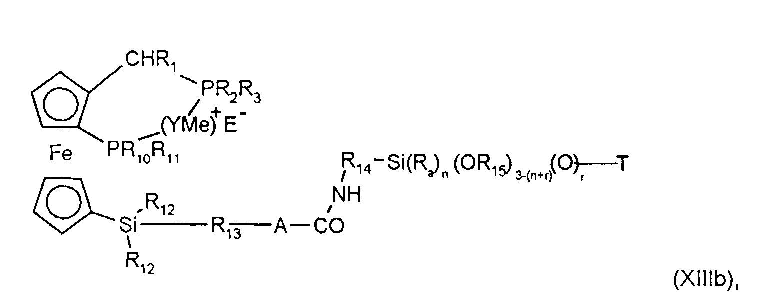 worin die Reste A, Rₐ, R₁, R₂, R₃, R₁₀, R₁₁, R₁₂, R₁₃, R₁₄ und R₁₅ die in Anspruch 1 angegebenen Bedeutungen haben; Y, Me, D, E⁻, r, n und T die in den Ansprüchen 22, 57 und 59 angegebenen Bedeutungen haben.

64. Verwendung der Metallkomplexe von Rhodium oder Iridium nach Anspruch 60 als heterogene oder homogene Katalysatoren zur asymmetrischen Hydrierung von prochiralen Verbindungen mit Kohlenstoff- oder Kohlenstoff-/Heteroatomdoppelbindungen.

65. Verwendung der Metallkomplexe nach Anspruch 64 zur Hydrierung unsymmetrischer Ketimine.

66. Verfahren zur asymmetrischen Hydrierung von Verbindungen mit Kohlenstoff- oder Kohlenstoff-Heteroatomdoppelbindungen, dadurch gekennzeichnet, dass man die Verbindungen bei einer Temperatur von -20 bis 80 °C und einem Wasserstoffdruck von 10⁵ bis 2x10⁷ Pa in Gegenwart katalytischer Mengen eines oder mehrerer erfindungsgemässer Metallkomplexe der Formel Xlla, Xllb, Xllla und Xlllb nach Anspruch 61 oder Anspruch 63 umsetzt.

## Claims

1. A compound of the formula I 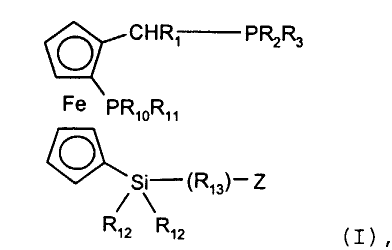 in which
R₁ is C₁-C₈alkyl, phenyl or phenyl substituted by from 1 to 3 C₁-C₄alkyl or C₁-C₄alkoxy groups;
R₂, R₃, R₁₀ and R₁₁, are independently of one another, C₁-C₁₂alkyl, C₅-C₁₂cycloalkyl, phenyl, C₅-C₁₂cycloalkyl substituted by C₁-C₄alkyl or C₁-C₄alkoxy, or phenyl substituted by from one to three C₁-C₄alkyl, C₁-C₄alkoxy, -SiR₄R₅R₆, halogen, -SO₃M, -CO₂M, -PO₃M, -NR₇R₈, -[⁺NR₇R₈R₉]X⁻ or C₁-C₅fluoroalkyl groups; or the groups -PR₂R₃ and -PR₁₀R₁₁ are each, independently of one another, a radical of the formula II, IIa, IIb or IIc 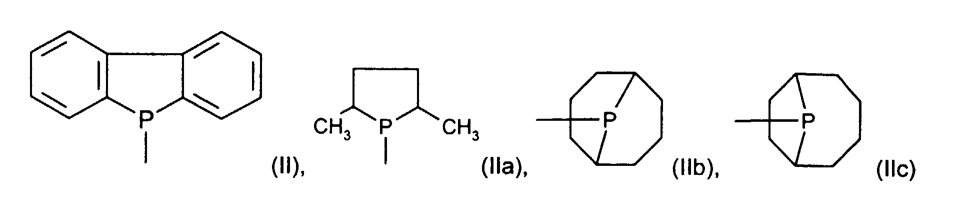 and
R₄, R₅ and R₆ are, independently of one another, C₁-C₁₂alkyl or phenyl;
R₇ and R₈ are H, C₁-C₁₂alkyl, phenyl or
R₇ and R₈ are together tetramethylene, pentamethylene or 3-oxa-1,5-pentylene,
R₉ is H or C₁-C₄alkyl;
R₁₂ are identical or different radicals and are, independently of one another, C₁-C₁₂alkyl, C₃-C₇cycloalkyl, benzyl or phenyl or together are C₅-C₁₂alkylene and
R₁₃ is C₁-C₁₂alkylene or phenylene,
M is H or an alkali metal,
X⁻ is the anion of a monobasic acid,
Z is Cl, NH₂, NH-C₁-C₁₂alkyl, or a group -A-CO-NH-R₁₄-Si(Rₐ)ₙ (OR₁₅)₃₋ₙ,
in which
A is NH or N(C₁-C₁₂alkyl),
R₁₄ is C₁-C₁₂alkylene,
R₁₅ is C₁-C₁₂alkyl,
Rₐ is C₁-C₄alkyl or OR₁₅,
n is 0, 1 or 2;
and the compound of the formula I is in the form of its racemates and diastereomers or a mixture of diastereomers.

2. A compound of the formula I according to claim 1, wherein R₁ is linear alkyl.

3. A compound of the formula I according to claim 2, wherein R₁ is methyl or ethyl.

4. A compound of the formula I according to claim 1, wherein R₁ is phenyl or phenyl substitued by 1 or 2 C₁-C₄alkyl or C₁-C₄alkoxy groups.

5. A compound of the formula I according to claim 1, wherein R₂, R₃, R₁₀ and R₁₁ are, independently of one another, C₁-C₈alkyl.

6. A compound of the formula I according to claim 5, wherein R₂ and R₃ are identical and are i-propyl or t-butyl.

7. A compound of the formula I according to claim 1, wherein R₂, R₃, R₁₀ and R₁₁ are cycloalkyl and contain from 5 to 8 C atoms.

8. A compound of the formula I according to claim 1, wherein R₂, R₃, R₁₀ and R₁₁ are, independently one of another, unsubstituted phenyl or phenyl substituted by 1, 2 or 3 substituents.

9. A compound of the formula I according to claim 8, wherein substitued phenyl R₂ , R₃ , R₁₀ and R₁₁ are 2-methyl-, 3-methyl-, 4-methyl-, 2- or 4-ethyl-, 2- or 4-i-propyl-, 2- or 4-t-butyl-, 2-methoxy-, 3-methoxy-, 4-methoxy-, 2- or 4-ethoxy-, 4-trimethylsilyl-, 2- or 4-fluoro-, 2,4-difluoro-, 2- or 4-chloro-, 2,4-dichloro-, 2,4-dimethyl-, 3,5-dimethyl, 2-methoxy-4-methyl-, 3,5-dimethyl-4-methoxy-, 3,5-dimethyl-4-(dimethylamino)-, 2- or 4-amino-, 2- or 4-methylamino-, 2- or 4-(dimethylamino)-, 2- or 4-SO₃H-, 2- or 4-SO₃Na-, 2- or 4-[⁺NH₃Cl⁻]-, 3,4,5-trimethylphen-1-yl, 2,4,6-trimethylphen-1-yl, 4-trifluoromethylphenyl or 3,5-di(trifluoromethyl)phenyl.

10. A compound of the formula I according to claim 1, wherein R₂ and R₃ are identical and are phenyl, cyclohexyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl or 3,5-dimethyl-4-methoxyphen-1-yl.

11. A compound of the formula I according to claim 1, wherein R₂ and R₃ are identical and are cylcohexyl or phenyl.

12. A compound of the formula I according to claim 1, wherein R₁₀ and R₁₁ are identical and are cyclohexyl, phenyl, t-butyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl or 3,5-dimethyl-4-methoxyphen-1-yl.

13. A compound of the formula I according to claim 1, wherein R₁ is methyl and R₂ and R₃ are each cyclohexyl or phenyl and R₁₀ and R₁₁ are phenyl, cyclohexyl or t-butyl.

14. A compound of the formula I according to claim 1, wherein R₁₂ is C₁-C₄alkyl.

15. A compound of the formula I according to claim 1, wherein R₁₃ is C₃-C₆alkylene.

16. A compound of the formula I according to claim 1, wherein R₁₄ is C₁-C₆alkylene.

17. A compound of the formula I according to claim 1, wherein R₁₅ is C₁-C₄alkyl.

18. A compound of the formula I according to claim 1, wherein Rₐ is OR₁₅.

19. A process for preparing compounds of the formula I according to claim 1, wherein, in a first step,
a) compounds of the formula III 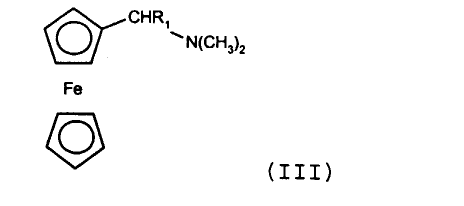 are reacted with butyllithium in an inert organic solvent in the presence of an amine complexing agent for lithium, the reaction product is subsequently reacted with a mixture of the compounds of the formulae IV ClPR₁₀R₁₁ (IV) and V ClSi(R₁₂)₂-(R₁₃)-Cl (V) to give compounds of the formula VI 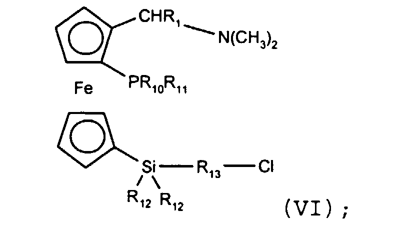 in a second step,
b) Compounds of the formula VI are reacted in an organic solvent with compounds of the formula VII HPR₂R₃ (VII) to give compounds of the formula Ia 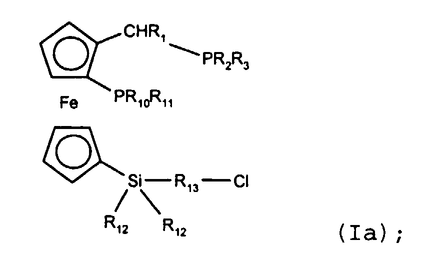
c) Compounds of the formula Ia are reacted with compounds of the formula VIII
NH₂(C₁-C₁₂alkyl) (VIII) to give compounds of the formula Ib 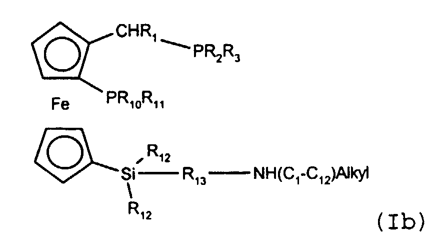 or compounds of the formula Ia are reacted first with potassium phthalimide and subsequently with hydrazine to give compounds of the formula Ic 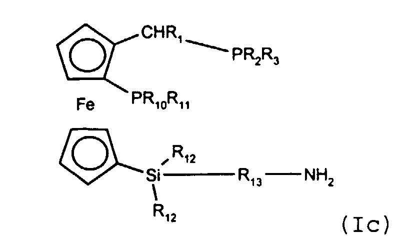 and, if desired in a further step,
d) Compounds of the formula Ib or Ic are reacted with a compound of the formula IX (Rₐ)ₙ(R₁₅O)₃₋ₙSi-R₁₄-NCO (IX) to give compounds of the formula Id 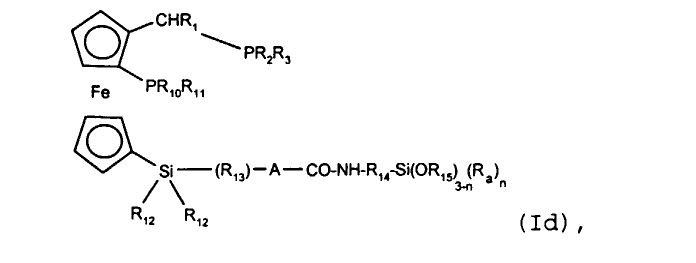 in which the radicals Rₐ, R₁, R₂, R₃, R₁₀, R₁₁, R₁₂, R₁₃, R₁₄, R₁₅, A and n are as defined in claim 1.

20. A process according to claim 19, wherein the mixture of the compounds of the formula IV ClPR₁₀ R₁₁ (IV) and V ClSi(R₁₂)₂-(R₁₃)-Cl (V) in the process step a) is present in a ratio of from 1:10 to 10:1.

21. A process according to claim 19, wherein, in the process step a), the reaction after the addition of the mixture of the compounds of the formula IV ClPR₁₀R₁₁ (IV) and V ClSi(R₁₂)₂-(R₁₃)-Cl (V) is carried out at a temperature of from 0°C to -40°C.

22. A metal complex of the formula Xa or Xb of a compound of the formula I 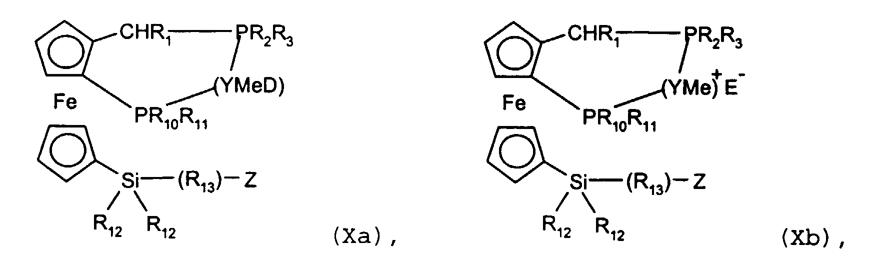
in which R₁, R₂, R₃, R₁₀, R₁₁, R₁₂ , R₁₃ and Z are as defined in claim 1;
Y is two monoolefin ligands or a diene ligand;
Me is Ir or Rh;
D is -Cl, -Br, -I;
E⁻ is the anion of an oxygen acid or a complex acid.

23. A metal complex of the formula Xa or Xb according to claim 22, wherein Y is 1,5-hexadiene, 1,5-cyclooctadiene or norbornadiene.

24. A metal complex of the formula Xa or Xb according to claim 22, wherein D is -Cl or -Br.

25. A metal complex of the formula Xa or Xb according to claim 22, wherein E⁻ is ClO₄⁻, CF₃SO₃⁻, CH₃SO₃⁻, HSO₄⁻, BF₄⁻, B(phenyl)₄⁻, PF₆⁻, SbCl₆⁻, AsF₆⁻ or SbF₆⁻.

26. A process for preparing metal complexes of the formula Xa or Xb according to claim 22, wherein compounds of the formula I are reacted with a metal compound of the formula [Me(Y)D]₂ or Me(Y)₂⁺ E⁻, in which Me is rhodium or iridium and Y, D and E⁻ are as defined in claim 22.

27. An inorganic or polymeric organic support material to which ferrocenyldiphosphines are bound, wherein the 1 and 2 positions of the one cyclopentadienyl ring bear 2 tertiary phosphine groups of which one is bound directly and the other via a group CHR₁ to the cyclopentadienyl ring and the other cyclopentadienyl ring bears a silylene group -Si(R₁₂)₂-R₁₃-A- which is bound via the Si atom and forms one end of an organic bridge at the other end of which the inorganic or polymeric organic support is bound via the group A directly or via an additional bridging group, where the radicals A, R₁, R₁₂ and R₁₃ are as defined in claim 1.

28. A polymeric organic support material according to claim 27, having structural repeating units of the formula XI 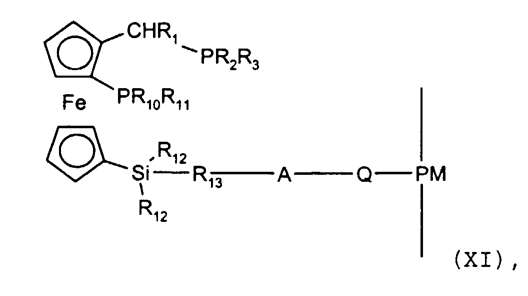 in which
A, R₁, R₂, R₃, R₁₀, R₁₁, R₁₂ and R₁₃ are as defined in claim 1; Q is a bridging group formed by a diisocyanate;
PM is the radical of a polymer-forming monomer which bears, directly or in a side chain, a hydroxyl group or a primary or secondary amino group as functional group which is bound to the diphosphine via a bridging group Q formed by a diisocyanate.

29. A polymeric organic support material according to claim 28, wherein the diisocyanates for the bridging group Q are selected from the group consisting of 1,6-bis(isocyanato)hexane, 5-isocyanato-3-(isocyanatomethyl)-1,1,3-trimethylcyclohexane, 1,3-bis(5-isocyanato-1,3,3-trimethylphenyl)-2,4-dioxo-1,3-diazetidine, 3,6-bis(9-isocyanatononyl)-4,5-di(1-heptenyl)cyclohexene, bis(4-isocyanatocyclohexyl)methane, trans-1,4-bis(isocyanato)cyclohexane, 1,3-bis-(isocyanatomethyl)benzene, 1,3-bis(1-isocyanato-1-methylethyl)benzene, 1,4-bis(2-isocyanatoethyl)cyclohexane, 1,3-bis(isocyanatomethyl)cyclohexane, 1,4-bis(1-isocyanato-1-methylethyl)benzene, bis(isocyanato)isododecylbenzene, 1,4-bis(isocyanato)benzene, 2,4-bis(isocyanato)toluene, 2,6-bis(isocyanato)toluene, 2,4-/2,6-bis(isocyanato)toluene, 2-ethyl-1,2,3-tris(3-isocyanato-4-methylanilinocarbonyloxy)propane, N,N'-bis(3-isocyanato-4-methylphenyl)urea, 1,4-bis(3-isocyanato-4-methylphenyl)-2,4-dioxo-1,3-diazetidine, 1,3,5-tris(3-isocyanato-4-methylphenyl)-2,4,6-trioxohexahydro-1,3,5-triazine, 1,3-bis(3-isocyanato-4-methylphenyl)-2,4,5-trioxoimidazolidine, bis(2-isocyanatophenyl)methane, (2-isocyanatophenyl)4-isocyanatophenyl)methane, bis(4-isocyanatophenyl)methane, 2,4-bis(4-isocyanatobenzyl)-1-isocyanatobenzene, [4-isocyanato-3-(4-isocyanatobenzyl)-phenyl][2-isocyanato-5-(4-isocyanatobenzyl)phenyl]methane, tris(4-isocyanatophenyl)-methane, 1,5-bis(isocyanato)naphthalene, or 4,4'-bis(isocyanato)-3,3'-dimethylbiphenyl.

30. A polymeric organic support material according to claim 28, wherein the diisocyanates for the bridging group Q are selected from the group consisting of 1,6-bis(isocyanato)hexane, 5-isocyanato-3-(isocyanatomethyl)-1,1,3-trimethylcyclohexane, 2,4-bis(isocyanato)toluene, 2,6-bis-(isocyanato)toluene, 2,4-/2,6-bis(isocyanato)toluene or bis(4-isocyanatophenyl)methane.

31. A polymeric organic support material according to claim 28 which is an uncrosslinked thermoplastic, crosslinked or structurally crosslinked polymer.

32. A polymeric organic support material according to claim 28 which is an essentially uncrosslinked polymer soluble in organic solvents, a partially crosslinked swellable polymer or a highly crosslinked porous polymer.

33. A polymeric organic support material according to claim 28, wherein the hydroxyl-functional or primary or secondary amino-functional monomer makes up from 1 to 100 mol% of the polymer.

34. A polymeric organic support material according to claim 33, wherein the hydroxyl-functional or primary or secondary amino-functional monomer makes up from 5 to 100 mol% of the polymer.

35. A polymeric organic support material according to claim 33, wherein the hydroxyl-functional or primary or secondary amino-functional monomer makes up from 10 to 100 mol% of the polymer.

36. A polymeric organic support material according to claim 28, wherein from 1 to 100 mol% of the hydroxyl groups or primary or secondary amino groups present in the polymer have been reacted to form compounds of the formula XI according to claim 28.

37. A polymeric organic support material according to claim 28, wherein the monomers forming the polymer are selected from the group consisting of styrene, p-methylstyrene or α-methylstyrene of which at least one contains a bonded hydroxyl group or a bonded primary or secondary amino group as functional group.

38. A polymeric organic support material according to claim 28 comprising further comonomers of dienes or acrylic derivatives, in particular butadiene, acrylonitrile, alkyl methacrylate, butadien-alkyl acrylate and methacrylate, maleic anhydride, acrylonitrile-methyl acrylate, which form random or block copolymers.

39. A polymeric organic support material according to claim 28, wherein the monomers forming the polymer are selected from the group consisting of α,β-unsaturated acids, their esters or amides, of which at least one contains a bonded hydroxyl group or a bonded primary or secondary amino group as functional group.

40. A polymeric organic support material according to claim 39, wherein the monomers forming the polymer are selected from the group consisting of acrylates and their C₁-C₄alkyl esters, methacrylates and their C₁-C₄alkyl esters, acrylamide and acrylonitrile of which at least one contains a bonded hydroxyl group or a bonded primary or secondary amino group as functional group.

41. A polymeric organic support material according to claim 39 comprising further olefinically unsaturated monomers which form random or block copolymers.

42. A polymeric organic support material according to claim 28, wherein the polymer is formed of monomers comprising vinyl alcohol as homopolymer or vinyl alcohol as copolymer with vinyl acetate, stearate, benzoate, maleate, vinyl butyral, allyl phthalate, allylmelamine.

43. A polymeric organic support material according to claim 28, wherein the monomers forming the polymer are phenol and a C₁-C₄aldehyde.

44. A polymeric organic support material according to claim 28, wherein the monomers forming the polymer form a polymer comprising cyclic C₃-C₆ethers or C₂-C₆alkylene glycols and bisglycidyl ethers.

45. A polymeric organic support material according to claim 28 comprising polysaccharides.

46. A polymeric organic support material according to claim 45, wherein the polysaccharides are cellulose, cellulose acetates, propionates or butyrates, cellulose ethers, starches, chitin or chitosan.

47. A polymeric organic support material according to claim 28 comprising polymers comprising structural repeating units of at least one monomer of a compound of the formula XIa, XIb XIc or XId 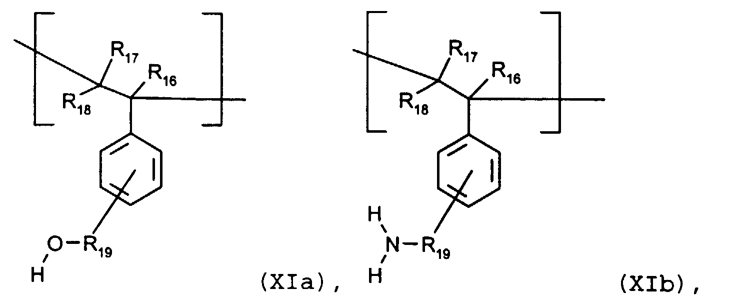 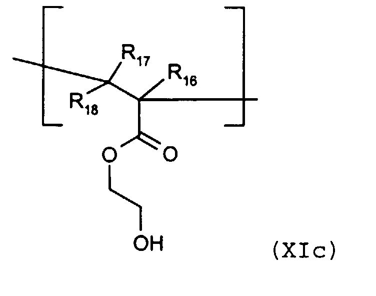 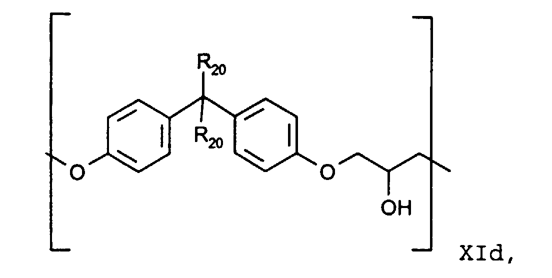
in which R₁₉ is C₁-C₄alkylene and
R₁₆, R₁₇, R₁₈ and R₂₀ are, independently of one another, hydrogen or C₁-C₄alkyl.

48. A polymeric organic support material according to claim 28, wherein the molecular weight is from 5000 to 5 000 000 dalton.

49. A polymeric organic support material according to claim 28, wherein the molecular weight is from 10 000 to 1 000 000 or 50 000 to 1000 000 dalton.

50. A polymeric organic support material according to claim 28 which is highly crosslinked macroporous polystyrene or polyacrylate.

51. A polymeric organic support material according to claim 50, wherein the particle size is from 10 µm to 2000 µm.

52. A polymeric organic support material according to claim 50, wherein the specific surface area of the porous highly crosslinked polymer is from 50 to 500 m²/g.

53. A process for preparing the polymeric support material according to claim 28, wherein the polymers containing structural repeating units of at least one monomer containing a bonded hydroxyl group or a bonded primary or secondary amino group as functional group directly in the polymer backbone or in a side chain
A) are, in a first step, completely or partially reacted with a diisocyanate forming a bridging group Q in an inert organic solvent, and the product is, in a second step, reacted with a diphosphine in which the 1 and 2 positions of one cyclopentadienyl radical bear 2 tertiary phosphine groups of which one is bound directly and the other via a group CHR₁ to the cyclopentadienyl ring, and the other cyclopentadienyl radical bears a silylene group -Si(R₁₂)₂-R₁₃-A-;
or
B) in a first step, a diphosphine in which the 1 and 2 positions of one cyclopentadienyl radical bear 2 tertiary phosphine groups of which one is bound directly and the other via a group CHR₁ to the cyclopentadienyl ring, and the other cyclopentadienyl radical bears a silylene group - Si(R₁₂)₂-R₁₃-A- is reacted with a diisocyanate forming a bridging group Q in an inert organic solvent and the product is, in a second step, completely or partially reacted with a polymer containing structural repeating units of at least one monomer containing a bonded hydroxyl group or a bonded primary or secondary amino group as functional group, where the radicals A,R₁,R₁₂ and R₁₃ are as defined in claim 1; and C) any free isocyanate groups still present are crosslinked with a C₂-C₂₄diol or C₂-C₂₄diamine or are reacted with a C₂-C₁₂alcohol or C₂-C₁₂amine.

54. A process for preparing the polymeric support material according to claim 28, wherein polymers containing structural repeating units of at least one monomer containing a bonded hydroxyl group or a bonded primary or secondary amino group as functional group
A) are, in a first step, completely or partially reacted with a diisocyanate forming a bridging group Q in an inert organic solvent and the product is, in a second step, completely or partially reacted with a diphosphine in which the 1 and 2 positions of one cyclopentadienyl radical bear 2 tertiary phosphine groups of which one is bound directly and the other via a group CHR₁ to the cyclopentadienyl ring, and the other cyclopentadienyl radical bears a silylene group - Si(R₁₂)₂-R₁₃-A- and the free isocyanate groups still present are allowed to react with an aliphatic C₂-C₁₂alcohol or C₂-C₁₂amine, where the radicals A, R₁, R₁₂ and R₁₃ are as defined in claim 1.

55. A process for preparing the polymeric support material according to claim 28, wherein the polymers containing structural repeating units of at least one monomer containing a bonded hydroxyl group or a bonded primary or secondary amino group as functional group
A) are, in a first step, completely or partially reacted with a diisocyanate forming a bridging group Q in an inert organic solvent and the product is, in a second step, completely or partially reacted with a diphosphine in which the 1 and 2 positions of one cyclopentadienyl radical bear 2 tertiary phosphine groups or which one is bound directly and the other via a group CHR₁ to the cyclopentadienyl ring, and the other cyclopentadienyl radical bears a silylene group - Si(R₁₂)₂-R₁₃-A- and the free isocyanate groups still present are crosslinked with an aliphatic C₂-C₂₄diol or C₂-C₂₄diamine.

56. A process for preparing the polymeric support material according to claim 55, wherein from 0.01 to 10 mol% of the total isocyanate groups present are crosslinked.

57. A solid inorganic support material according to claim 27 having ferrocenyldiphosphine ligands of the formula XIII bound to its surface 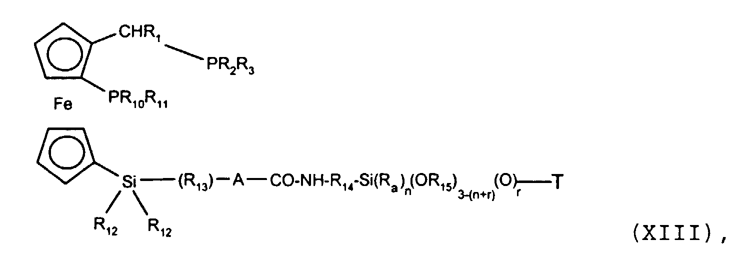
in which Rₐ, R₁, R₂, R₃, R₁₀, R₁₁, R₁₂, R₁₃, R₁₄, R₁₅, A and n are as defined in claim 1 and T is a solid inorganic support material where
r is 1, 2 or 3 when n is 0,
r is 1 or 2 when n is 1 and
r is 1 when n is 2.

58. A solid inorganic support material T according to claim 57 which is selected from the group consisting of silicates, semimetal or metal oxides and glasses or mixtures thereof.

59. A process for preparing a solid inorganic support material of the formula XIII according to claim 58, wherein compounds of the formula Id 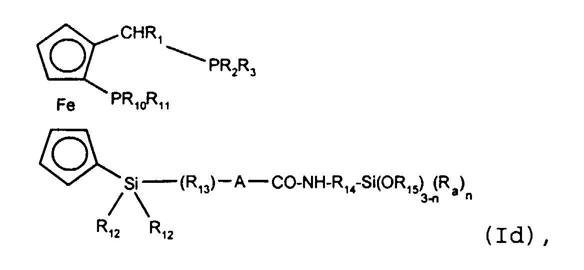 in which Rₐ, R₁, R₂, R₃, R₁₀, R₁₁, R₁₂, R₁₃, R₁₄, R₁₅, A and n are as defined in claim 1, are allowed to react in an inert organic solvent with a solid inorganic support material T.

60. A complex of rhodium or iridium with the inorganic or organic polymeric support material according to claim 27 to which are bound ferrocenyldiphosphines in which the 1 and 2 positions of one cyclopentadienyl ring bear tertiary phosphine groups of which one is bound directly and the other via a group CHR₁ to the cyclopentadienyl ring and the other cyclopentadienyl radical bears a silylene group - Si(R₁₂)₂-R₁₃-A-, bonded via the Si atom, which forms one end of an organic bridging group and at the other end of which the inorganic or polymeric organic support is bound via the group A, directly or via an additional group, where the radicals A, R₁, R₁₂ and R₁₃ are as defined in claim 1.

61. A complex of rhodium or iridium with the polymeric organic material of the formula XIIa or XIIb according to claim 28, 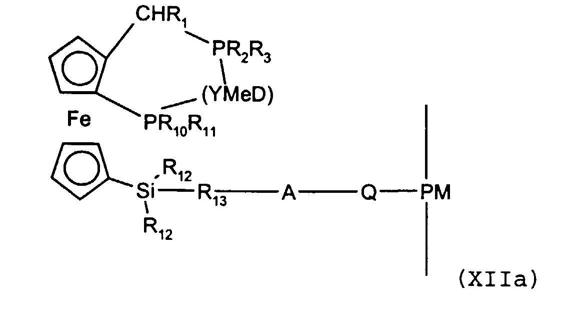 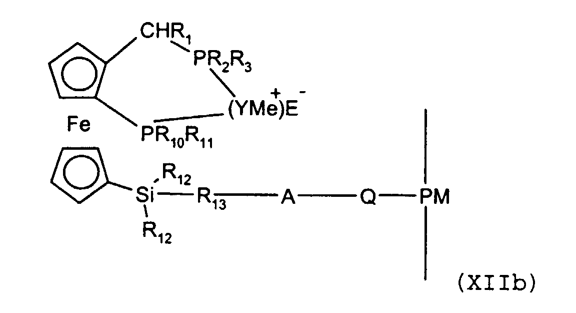 in which R₁, R₂, R₃, R₁₀, R₁₁, R₁₂, R₁₃, Q, PM, Y, Me, D and E⁻ are as defined in claims 1 and 22.

62. A process for preparing metal complexes according to claim 61, wherein compounds of the formula XI are reacted with a metal compound of the formula [M(Y)D]₂ or M(Y)₂⁺ E⁻ , in which M is rhodium or iridium and Y, D and E⁻ are as defined in claim 22.

63. A rhodium or iridium complex of the formula XIIIa or XIIIb of the solid inorganic support material of the formula XIII according to claim 57 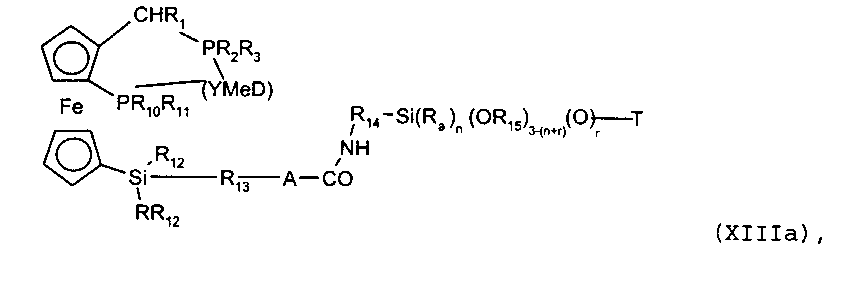 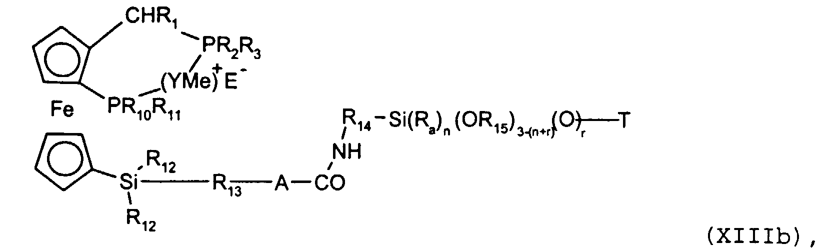 in which the radicals A, Rₐ, R₁, R₂, R₃, R₁₀, R₁₁, R₁₂, R₁₃, R₁₄ and R₁₅ are as defined in claim 1; Y, Me, D, E⁻, r, n and T are as defined in claims 22, 57 and 59.

64. Use of the metal complexes of rhodium or iridium according to claim 60 as heterogeneous or homogeneous catalysts for the assymetric hydrogenation of prochiral compounds containing carbon-carbon or carbon-heteroatom double bonds.

65. Use of the metal complexes according to claim 64 for the hydrogenation of assymetric ketimines.

66. A process for the assymetric hydrogenation of compounds containing carbon-carbon or carbon-heteroatom double bonds, wherein the compounds are reacted at a temperature of from - 20 to 80°C and a hydrogen pressure of from 10⁵ to 2x10⁷ Pa in the presence of catalytic amounts of one or more novel metal complexes of the formulae XIIa, XIIb, XIIIa and XIIIb according to claim 61 or claim 63.

## Revendications

1. Composés de formule I 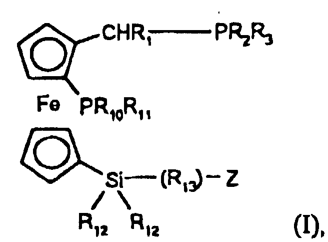 dans laquelle
R₁ représente un alkyle en C₁-C₈, un phényle ou un phényle substitué par un à trois alkyle en C₁-C₄ ou alcoxy en C₁-C₄ ;
R₂, R₃, R₁₀ et R₁₁ représentent chacun indépendamment l'un de l'autre un alkyle en C₁-C₁₂, un cycloalkyle en C₅-C₁₂, un phényle, un cycloalkyle en C₅-C₁₂ substitué par un alkyle en C₁-C₄ ou un alcoxy en C₁-C₄ ou un phényle mono- ou poly-substitué par un à trois groupes alkyle en C₁-C₄, alcoxy en C₁-C₄, -SiR₄R₅R₆, halogène, -SO₃M, -CO₂M, -PO₃M, -NR₇R₈, -[⁺NR₇R₈R₉]X⁻ ou fluoroalkyle en C₁-C₅ ; ou les groupes -PR₂R₃ et -PR₁₀R₁₁ représentent indépendamment l'un de l'autre un radical de formule II, IIa, IIb ou IIc 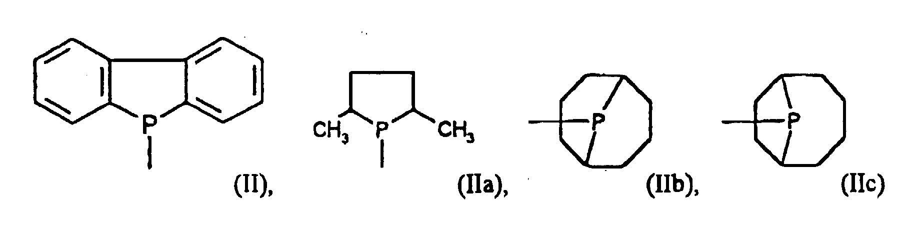 et,
R₄, R₅ et R₆ représentent chacun indépendamment l'un de l'autre un alkyle en C₁-C₁₂ ou un phényle ;
R₇ et R₈ représentent H, un alkyle en C₁-C₁₂, un phényle ou
R₇ et R₈ représentent ensemble un tétraméthyléne, un pentaméthylène ou un 3-oxa-1,5-pentylène,
R₉ représente H ou un alkyle en C₁-C₄ ;
les radicaux R₁₂ représentent des radicaux identiques ou différents, et représentent indépendamment l'un de l'autre un alkyle en C₁-C₁₂, un cycloalkyle en C₃-C₇, un benzyle ou un phényle ou représentent ensemble un alkylène C₅-C₁₂ et
R₁₃ représente un alkylène en C₁-C₁₂ ou un phénylène,
M représente H ou un métal alcalin,
X⁻ est un anion d'un acide monobasique,
Z représente Cl, NH₂, NH-alkyle en C₁-C₁₂, ou un groupe -A-CO-NH-R₁₄-Si(Rₐ)ₙ(OR₁₅)₃₋ₙ,
où
A représente NH ou N(alkyle en C₁-C₁₂),
R₁₄ représente un alkylène en C₁-C₁₂,
R₁₅ représente un alkyle en C₁-C₁₂,
Rₐ représente un alkyle en C₁-C₄ ou OR₁₅,
n vaut 0, 1 ou 2 ;
et les composés de formule I sont sous la forme de leurs racémates et diastéréoméres ou mélanges de diastéréomères.

2. Composés de formule I selon la revendication 1, caractérisés en ce que R₁ est un alkyle linéaire.

3. Composés de formule I selon la revendication 2, caractérisés en ce que R₁ représente un méthyle ou un éthyle.

4. Composés de formule I selon la revendication 1, caractérisés en ce que R₁ est un phényle ou un phényle substitué par un ou deux groupes alkyle en C₁-C₄ ou alcoxy en C₁-C₄.

5. Composés de formule I selon la revendication 1, caractérisés en ce que R₂, R₃, R₁₀ et R₁₁ représentent chacun indépendamment l'un de l'autre un alkyle en C₁-C₈.

6. Composés de formule I selon la revendication 5, caractérisés en ce que R₂ et R₃ sont identiques et représentent un i-propyle ou un t-butyle.

7. Composés de formule I selon la revendication 1, caractérisés en ce que R_{2,} R₃, R₁₀ et R₁₁ représentent un cycloalkyle ayant 5 à 8 atomes de carbone.

8. Composés de formule I selon la revendication 1, caractérisés en ce que R₂, R₃, R₁₀ et R₁₁ représentent indépendamment l'un de l'autre un phényle non substitué ou substitué par 1, 2 ou 3 substituants.

9. Composés de formule I selon la revendication 8, caractérisés en ce que R₂, R₃, R₁₀ et R₁₁ représentent, comme phényle substitué, un 2-méthyl-, 3-méthyl-, 4-méthyl-, 2- ou 4-éthyl-, 2- ou 4-i-propyl-, 2- ou 4-t-butyl-, 2-méthoxy-, 3-méthoxy-, 4-méthoxy-, 2- ou 4-éthoxy-, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino-, 2- ou 4-(diméthylamino)-, 2- ou 4-SO₃H, 2- ou 4-SO₃Na-, 2-ou 4-[⁺NH₃Cl⁻]-, 3,4,5-triméthylphén-1-yl-, 2,4,6-triméthylphén-1-yle, 4-trifluorométhylphényle ou 3,5-di-(trifluorométhyl)phényle.

10. Composés de formule I selon la revendication 1, caractérisés cn ce que R₂ et R₃ sont identiques et représentent un phényle, cyclohexyle, 2-ou 4-méthylphén-1-yle, 2- ou 4-méthoxyphén-1-yle, 2- ou 4-(diméthylamino)phén-1-yle, 3,5-diméthyl-4-(diméthylamino)phén-1-yle et 3,5-diméthyl-4-méthoxyphén-1-yle.

11. Composés de formule I selon la revendication 1, caractérisés en ce que R₂ et R₃ sont des radicaux identiques et représentent un cyclohexyle ou un phényle.

12. Composés de formule I selon la revendication 1, caractérisés en ce que R₁₀ et R₁₁ sont identiques et représentent un cyclohexyle, phényle, t-butyle, 2- ou 4-méthylphén-1-yle, 2- ou 4-méthoxyphén-1-yle, 2- ou 4-(diméthylamino)phén-1-yle, 3,5-diméthyl-4-(diméthylamino)phén-1-yle et 3,5-diméthyl-4-méthoxyphén-1-yle.

13. Composés de formule I selon la revendication 1, caractérisés en ce que R₁ représente un méthyle et R₂ et R₃ représentent chacun un cyclohexyle ou un phényle et R₁₀ et R₁₁ représentent un phényle, un cyclohexyle ou un t-butyle.

14. Composés de formule I selon la revendication 1, caractérisés en ce que R₁₂ représente un alkyle en C₁-C₄.

15. Composés de formule I selon la revendication 1, caractérisés en ce que R₁₃ représente un alkylène en C₃-C₆.

16. Composé de formule I selon la revendication 1, caractérisés en ce que R₁₄ représente un alkylène en C₁-C₆.

17. Composés de formule I selon la revendication 1, caractérisés en ce que R₁₅ représente un alkyle en C₁-C₄.

18. Composés de formule I selon la revendication 1, caractérisés en ce que Rₐ représente OR₁₅.

19. Procédé de préparation de composés de formule I selon la revendication 1, caractérisé en ce que dans une première étape
a) on fait réagir des composés de formule III 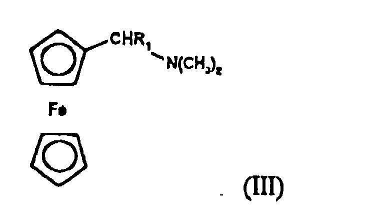 dans un solvant organique inerte en présence d'un formateur de complexe aminé pour Li avec du butyllithium, puis on fait réagir le produit de la réaction avec un mélange des composés de formule IV ClPR₁₀R₁₁ (IV) et V ClSi(R₁₂)₂-(R₁₃)-Cl (V) pour donner des composés de formule VI 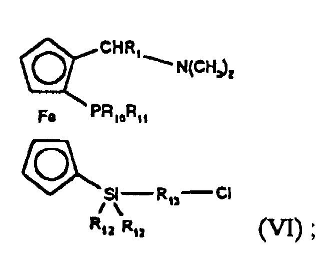 dans une deuxième étape
b) on fait réagir les composés de formule VI dans un solvant organique avec des composés de formule VII HPR₂R₃ (VII) pour donner des composés de formule Ia 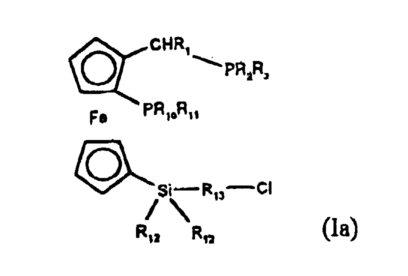
c) on fait réagir les composés de formule la avec des composés de formule VIII NH₂(alkyle en C₁-C₁₂) (VIII) pour donner des composés de formule Ib 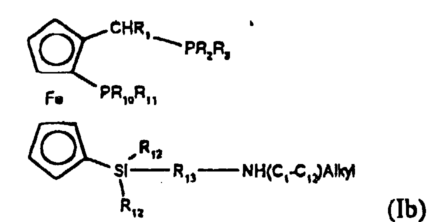 ou des composés de formule Ia tout d'abord avec du phtalimide de potassium puis avec de l'hydrazine pour donner des composés de formule Ic 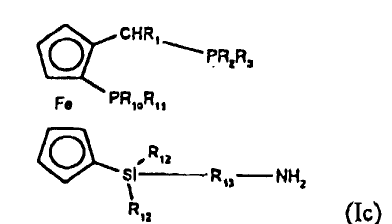 et le cas échéant dans une étape ultérieure
d) on fait réagir les composés de formule Ib ou Ic avec un composé de formule IX (Rₐ)ₙ(R₁₅O)₃₋ₙSi-R₁₄-NCO (IX) pour donner des composés de formule Id 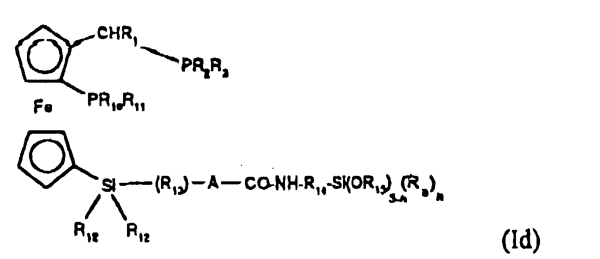 où les radicaux Rₐ, R₁, R₂, R₃, R₁₀, R₁₁, R₁₂, R₁₃, R₁₄, R₁₅, A et n ayant les significations indiquées dans la revendication 1.

20. Procédé selon la revendication 19, caractérisé en ce que le mélange des composés de formule IV ClPR₁₀R₁₁ (IV) et V ClSi(R₁₂)₂-(R₁₃)-Cl (V) dans l'étape a) du procédé se présente dans un rapport de 1:10 à 10:1.

21. Procédé selon la revendication 19, caractérisé en ce que dans l'étape a) du procédé, après addition du mélange des composés de formule IV CIPR₁₀R₁₁ (IV) et V ClSi(R₁₂)₂-(R₁₃)-Cl (V), on travaille à une température comprise entre 0°C et -40°C.

22. Complexes métalliques de formules Xa et Xb des composés de formule I 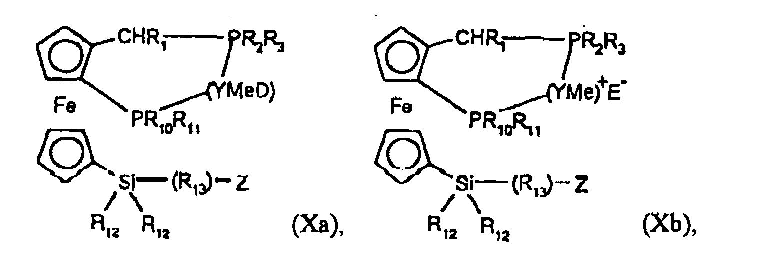 où
R₁, R₂, R₃, R₁₀, R₁₁, R₁₂, R₁₃ et Z ont les significations indiquées dans la revendication 1 ;
Y représente deux ligands mono-oléfines ou un ligand diène ;
Me représente Ir ou Rh ;
D représente -Cl, -Br, -I ;
E⁻ est l'anion d'un acide oxygéné ou d'un acide complexe.

23. Complexes métalliques de formules Xa ou Xb selon la revendication 22, caractérisés en ce que Y représente le 1,5-hexadiène, le 1,5-cyclo-octadiène ou le norbornadiène.

24. Complexes métalliques de formules Xa ou Xb selon la revendication 22, caractérisés en ce que D représente -Cl ou -Br.

25. Complexes métalliques de formule Xa ou Xb selon la revendication 22, caractérisés en ce que E⁻ représente ClO₄⁻, CF₃SO₃⁻, CH₃SO₃⁻, HSO₄⁻, BF₄⁻, B(phényl)₄⁻, PF₆⁻, SbCl₆⁻, AsF₆⁻ ou SbF₆⁻.

26. Procédé de préparation de complexes métalliques de formule Xa ou Xb selon la revendication 22, caractérisé en ce qu'on fait réagir des composés de formule I avec un composé métallique de formule [Me(Y)D]₂ ou Me(Yb)₂⁺E⁻ , où Me représente le rhodium ou l'iridium et Y, D et E⁻ ont les significations indiquées dans la revendication 22.

27. Matériaux-supports inorganiques ou organiques polymères auxquels sont liées des ferrocényl diphosphines, caractérisés en ce que deux groupes phosphine tertiaires sont liés en positions 1,2 d'un noyau cyclopentadiényle, dont l'un est lié directement et l'autre par l'intermédiaire d'un groupe CHR₁ sur le noyau cyclopentadiènyle, et sur l'autre noyau cyclopentadiényle un groupe silylène =Si(R₁₂)₂-R₁₃A- est lié par l'intermédiaire de l'atome de Si, qui forme l'extrémité d'un groupe-pont organique et à l'autre extrémité duquel le matériau-support inorganique ou organique polymère est lié directement par l'intermédiaire du groupe A ou par l'intermédiaire d'un autre groupe-pont, les radicaux A, R₂, R₁₂ et R₁₃ ayant la signification indiquée dans la revendication 1.

28. Matériau-support organique polymère selon la revendication 27, caractérisé en ce qu'il contient des éléments structuraux répétitifs de formule XI 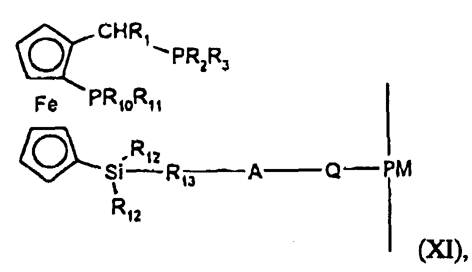 dans laquelle
A, R₁, R₂, R₃, R₁₀, R₁₁, R₁₂ et R₁₃ ont la signification indiquée dans la revendication 1 ;
Q représente un groupe-pont formé par un diisocyanate ;
PM est le radical d'un monomère formateur de polymère, qui contient lié directement ou dans une chaîne latérale, un groupe hydroxyle ou un groupe amine primaire ou secondaire comme groupe fonctionnel, qui est lié à la diphosphine par l'intermédiaire d'un groupe-pont Q formé par un diisocyanate.

29. Matériau-support organique polymère selon la revendication 28, caractérisé en ce que les diisocyanates pour le groupe formateur de pont Q sont choisis dans le groupe constitué par le 1,6-bis-[isocyanato]-hexane, le 5-isocyanato-3-(isocyanatométhyl)-1,1,3-triméthylcyclohexane, la 1,3-bis-[5-isocyanato-1,3,3-triméthylphényl]-2,4-dioxo-1,3-diazétidine, le 3,6-bis-(9-isocyanatononyl]-4,5-di-(1-heptényl)-cyclohexène, le bis-[4-isocyanato-cyclohexyl]-méthane, le trans-1,4-bis-[isocyanato]-cyclohexane, le 1,3-bis-[isocyanatométhyl]-benzène, le 1,3-bis-[1-isocyanato-1-méthyléthyl]-benzène, le 1,4-bis-[2-isocyanatoéthyl]-cyclohexane, le 1,3-bis-[isocyanatométhyl]-cyclohexane, le 1,4-bis-[1-isocyanato-1-méthyléthyl]-benzène, le bis-[isocyanato]-isododécylbenzène, le 1,4-bis-[isocyanato]-benzène, le 2,4-bis-[isocyanato]-toluène, le 2,6-bis-[isocyanato]-toluène, le 2,4-/2,6-bis-[isocyanato]-toluène, le 2-éthyl-1,2,3-tris-[3-isocyanato-4-méthyl-anilinocarbonyloxy]-propane, la N,N'-bis-[3-isocyanato-4-méthylphényl]-urée, la 1,4-bis-[3-isocyanato-4-méthylphényl]-2,4-dioxo-1,3-diazétidine, la 1,3,5-tris-[3-isocyanato-4-méthylphényl]-2,4,6-trioxohexahydro-1,3,5-triazine, la 1,3-bis-[3-isocyanato-4-méthylphényl]-2,4,5-trioxoimidazolidine, le bis-[2-isocyanatophényl]-méthane, le (2-isocyanatophényl)-(4-isocyanatophényl)-méthane, le bis-[4-isocyanatophényl]-méthane, le 2,4-bis-[4-isocyanatobenzyl]-1-isocyanatobenzène, le [4-isocyanato-3-(4-isocyanatobenzyl)-phényl)-[2-isocyanato-5(4-isocyanatobenzyl)-phényl]-méthane, le tris-[4-isocyanatophényl]-méthane, le 1,5-bis-[isocyanato]-naphtalène ou le 4,4'-bis[isocyanato]-3,3'-diméthyl-biphényle.

30. Matériau-support organique polymère selon la revendication 28, caractérisé en ce que les diisocyanates pour le groupe formateur de pont Q sont choisis dans le groupe constitué par le 1,6-bis-[isocyanato]-hexane, le 5-isocyanato-3-(isocyanatométhyl)-1,1,3-triméthylcyclohexane, le 2,4-bis-[isocyanato]-toluène, le 2,6-bis-[isocyanato]-toluène, le 2,4-/2,6-bis-[isocyanato]-toluène ou le bis-[4-isocyanato-phényl]-méthane.

31. Matériau-support organique polymère selon la revendication 28, caractérisé en ce qu'il s'agit de polymères thermoplastiques non réticulés, réticulés ou réticulés dans leur structure.

32. Matériau-support organique polymère selon la revendication 28, caractérisé en ce qu'il s'agit de polymères essentiellement non réticulés, solubles dans des solvants organiques, partiellement réticulés gonflables ou hautement réticulés poreux.

33. Matériau-support organique polymère selon la revendication 28, caractérisé en ce que le monomère à fonction hydroxyle, amine primaire ou secondaire participe à raison de 1 à 100 % molaire à la constitution du polymère.

34. Matériau-support organique polymère selon la revendication 33, caractérisé en ce que le monomère à fonction hydroxyle, amine primaire ou secondaire participe à raison de 5 à 100 % molaire à la constitution du polymère.

35. Matériau-support organique polymère selon la revendication 33, caractérisé en ce que le monomère à fonction hydroxyle, amine primaire ou secondaire participe à raison de 10 à 100 % molaire à la constitution du polymère.

36. Matériau-support organique polymère selon la revendication 28, caractérisé en ce que de 1 à 100 % molaire des groupes hydroxyle, amine primaire ou secondaire présents dans le polymère ont réagi, pour donner des composés de formule Xl selon la revendication 28.

37. Matériau-support organique polymère selon la revendication 28, caractérisé en ce que les monomères formant le polymère sont choisis dans le groupe constitué par le styrène, le p-méthylstyréne ou l'alpha-méthylstyrène, dont au moins un contient un loupe hydroxyle ou un groupe amine primaire ou secondaire comme groupe fonctionnel lié.

38. Matériau-support organique polymère selon la revendication 28, caractérisé en ce que sont présents d'autres comonomères de diènes ou de dérivés acryliques, en particulier le butadiène, l'acrylonitrile, un méthacrylate d'alkyle, un alkylacrylate de butadiène et le méthacrylate de butadiène, l'anhydride maléfique, le méthylacrylate d'acrylonitrile, qui forment des copolymères statistiques ou séquencés.

39. Matériau-support organique polymère selon la revendication 28, caractérisé en ce que les monomères formant le polymère sont choisis dans le groupe constitué par les acides insaturés en α, β leurs esters ou amides, dont au moins un contient un groupe hydroxyle ou un groupe amine primaire ou secondaire comme groupe fonctionnel lié.

40. Matériau-support organique polymère selon la revendication 39, caractérisé en ce que les monomères formant le polymère sont choisis dans le groupe constitué par les acrylates et leurs esters d'alkyle en C₁-C₄, et les méthacrylates et leurs esters d'alkyle en C₁-C₄, l'acrylamide et l'acrylonitrile, dont au moins un contient un groupe hydroxyle ou un groupe amine primaire ou secondaire comme groupe fonctionnel lié.

41. Matériau-support organique polymère selon la revendication 39, caractérisé en ce que d'autres monomères oléfiniquement insaturés sont présents, qui forment des copolymères statistiques ou séquences.

42. Matériau-support organique polymère selon la revendication 28, caractérisé en ce que le polymère est formé de monomères qui contiennent de l'alcool vinylique comme homopolymère ou de l'alcool vinylique comme copolymère avec de l'acétate, du stéarate, du benzoate, ou du maléate de vinyle, du vinylbutyral, du phtalate d'allyle, de l'allylmélamine.

43. Matériau-support organique polymère selon la revendication 28, caractérisé en ce que les monomères formant le polymère sont le phénol, et un aldéhyde en C₁-C₄.

44. Matériau-support organique polymère selon la revendication 28, caractérisé en ce que les monomères formant le polymère forment un polymère d'éthers en C₃-C₆ cycliques ou d'alkylène glycols en C₂-C₆ et de bis-glycidyl éthers.

45. Matériau-support organique polymère selon la revendication 28, caractérisé en ce qu'il s'agit de polysaccharides.

46. Matériau-support organique polymère selon la revendication 45, caractérisé en ce que les polysaccharides sont la cellulose, l'acétate, le propionate ou le butyrate de cellulose, les éthers de cellulose, l'amidon, la chitine et le chitosane.

47. Matériau-support organique polymère selon la revendication 28, caractérisé en ce qu'il s'agit de polymères à éléments structuraux répétitifs d'au moins un monomère, d'un composé de formule XIa, XIb, Xlc ou XId 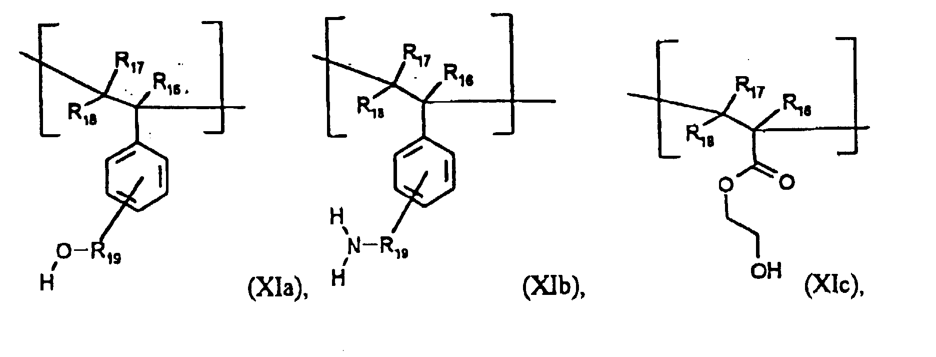 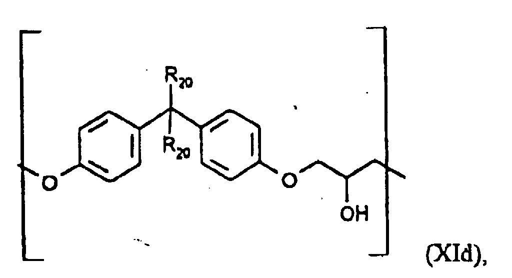 où
R₁₉ est un alkylène en C₁-C₄, et
R₁₆, R₁₇, R₁₈ et R₂₀ représentent indépendamment l'un de l'autre un hydrogène ou un alkyle en C₁-C₄.

48. Matériau-support organique polymère selon la revendication 28, caractérisé en ce que le poids moléculaire est de 5000 à 5 millions de daltons.

49. Matériau-support organique polymère selon la revendication 28, caractérisé en ce que le poids moléculaire est de 10 000 à 1 million, en particulier de 50 000 à 1 million de daltons.

50. Matériau-support organique polymère selon la revendication 28, caractérisé en ce qu'il s'agit de polystyrène ou de polyacrylate macroporeux hautement réticulé.

51. Matériau-support organique polymère selon la revendication 50, caractérisé en ce que la taille particulaire est de 10 µm à 2000 µm.

52. Matériau-support organique polymère selon la revendication 50, caractérisé en ce que la surface spécifique des polymères hautement réticulés poreux est de 50 à 500 m²/g.

53. Procédé de préparation du matériau-support polymère selon la revendication 28, caractérisé en ce qu'on fait réagir les polymères ayant des éléments structuraux répétitifs d'au moins un monomère, qui contient directement dans l'ossature polymère ou dans une chaîne latérale un groupe hydroxyle ou un groupe amine primaire ou secondaire comme groupe fonctionnel,
A) dans une première étape avec un diisocyanate formant un groupe-pont Q dans un solvant organique inerte, de façon totale ou partielle, et en ce qu'on fait réagir le produit dans une seconde étape avec une diphosphine qui contient liés sur un radical cyclopentadiényle deux groupes phosphine tertiaire en positions 1, 2, dont l'un est lié directement et l'autre par un groupe CHR₁ au noyau cyclopentadiényle, et contient sur l'autre radical cyclopentadiényle un groupe silylène -Si(R₁₂)₂-A- ; ou
B) dans une première étape on fait réagir une diphosphine qui contient liés sur un radical cyclopentadiényle deux groupes phosphine tertiaire dans les positions 1, 2, dont l'un est lié directement et l'autre par l'intermédiaire d'un groupe CHR₁ au noyau cyclopentadiényle, et sur l'autre radical cyclopentadiényle un groupe silylène -Si(R₁₂)₂-R₁₃-A- lié, avec un diisocyanate formant un groupe-pont Q dans un solvant organique inerte, et en ce que dans une seconde étape on fait réagir entièrement ou partiellement le produit avec un polymère à éléments structuraux répétitifs d'au moins un monomère qui contient un groupe hydroxyle ou un groupe amine primaire ou secondaire comme groupe fonctionnel lié, les radicaux A, R₁, R₁₂ et R₁₃ ayant la signification indiquée dans la revendication 1 ; et C) le cas échéant on réticule les groupes isocyanates encore libres avec un diol en C₂-C₂₄ ou une diamine en C₂-C₂₄ ou on les fait réagir avec un alcool en C₂-C₁₂ ou une amine en C₂-C₁₂.

54. Procédé de préparation du matériau-support polymère selon la revendication 28, caractérisé en ce qu'on fait réagir des polymères à éléments structuraux répétitifs d'au moins un monomère qui contient un groupe hydroxyle ou un groupe amine primaire ou secondaire comme groupe fonctionnel lié
A) dans une première étape avec un diisocyanate formant un groupe-pont Q dans un solvant organique inerte, de façon totale ou partielle, et en ce qu'on fait réagir le produit dans une seconde étape avec une diphosphine, qui contient sur un radical cyclopentadiényle deux groupes phosphine tertiaire liés dans les positions 1, 2, dont l'un est lié directement et l'autre par l'intermédiaire d'un groupe CHR₁ au noyau cyclopentadiényle et sur l'autre radical cyclopentadiényle un groupe silylène -Si(R₁₂)₂-R₁₃-A- lié, cette réaction étant totale ou partielle, et en ce qu'on fait réagir les groupes isocyanates encore libres avec un alcool aliphatique en C₂-C₁₂ ou une amine en C₂-C₁₂, les radicaux A, R₁, R₁₂ et R₁₃ ayant la signification indiquée dans la revendication 1.

55. Procédé de préparation du matériau-support polymère selon la revendication 28, caractérisé en ce qu'on fait réagir de façon totale ou partielle les polymères à éléments structuraux répétitifs d'au moins un monomère qui contient un groupe hydroxyle ou un groupe amine primaire ou secondaire comme groupe fonctionnel lié,
A) dans une première étape on fait réagir avec un diisocyanate formant un groupe-pont Q dans un solvant organique inerte, de façon totale ou partielle, et on fait réagir le produit dans une seconde étape avec une diphosphine qui contient sur un radical cyclopentadiényle deux groupes phosphine tertiaire liés dans les positions 1, 2, dont l'un est lié directement et l'autre par l'intermédiaire d'un groupe CHR₁ au noyau cyclopentadiényle, et sur l'autre radical cyclopentadiényle un groupe silylène -Si(R₁₂)₂-R₁₃-A- lié, cette réaction étant totale ou partielle, et on réticule les groupes isocyanate encore libres avec un diol en C₂-C₂₄ aliphatique ou une diamine en C₂-C₂₄.

56. Procédé de préparation du matériau-support polymère selon la revendication 55, caractérisé en ce qu'on réticule de 0,01 à 10 % molaire des groupes isocyanates présents au total.

57. Matériau-support inorganique solide selon la revendication 27, caractérisé en ce qu'il présente à la surface des ligands ferrocényl diphosphines de formule XIII 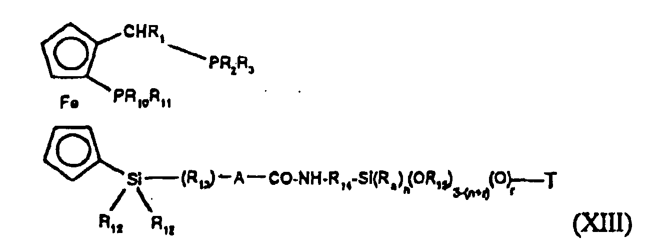
dans laquelle Rₐ, R₁, R₂, R₃, R₁₀, R₁₁, R₁₂, R₁₃, R₁₄, R₁₅, A et n ont les significations indiquées dans la revendication 1 et T représente un matériau-support inorganique solide, où
lorsque n vaut 0, r vaut 1, 2 ou 3,
lorsque n vaut 1, r vaut 1 ou 2, et
lorsque n vaut 2, r vaut 1.

58. Matériau-support inorganique solide T selon la revendication 57, caractérisé en ce que le matériau-support inorganique est choisi dans le groupe constitué par les silicates, les semi-oxydes métalliques ou oxydes métalliques et les verres ou leurs mélanges.

59. Procédé de préparation d'un matériau-support inorganique de formule XIII selon la revendication 58, caractérisé en ce qu'on fait réagir des composés de formule Id 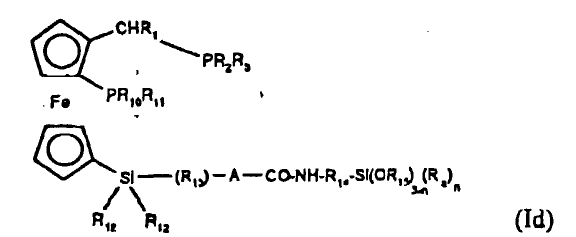 dans laquelle Rₐ, R₁, R₂, R₃, R₁₀, R₁₁, R₁₂, R₁₃, R₁₄, R₁₅, A et n ont les significations indiquées dans la revendication 1 dans un solvant organique inerte avec un matériau-support inorganique solide T.

60. Complexes métalliques de rhodium ou d'iridium avec des matériaux-supports polymères inorganiques ou organiques selon la revendication 27, auxquels sont liées des ferrocényl diphosphines, caractérisés en ce que des groupes phosphine tertiaire sont liés dans les positions 1, 2 d'un noyau cyclopentadiényle, dont l'un est lié directement et l'autre par l'intermédiaire d'un groupe CHR₁ au noyau cyclopentadiényle ct en ce que sur l'autre radical cyclopentadiényle un groupe silylène -Si(R₁₂)₂-R₁₃-A- est lié par l'intermédiaire de l'atome de Si qui forme une extrémité d'un groupe-pont organique et à l'autre extrémité duquel le matériau-support inorganique ou organique polymère est lié directement par l'intermédiaire du groupe A ou par l'intermédiaire d'un autre groupe supplémentaire, les radicaux A, R₁, R₁₂ et R₁₃ ayant les significations indiquées dans la revendication 1.

61. Complexes métalliques de rhodium ou d'iridium avec le matériau organique polymère de formule XIIa ou XIIb selon la revendication 28, 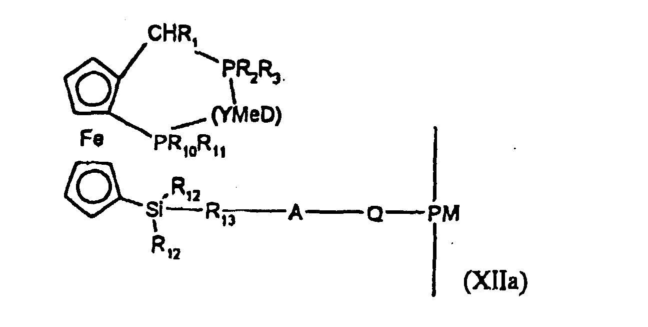 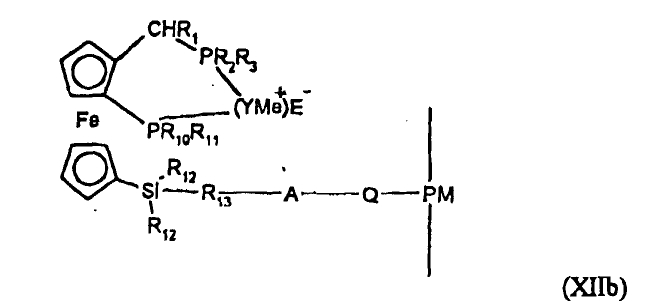 dans lesquelles R₁, R₂, R₃, R₁₀, R₁₁, R₁₂, R₁₃, Q, PM, Y, Me, D et E⁻ ont les significations indiquées dans les revendications 1 et 22.

62. Procédé de préparation de complexes métalliques selon la revendication 61, caractérisé en ce qu'on fait réagir des composés de formule XI avec un composé métallique de formule [M(Y)D]₂ ou M(Y)₂⁺E⁻, où M représente le rhodium ou l'iridium et Y, D et E⁻ ont les significations indiquées dans la revendication 22.

63. Complexes de rhodium ou d'iridium de formules XIIIa et XIIIb du matériau-support inorganique solide de formule XIII selon la revendication 57 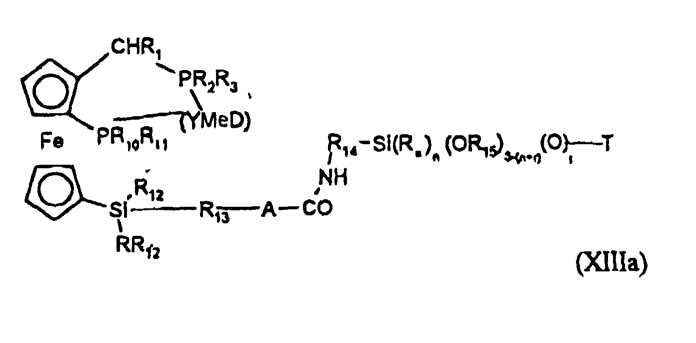 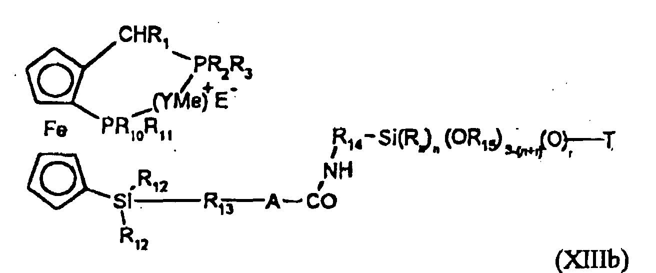 dans lesquelles les radicaux A, Rₐ, R₁, R₂, R₃, R₁₀, R₁₁, R₁₂, R₁₃, R₁₄ et R₁₅ ont les significations indiquées dans la revendication 1 ; Y, Me, D, E⁻, r, n et T ont les significations indiquées dans les revendications 22, 57 et 59.

64. Utilisation des complexes métalliques de rhodium ou d'iridium selon la revendication 60 comme catalyseurs hétérogènes ou homogènes pour l'hydrogénation asymétrique de composés prochiraux avec des doubles liaisons carbone-carbone ou carbone-hétéroatome.

65. Utilisation des complexes métalliques selon la revendication 64 pour l'hydrogénation de cétimines asymétriques.

66. Procédé d'hydrogénation asymétrique de composés à doubles liaisons carbone-carbone ou carbone-hétéroatome, caractérisé en ce qu'on fait réagir les composés à une température allant de -20 à 80°C et sous une pression d'hydrogène allant de 10⁵ à 2 x 10⁷ Pa en présence de quantités catalytiques d'un ou plusieurs complexes métalliques selon l'invention se formules XIIa, XIIb, XIIIa et XIIIb selon la revendication 61 ou la revendication 63.
